# EUROPEAN PATENT APPLICATION

(11) **EP 4 538 273 A1**
(43) Date of publication of application: **16.04.2025**
(21) Application number: 23818979.9
(22) Date of filing: 30.05.2023
(51) Int. Cl.: C07D 471/04, C07D 417/14, A61K 31/45, A61P 35/00

(54) **COMPOUND AND USE THEREOF IN PREPARATION OF BCL-XL INHIBITOR**

(30) Priority: 10.06.2022 CN 202210653733
(71) Applicant: Hitgen Inc., Chengdu, Sichuan 610200 (CN); Sichuan Kelun-Biotech Biopharmaceutical Co., Ltd., Chengdu, Sichuan 611138 (CN)
(72) Inventor: LIU, Chuan, Chengdu, Sichuan 610200 (CN); LIU, Jinming, Chengdu, Sichuan 611138 (CN); DOU, Dengfeng, Chengdu, Sichuan 610200 (CN); LIU, Qian, Chengdu, Sichuan 61113 (CN); XIA, Shuai, Chengdu, Sichuan 610200 (CN); TAI, Zhengfu, Chengdu, Sichuan 611138 (CN); ZHANG, Wei, Chengdu, Sichuan 610200 (CN); YANG, Xiaojiao, Chengdu, Sichuan 611138 (CN); CAI, Longying, Chengdu, Sichuan 610200 (CN); SONG, Hongmei, Chengdu, Sichuan 611138 (CN); LI, Jin, Chengdu, Sichuan 610200 (CN); GE, Junyou, Chengdu, Sichuan 611138 (CN)
(74) Representative: Meissner Bolte Partnerschaft mbB
(86) International application number: PCT/CN2023/097145
(87) International publication number: WO 2023/236814

(57) **Abstract**

Disclosed in the present invention is a compound and the use thereof in preparation of BCL-XL inhibitor, belong to the field of pharmacy. The structure of the compound provided by the present invention is represnted in Formula I. The compound can effectively bind to BCL-XL protein, thereby further inhibiting activity of the BCL-XL protein. The compound an be used for preparing a BCL-XL inhibitor and drugs for preventing and/or treating diseases related to the BCL-XL protein activity (such as cancer and autoimmune diseases, etc.), and has broad prospect in clinical application. The compound has good pharmacological characteristics, good stability, high drugability. **In** assition, the compound has simple preparation method, high yield and low cost, and thus is suitable for industrial production.

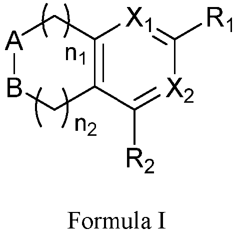

## Description

### Cross-reference to related application

The present application claims priority to Chinese patent application No. 202210653733.X filed on June 10, 2022, entitled "Compound and use thereof in preparation of BCL-XL inhibitor", and the entire content of which is incorporated herein by reference.

### Technical Field

The present invention belongs to the pharmaceutical field, and specifically relates to a compound and its use in the preparation of BCL-XL inhibitor.

### Background Art

Cancer is the number one killer that seriously threatens human health, with a high incidence rate and high mortality rate. Studies have shown that one of the causes of tumor occurrence and drug resistance is that the high expression of BCL-XL genes and proteins in human cells prevents the body from normally clearing abnormal genes, which inhibits the cell apoptosis. Studies have shown that cell apoptosis plays a negative regulatory role in the development of tumors and can inhibit the proliferation of tumor cells. The ratio of BCL-XL in the heterodimer formed by BCL-XL and Bak determines whether the cell survives after receiving the apoptotic signal. If the expression level of BCL-XL decreases, it will not be able to balance the effect of the pro-apoptotic gene Bak, which can cause cells to undergo apoptosis.

BCL-XL is a member of the BCL-2 family and, as an anti-apoptotic protein, is overexpressed in a variety of cancer cells and is an anti-tumor therapeutic target with a clear effect. At present, studies have reported that BCL-XL inhibitors have positive therapeutic effects on autoimmune diseases and various cancers (including bladder cancer, brain cancer, breast cancer, bone marrow cancer, cervical cancer, chronic lymphocytic leukemia, colorectal cancer, esophageal cancer, hepatocellular carcinoma, primitive lymphocytic leukemia, follicular lymphoma, lymphoid malignancy of T cell or B cell origin, melanoma, granulocytic leukemia, myeloma, oral cancer, ovarian cancer, non-small cell lung cancer, prostate cancer, small cell lung cancer, spleen cancer, etc.). The development of drugs that can effectively inhibit the activity of BCL-XL anti-apoptotic proteins is of great significance for the clinical treatment of autoimmune diseases and various cancers.

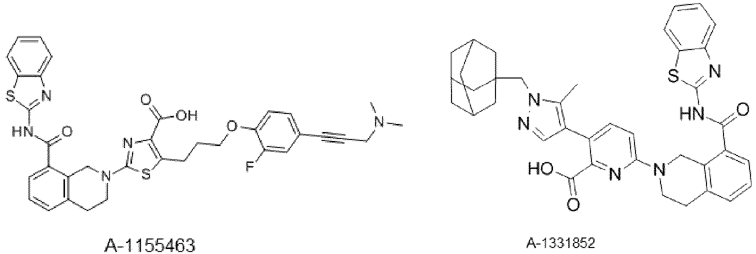

At present, several BCL-XL inhibitors have been reported, such as A-1155463, A-1331852, etc. Among them, A-1155463 is a highly effective and selective BCL-XL inhibitor with an EC₅₀ value of 70 nM in Molt-4 cells; A-1331852 is a BCL-XL selective inhibitor with a Ki value of less than 10 pM. However, in order to meet the large number of clinical needs, it is of great significance to develop more new BCL-XL inhibitors.

### Contents of the Invention

The purpose of the present invention is to provide a new compound and a use of the compound in the manufacture of a BCL-XL inhibitor, and a drug for the prevention and/or treatment of a disease related to BCL-XL protein activity.

The present invention provides a compound, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, the structure of which is shown in Formula I: 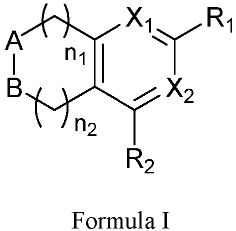
X₁, X₂ are each independently selected from the group consisting of N and CR^{x};
R^{×} is independently selected from the group consisting of hydrogen, -C_{1~6} alkyl, halogen-substituted C_{1~6} alkyl, halogen, -OH, -O(C_{1~6} alkyl), -NH₂, -NH(C_{1~6} alkyl), and -N(C_{1~6} alkyl)(C_{1~6} alkyl);
n₁, n₂ are each independently selected from the group consisting of 0, 1 and 2;
R₁ is selected from the group consisting of 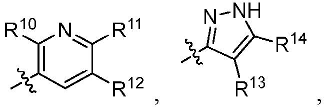 8- to 12-membered fused heteroaromatic ring, -NH(6- to 12-membered fused heteroaromatic ring), 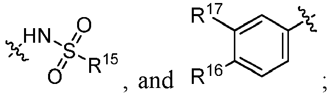 preferably the 8- to 12-membered fused heteroaromatic ring is 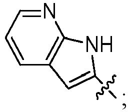
R¹⁰ is selected from the group consisting of hydrogen, halogen, cyano, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl), and -N(C_{1~10} alkyl)(C_{1~10} alkyl);
R¹¹ is selected from the group consisting of hydrogen, -C_{1~10} alkyl, halogen-substituted -C_{1~10} alkyl, -NH₂, -NH(C_{1~10} alkyl), -N(C_{1~10} alkyl)(C_{1~10} alkyl), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-C(O)NH(C_{1~10} alkyl), -C_{0~2} alkylene-C(O)N(C_{1~10} alkyl)(C_{1~10} alkyl), -C_{0~2} alkylene-O-(C_{6~10} aromatic ring), and -C_{0~2} alkylene-O-(5- to 10-membered aromatic heterocyclic ring);
R¹² is selected from the group consisting of hydrogen, halogen, cyano, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl), -N(C_{1~10} alkyl)(C_{1~10} alkyl), -C_{0~2} alkylene-C(O)NH(C_{1~10} alkyl), and -C_{0~2} alkylene-C(O)N(C_{1~10} alkyl)(C_{1~10} alkyl);
or, R¹¹, R¹² together with the atoms connected thereto form a 3- to 10-membered carbocyclic ring, a 3- to 10-membered heterocyclic ring, a C_{6~10} aromatic ring, or a 5- to 10-membered aromatic heterocyclic ring; the carbocyclic ring, heterocyclic ring, aromatic ring, or aromatic heterocyclic ring is unsubstituted or substituted with one, two, or three substituents independent selected from the group consisting of halogen, oxo, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl) and -N(C_{1~10} alkyl)(C_{1~10} alkyl);
R¹³ is selected from the group consisting of hydrogen, halogen, cyano, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl), and -N(C_{1~10} alkyl)(C_{1~10} alkyl);
R¹⁴ is selected from the group consisting of hydrogen, -C_{1~10} alkyl, halogen-substituted -C_{1~10} alkyl, -OH, -O(C_{1~10} alkyl), -NH₂, -NH(C_{1~10} alkyl), and -N(C_{1~10} alkyl)(C_{1~10} alkyl);
or, R¹³, R¹⁴ together with the atoms connected thereto form a C_{6~10} aromatic ring, and a 5-to 10-membered aromatic heterocyclic ring;
R¹⁵ is selected from the group consisting of -C_{1~10} alkyl, halogen-substituted -C_{1~10} alkyl, -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), and -C_{0~2} alkylene-(5- to 10-membered aromatic heterocyclic ring);
R¹⁶ is selected from the group consisting of hydrogen, halogen, cyano, -C_{2~10} alkenyl, -C_{2~10} alkynyl, -C_{1~10} alkyl, halogen-substituted -C_{1~10} alkyl, -NH₂, -NH(C_{1~10} alkyl), -N(C_{1~10} alkyl)(C_{1~10} alkyl), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-C(O)NH(C_{1~10} alkyl), -C_{0~2} alkylene-C(O)NH(C_{6~10} aromatic ring), -C_{0~2} alkylene-NHC(O)(C_{1~10} alkyl), -C_{0~2} alkylene-O-(C_{6~10} aromatic ring), -C_{0~2} alkylene-O-(5- to 10-membered aromatic heterocyclic ring), and -C_{0~2} alkylene-NHS(O)O(C_{1~10} alkyl);
R¹⁷ is selected from the group consisting of hydrogen, halogen, cyano, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl), -N(C_{1~10} alkyl)(C_{1~10} alkyl), -C_{0~2} alkylene-C(O)NH(C_{1~10} alkyl), -C_{0~2} alkylene-C(O)N(C_{1~10} alkyl)(C_{1~10} alkyl), -C_{0~2} alkylene-NHC(O)(C_{1~10} alkyl), and -C_{0~2} alkylene-NHS(O)O(C_{1~10} alkyl);
R¹⁶ and R¹⁷ are not hydrogen at the same time;
R₂ is selected from the group consisting of hydrogen, -OH, -O(C_{1~10} alkyl), -NH₂, -NH(C_{1~10} alkyl), and -N(C_{1~10} alkyl)(C_{1~10} alkyl); the alkyl is unsubstituted or substituted with one, two or three independent R²¹;
R²¹ is selected from the group consisting of halogen, -OH, -O(C_{1~10} alkyl), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), and -C_{0~2} alkylene-(5- to 10-membered aromatic heterocyclic ring);
one of A and B is CR⁴R⁵ and the other is NR₃;
R⁴ is selected from the group consisting of hydrogen, -C_{1~6} alkyl, and halogen-substituted C_{1~6} alkyl;
R⁵ is selected from the group consisting of hydrogen, -C_{1~6} alkyl, and halogen-substituted C_{1~6} alkyl;
R₃ is selected from the group consisting of hydrogen, -C_{1~6} alkyl, halogen-substituted C_{1~6} alkyl, -C_{0~2} alkylene-C(O)R³¹, -C_{0~2} alkylene-(3- to 10-membered cycloalkyl), -C_{0~2} alkylene-(3-to 10-membered heterocycloalkyl), -C_{0~2} alkylene-(C_{6~10} aromatic ring), and -C_{0~2} alkylene-(5-to 10-membered aromatic heterocyclic ring); the alkylene, alkyl, cycloalkyl, heterocycloalkyl, aromatic ring, aromatic heterocyclic ring are unsubstituted or substituted with one, two or three independent R³²;
R³¹ is selected from the group consisting of hydrogen, -C_{1~10} alkyl, halogen-substituted C_{1~10} alkyl, halogen, -OH, -O(C_{1~10} alkyl), -NH₂, -NH(C_{1~6} alkyl), -N(C_{1~6} alkyl)(C_{1~6} alkyl), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), and -C_{0~2} alkylene-(9- to 12-membered fused aromatic ring); the alkylene, alkyl, cycloalkyl, heterocycloalkyl, aromatic ring, aromatic heterocyclic ring are unsubstituted or substituted with one, two or three independent R^{32'};
R³² and R^{32'} are each independently selected from the group consisting of carboxyl, halogen, cyano, carbonyl, nitro, -C_{2~10} alkenyl, -C_{2~10} alkynyl, -C_{1~10} alkyl, halogen-substituted C_{1~10} alkyl, -OH, -O(C_{1~10} alkyl), -O(halogen-substituted C_{1~6} alkyl), -NH₂, -NH(C_{1~10} alkyl), -N(C_{1~10} alkyl)(C_{1~10} alkyl), -C_{0~2} alkylene-O-(C_{6~10} aromatic ring), -C_{0~2} alkylene-O-(5- to 10-membered aromatic heterocyclic ring), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), -C_{0~2} alkylene-C(O)OLi, -C_{0~2} alkylene-C(O)OH, -C_{0~2} alkylene-C(O)NHR³³, and -C_{0~2} alkylene-NHC(O)R³³; the alkylene, alkyl, alkenyl, alkynyl, cycloalkyl, heterocycloalkyl, aromatic ring, aromatic heterocyclic ring are unsubstituted or substituted with one, two or three independent R^{33'};
R³³ and R^{33'} are each independently selected from the group consisting of hydrogen, carboxyl, halogen, -C_{1~10} alkyl, -C_{2~10} alkenyl, -C_{2~10} alkynyl, -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-NR³⁴R³⁵, -C_{0~2} alkylene-NHC(O)R³⁴, -C_{0~4} alkylene-C(O)NHR³⁴, and -C_{0~2} alkylene-NHC(O)OR³⁴; the alkylene, alkyl, alkenyl, alkynyl, cycloalkyl, heterocycloalkyl, aromatic ring, aromatic heterocyclic ring are unsubstituted or substituted with one, two or three independent R^{34'};
R³⁴ and R^{34'} are each independently selected from the group consisting of hydrogen, halogen, -C_{1~10} alkyl, -C_{2~10} alkenyl, -C_{2~10} alkynyl, -NH₂, -NH(C_{1~10} alkyl), -N(C_{1~10} alkyl)(C_{1~10} alkyl), -C_{0~2} alkylene-(3- to 10-membered cycloalkyl), -C_{0~2} alkylene-(3- to 10-membered heterocycloalkyl), -C_{0~2} alkylene-NHC(O)R³⁵, and -C_{0~2} alkylene-C(O)NHR³⁵; the alkylene, alkyl, alkenyl, alkynyl, cycloalkyl, heterocycloalkyl are unsubstituted or substituted with one, two or three independent R^{35'}*;*
the halogen in R³³, R^{33'}, R³⁴, R^{34'} does not comprise iodine;
R³⁵ and R^{35'} are each independently selected from the group consisting of hydrogen, halogen, -C_{1~10} alkyl, -NH₂, -NH(C_{1~6} alkyl), -N(C_{1~6} alkyl)(C_{1~6} alkyl), -C_{0~2} alkylene-(3- to 10-membered cycloalkyl), -C_{0~2} alkylene-(3- to 10-membered heterocycloalkyl), -C_{0~2} alkylene-NHC(O)R³⁶, -C_{0~2} alkylene-C(O)NHR³⁶, -C_{0~2} alkylene-C(O)OR³⁶, and -C_{0~2} alkylene-C(O)R³⁶; the alkylene, alkyl, cycloalkyl, heterocycloalkyl are unsubstituted or substituted with one, two or three independent R^{36'};
R³⁶ and R^{36'} are each independently selected from the group consisting of hydrogen, halogen, -C_{1~10} alkyl, -NH₂, -NH(C_{1~6} alkyl), and -N(C_{1~6} alkyl)(C_{1~6} alkyl).

Further, the structure of the compound is shown in Formula IIa, Formula IIb, Formula IIc, Formula IId or Formula IIe: 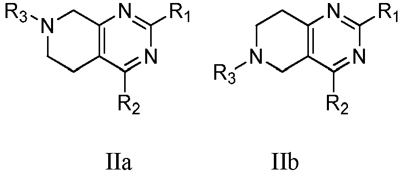 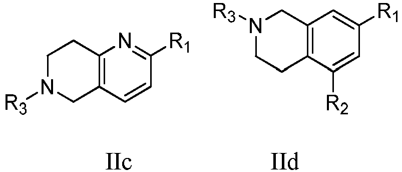 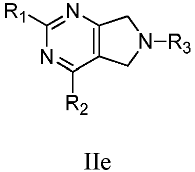 wherein, R₁, R₂, and R₃ are as described above.

Further, R¹⁰ is selected from the group consisting of hydrogen, -OH, halogen, and -C_{1~6} alkyl;
R¹¹ is selected from the group consisting of hydrogen, -C_{1~6} alkyl, -C_{0~2} alkylene-C(O)NH(C_{1~6} alkyl), and -C_{0~2} alkylene-O-(C₆ aromatic ring);
R¹² is selected from the group consisting of hydrogen, halogen, -C_{1~6} alkyl, and -C(O)NH(C_{1~6} alkyl);
or, R¹¹, R¹² together with the atoms connected thereto form a 3- to 6-membered heterocyclic ring or a C₆ aromatic ring; the heterocyclic ring or aromatic ring is unsubstituted or substituted with one, two or three substituents independently selected from the group consisting of hydrogen, halogen, hydroxyl, oxo, -C_{1~6} alkyl, and halogen-substituted C_{1~6} alkyl;
R¹³ is selected from the group consisting of hydrogen, halogen, cyano, and -C_{1~6} alkyl;
R¹⁴ is selected from the group consisting of hydrogen, -C_{1~6} alkyl, halogen-substituted -C_{1~6} alkyl, -OH, -O(C_{1~10} alkyl), -NH₂, and -NH(C_{1~6} alkyl);
or, R¹³, R¹⁴ together with the atoms connected thereto form a C₆ aromatic ring or a 5- to 6-membered aromatic heterocyclic ring;
R¹⁵ is selected from the group consisting of -C_{1~6} alkyl, halogen-substituted -C_{1~6} alkyl, -C_{0~2} alkylene-(C₆ aromatic ring), and -C_{0~2} alkylene-(5- to 6-membered aromatic heterocyclic ring);
R¹⁶ is selected from the group consisting of hydrogen, halogen, -C_{1~6} alkyl, -C_{0~2} alkylene-C(O)NH(C₆ aromatic ring), -C_{0~2} alkylene-NHC(O)(C_{1~6} alkyl), and -C_{0~2} alkylene-NHS(O)O(C_{1~6} alkyl);
R¹⁷ is selected from the group consisting of hydrogen, halogen, cyano, -C_{1~6} alkyl, halogen-substituted C_{1~6} alkyl, -C_{0~2} alkylene-C(O)NH(C_{1~6} alkyl), -C_{0~2} alkylene-C(O)N(C_{1~6} alkyl)(C_{1~6} alkyl), -C_{0~2} alkylene-NHC(O)(C_{1~6} alkyl), and -C_{0~2} alkylene-NHS(O)O(C_{1~6} alkyl);
R¹⁶ and R¹⁷ are not hydrogen at the same time.

Further, R₁ is selected from the group consisting of 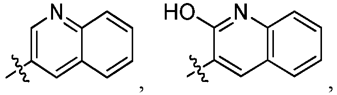 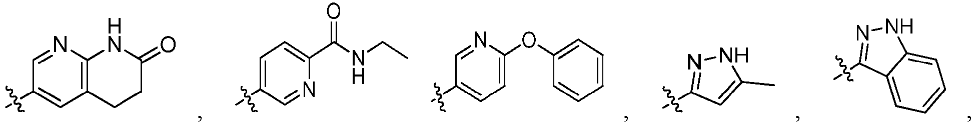 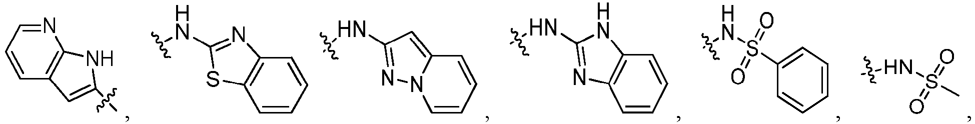 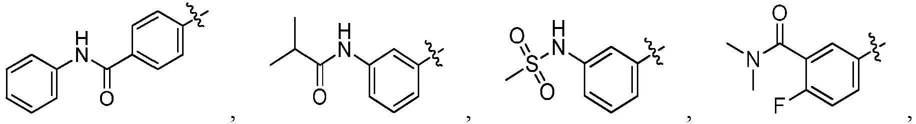 and

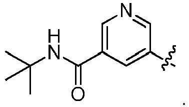

Further, R₂ is selected from the group consisting of hydrogen, OH, -O(C_{1~6} alkyl), -NH₂, -NH(C_{1~6} alkyl), and -N(C_{1~6} alkyl)(C_{1~6} alkyl); the alkyl is unsubstituted or substituted with one, two or three independent R²¹;
R²¹ is selected from the group consisting of halogen, -C_{0~2} alkylene-(C₆ aromatic ring), and -C_{0~2} alkylene-(5- to 6-membered aromatic heterocyclic ring).

Further, R₂ is selected from the group consisting of hydrogen, -OH, 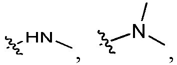 methoxy and

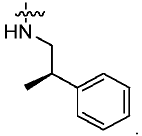

Further, R₃ is selected from the group consisting of hydrogen, -C_{1~6} alkyl, halogen-substituted C_{1~6} alkyl, -C_{0~2} alkylene-C(O)R³¹, -C_{0~2} alkylene-(C_{6~10} aromatic ring), and -C_{0~2} alkylene-(5- to 10-membered aromatic heterocyclic ring); the alkylene, alkyl, aromatic ring, aromatic heterocyclic ring is unsubstituted or substituted with one, two or three independent R³²;
R³¹ is selected from the group consisting of hydrogen, -C_{1~6} alkyl, halogen-substituted C_{1~6} alkyl, -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), and -C_{0~2} alkylene-(9- to 12-membered fused aromatic ring); the alkylene, alkyl, aromatic ring, aromatic heterocyclic ring are unsubstituted or substituted with one, two or three independent R^{32'};
R³² and R^{32'} are each independently selected from the group consisting of hydrogen, carboxyl, halogen, -C_{2~6} alkenyl, -C_{2~6} alkynyl, -C_{1~6} alkyl, halogen-substituted C_{1~6} alkyl, -OH, -O(C_{1~6} alkyl), -O(halogen-substituted C_{1~6} alkyl), -NH₂, -C_{0~2} alkylene-O-(C_{6~10} aromatic ring), -C_{0~2} alkylene-O-(5- to 10-membered aromatic heterocyclic ring), -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), -C_{0~2} alkylene-C(O)OLi, -C_{0~2} alkylene-C(O)OH, -C_{0~2} alkylene-C(O)NHR³³, and -C_{0~2} alkylene-NHC(O)R³³; the alkylene, alkyl, alkenyl, alkynyl, aromatic ring, aromatic heterocyclic ring are unsubstituted or substituted with one, two or three independent R^{33'};
R³³ and R^{33'} are each independently selected from the group consisting of hydrogen, halogen, -C_{1~6} alkyl, -C_{2~6} alkenyl, -C_{2~6} alkynyl, -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-NR³⁴R³⁵, -C_{0~2} alkylene-NHC(O)R³⁴, -C_{0~4} alkylene-C(O)NHR³⁴, and -C_{0~2} alkylene-NHC(O)OR³⁴; the alkylene, alkyl, alkenyl, alkynyl, cycloalkyl, heterocycloalkyl, aromatic ring, aromatic heterocyclic ring are unsubstituted or substituted with one, two or three independent R^{34'};
R³⁴ and R^{34'} are each independently selected from the group consisting of hydrogen, halogen, -C_{1~6} alkyl, -C_{2~6} alkenyl, -C_{2~6} alkynyl, -N(C_{1~6} alkyl)(C_{1~6} alkyl), -C_{0~2} alkylene-(3- to 10-membered cycloalkyl), -C_{0~2} alkylene-(3- to 10-membered heterocycloalkyl), -C_{0~2} alkylene-NHC(O)R³⁵, and -C_{0~2} alkylene-C(O)NHR³⁵; the alkylene, alkyl, alkenyl, alkynyl, cycloalkyl, heterocycloalkyl are unsubstituted or substituted with one, two or three independent R^{35'}
the halogen in R³³, R³⁴, R^{33'}, R^{34'} does not comprise iodine;
R³⁵ and R^{35'} are each independently selected from the group consisting of hydrogen, halogen, -C_{1~6} alkyl, -C_{0~2} alkylene-(3- to 10-membered heterocycloalkyl), -C_{0~2} alkylene-C(O)OR³⁶, and -C_{0~2} alkylene-C(O)R³⁶; the alkylene, alkyl, heterocycloalkyl are unsubstituted or substituted with one, two or three independent R^{36'};
R³⁶ and R^{36'} are each independently selected from the group consisting of hydrogen, halogen, and -C_{1~6} alkyl.

Further, R₃ is selected from the group consisting of: 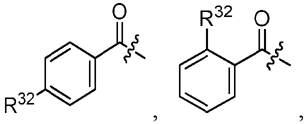 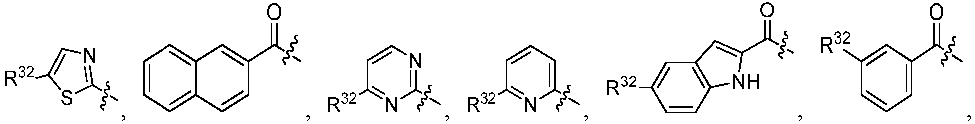 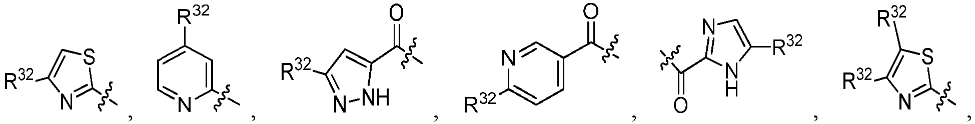 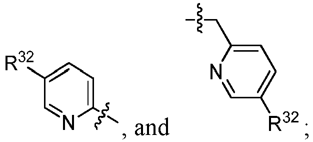
R³² is independently selected from the group consisting of hydrogen, carboxyl, halogen, -C_{2~6} alkenyl, -C_{2~6} alkynyl, -C_{1~6} alkyl, halogen-substituted C_{1~6} alkyl, -O(C_{1~6} alkyl), -O(halogen-substituted C_{1~6} alkyl), -NH₂, -C_{0~2} alkylene-O-(C_{6~10} aromatic ring), -C_{0~2} alkylene-O-(5- to 10-membered aromatic heterocyclic ring), -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), -C_{0~2} alkylene-C(O)OLi, -C_{0~2} alkylene-C(O)OH, and -C_{0~2} alkylene-C(O)NHR³³; the alkylene, alkyl, alkenyl, alkynyl, aromatic ring, aromatic heterocyclic ring are unsubstituted or substituted with one, two or three independent R^{33'};
R³³ and R^{33'} are each independently selected from the group consisting of hydrogen, halogen, -C_{1~6} alkyl, -C_{2~6} alkenyl, -C_{2~6} alkynyl, -C_{0~2} alkylene-(3- to 10-membered heterocycloalkyl), -C_{0~2} alkylene-(C_{6~10} aromatic ring), -C_{0~2} alkylene-NR³⁴R³⁵, -C_{0~2} alkylene-NHC(O)R³⁴, -C_{0~4} alkylene-C(O)NHR³⁴, and -C_{0~2} alkylene-NHC(O)OR³⁴; the alkylene, alkyl, alkenyl, alkynyl, heterocycloalkyl, aromatic ring are unsubstituted or substituted with one, two or three independent R^{34'};
R³⁴ and R^{34'} are each independently selected from the group consisting of hydrogen, -C_{1~6} alkyl, -C_{2~6} alkynyl, -N(C_{1~6} alkyl)(C_{1~6} alkyl), -C_{0~2} alkylene-(3- to 10-membered heterocycloalkyl), and -C_{0~2} alkylene-NHC(O)R³⁵; the alkylene, alkyl, alkynyl, heterocycloalkyl are unsubstituted or substituted with one, two or three independent R^{35'}:
R³⁵ and R^{35'} are each independently selected from the group consisting of hydrogen, -C_{1~6} alkyl, -C_{0~2} alkylene-(3- to 10-membered heterocycloalkyl), -C_{0~2} alkylene-C(O)OR³⁶, and -C_{0~2} alkylene-C(O)R³⁶; the alkylene, alkyl, heterocycloalkyl are unsubstituted or substituted with one, two or three independent R^{36'}; R³⁶ and R^{36'} are each independently selected from the group consisting of hydrogen, and -C_{1~6} alkyl.

Further, R₃ is 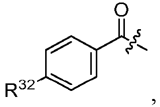 R³² is selected from the group consisting of carboxyl, -C₂ alkenyl, -C_{0~2} alkylene-O-phenyl, -C_{0~2} alkylene-O-benzimidazolyl (e.g., -2-C_{0~2} alkylene-benzimidazolyl), and -C_{0~2} alkylene-C(O)NHR³³; the alkylene, alkenyl, phenyl, benzimidazolyl are unsubstituted or substituted with one, two or three independent R^{33'};
R³³ and the R^{33'} are each independently selected from the group consisting of hydrogen, halogen, -C_{1~3} alkyl, -C₂ alkynyl, -C_{0~2} alkylene-phenyl, -C_{0~2} alkylene-piperidinyl (e.g., -4-C_{0~2} alkylene-piperidinyl), -C_{0~2} alkylene-NHC(O)R³⁴, and -C_{0~4} alkylene-C(O)NHR³⁴; the alkylene, alkyl, alkynyl, phenyl, piperidinyl are unsubstituted or substituted with one, two or three independent R^{34'};
R³⁴ and R^{34'} are each independently selected from the group consisting of hydrogen, -C_{1~2} alkyl, -N(C_{1~2} alkyl)(C_{H} alkyl), and -C_{0~2} alkylene-piperazinyl (e.g., -4-C_{0~2} alkylene-1-piperazinyl, further such as -4-C_{0~2} alkylene-1-piperazinyl that replaces the H on the alkynyl); the alkylene, alkyl, piperazinyl are unsubstituted or substituted with one, two or three independent R^{35'};
R³⁵ and R^{35'} are each independently selected from the group consisting of hydrogen, -C_{1~2} alkyl, and -C_{0~2} alkylene-C(O)OR³⁶; the alkyl is unsubstituted or substituted with one, two or three independent R^{36'}; R³⁶ and R^{36'} are independently selected from the group consisting of hydrogen, and -C_{1~4} alkyl.

Or further, R₃ is 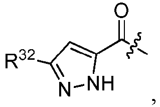 R³² is independently selected from the group consisting of hydrogen, and phenyl; the phenyl is unsubstituted or substituted with one, two or three independent R^{33'};
R^{33'} is selected from the group consisting of hydrogen, halogen, -C_{1~6} alkyl, -C_{2~6} alkynyl, -C_{0~2} alkylene-piperidinyl (e.g., -4-C_{0~2} alkylene-piperidinyl), -C_{0~2} alkylene-NHC(O)R³⁴, -C_{0~4} alkylene-C(O)NHR³⁴, and -C_{0~2} alkylene-NHC(O)OR³⁴; the alkylene, alkyl, alkynyl, piperidinyl are unsubstituted or substituted with one, two or three independent R^{34'};
R³⁴ and R^{34'} are each independently selected from the group consisting of hydrogen, -C_{1~2} alkyl, piperazinyl, and -N(C_{1~2} alkyl)(C_{1~2} alkyl); the alkyl and piperazinyl are unsubstituted or substituted with one, two or three independent R^{35'}, such as piperazinyl that replaces the H on the alkynyl with the nitrogen at position 1, or piperazinyl that replaces the H on the alkyl with the nitrogen at position 1;
R^{35'} is selected from the group consisting of hydrogen, and -C_{0~2} alkylene-C(O)R³⁶, such as -C_{0~2} alkylene-C(O)R³⁶ that replaces the H on the nitrogen at position 4 of piperazinyl; the alkylene is unsubstituted or substituted with one, two or three independent R^{36'}; R³⁶ and R^{36'} are each independently selected from the group consisting of hydrogen, and -C_{1~2} alkyl.

Further, R₃ is selected from the group consisting of 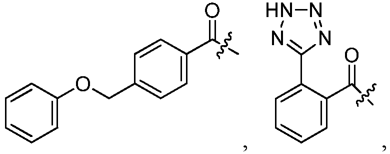 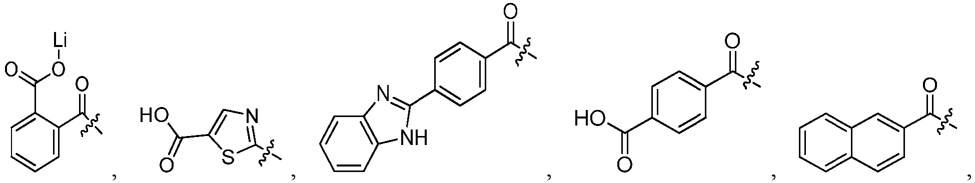 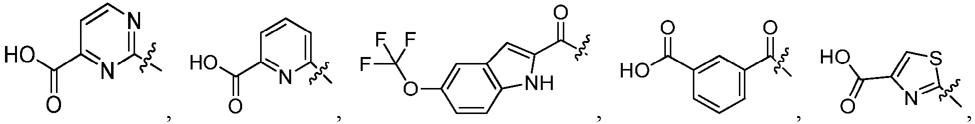 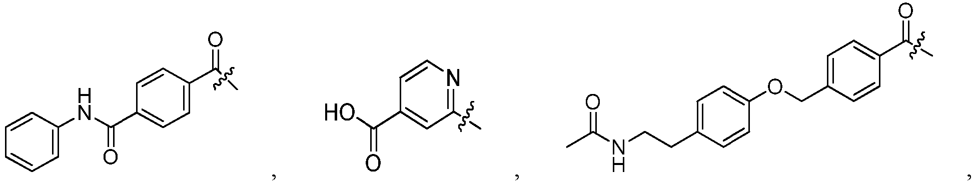 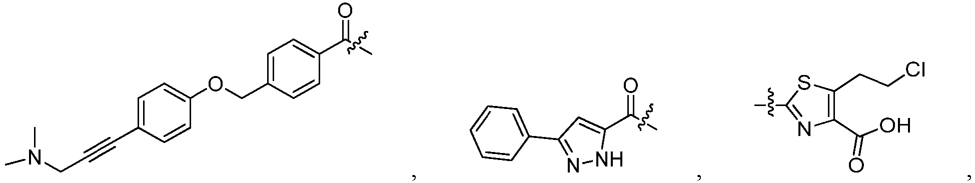 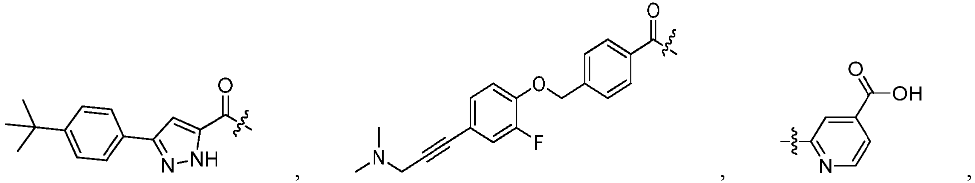 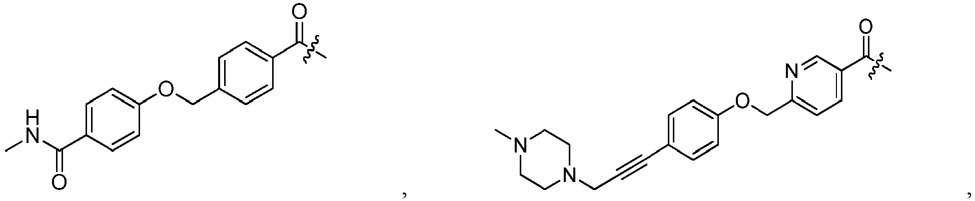 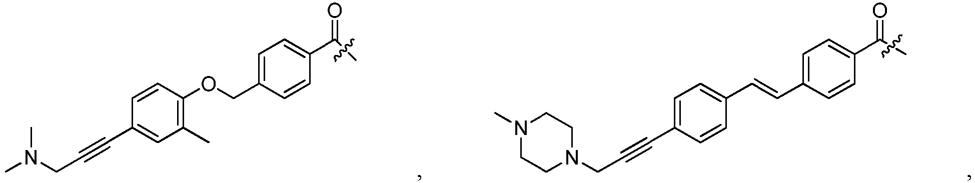 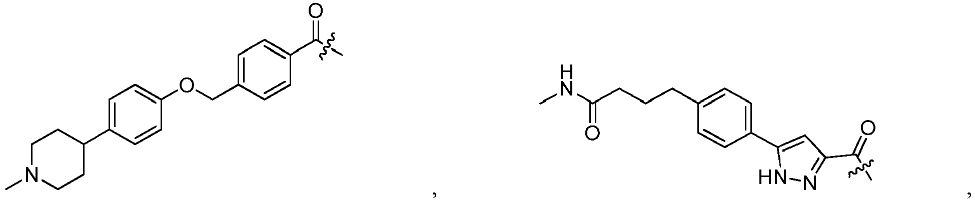 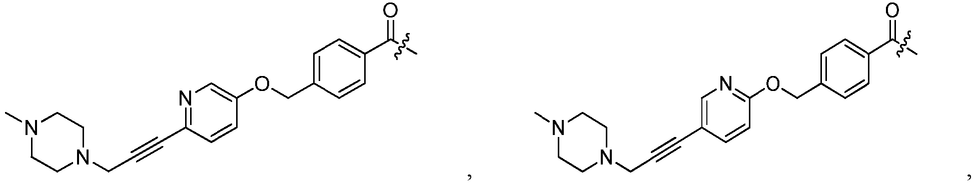 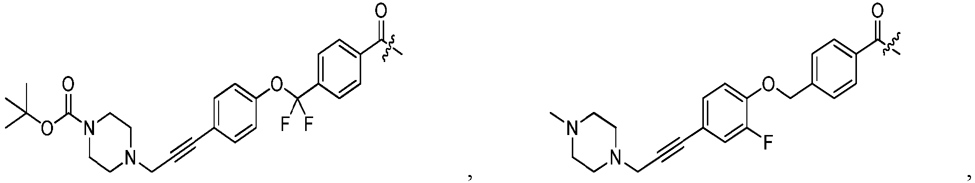 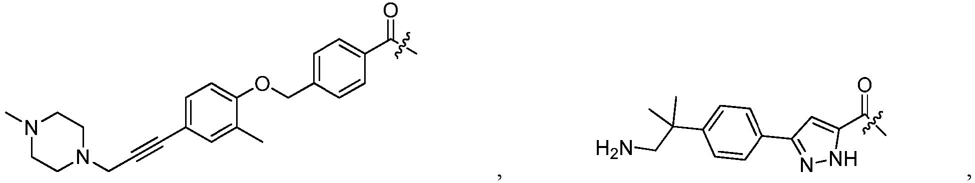 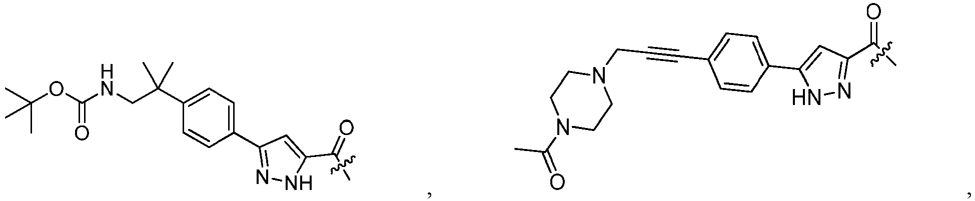 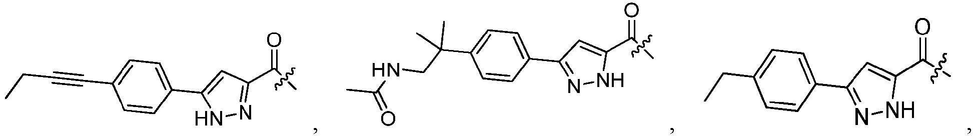 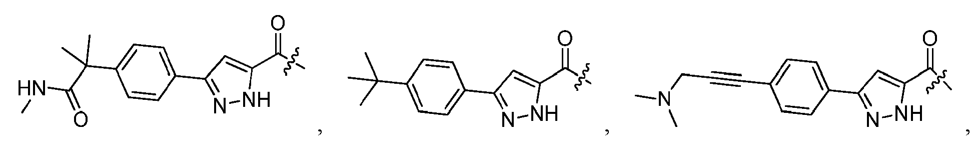 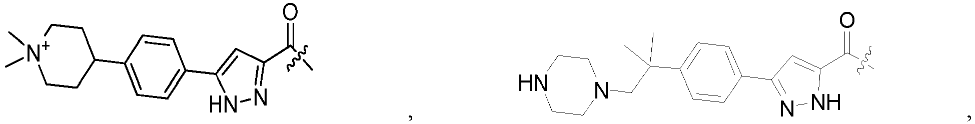 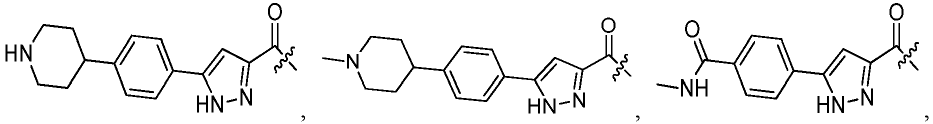 and

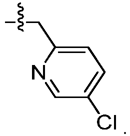

Further, R₃ is selected from the group consisting of 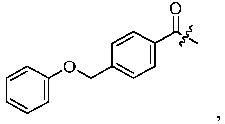 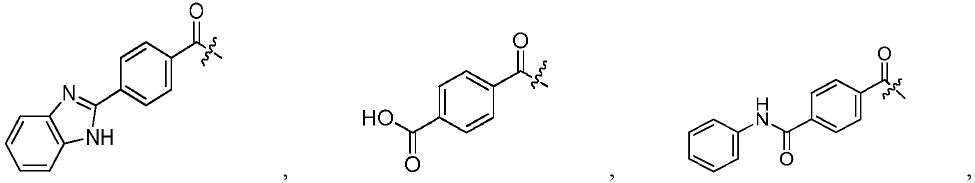 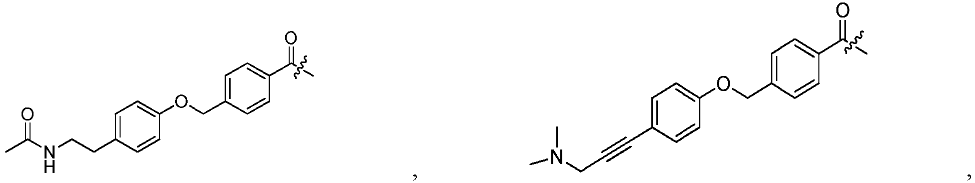 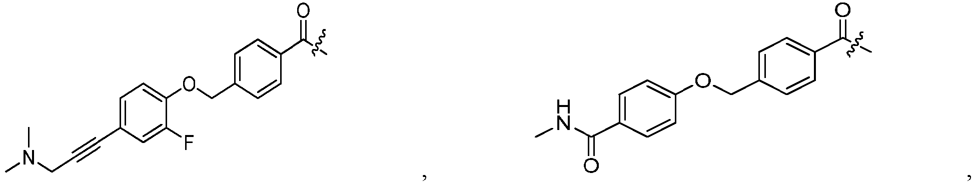 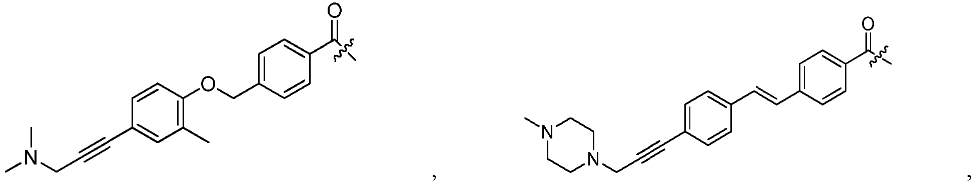 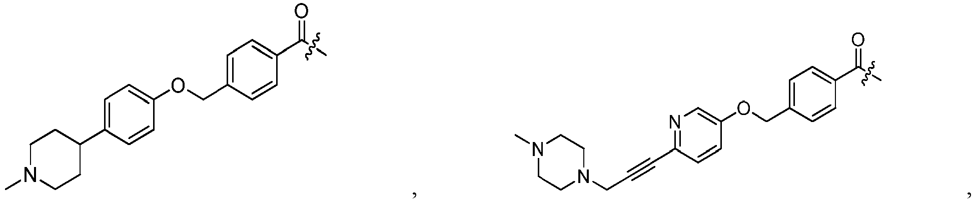 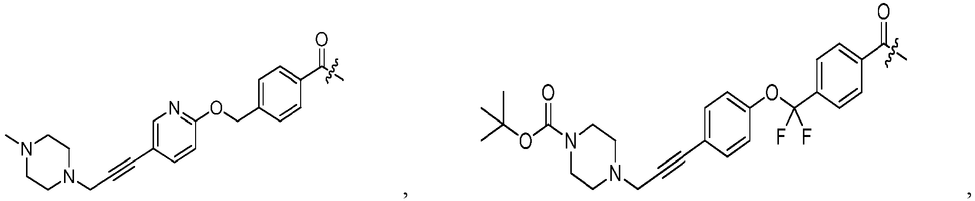 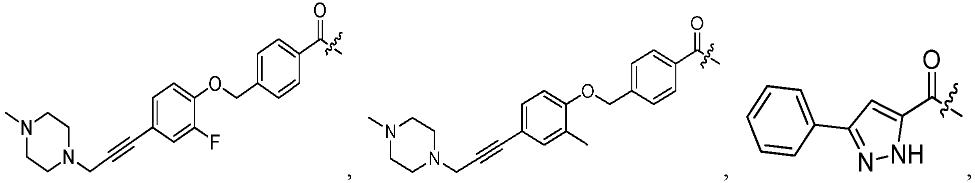 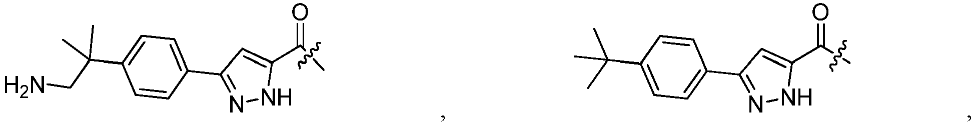 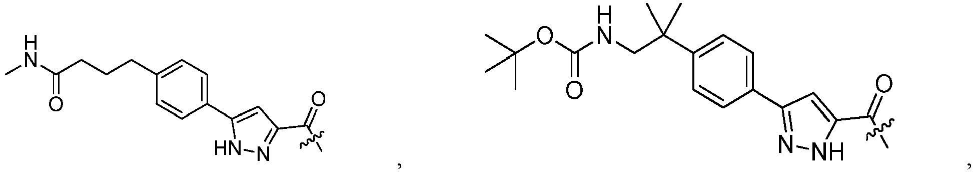 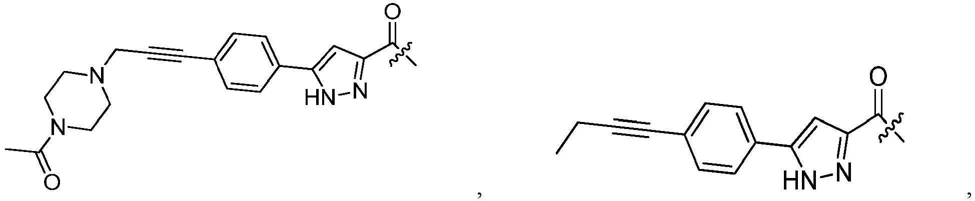 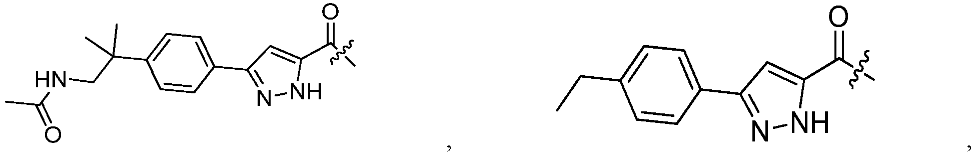 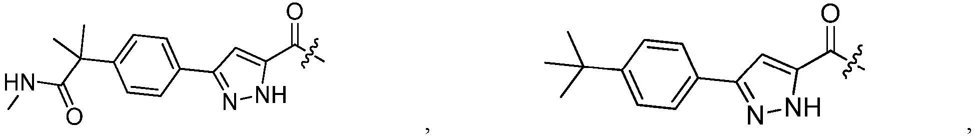 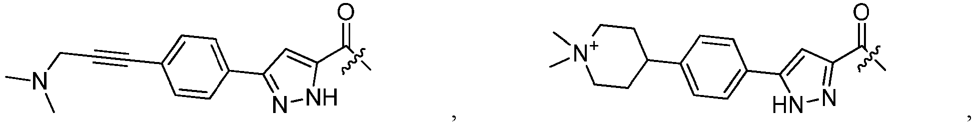 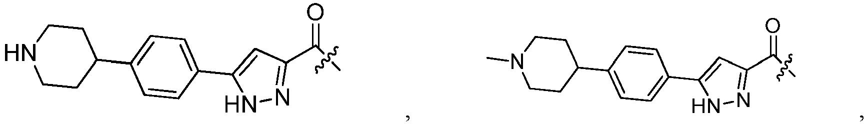 and

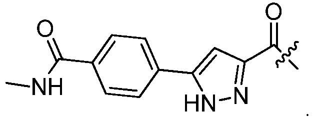

Further, the structure of the compound is shown in Formula III: 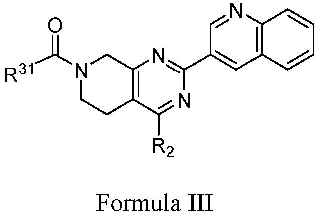 wherein, R³¹ and R₂ are as described above. Preferably, R₂ is selected from the group consisting of hydrogen, and

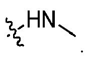

Further, R³¹ is selected from the group consisting of

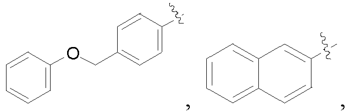

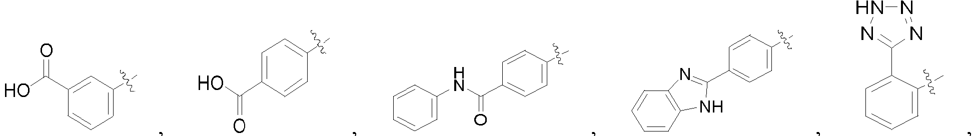

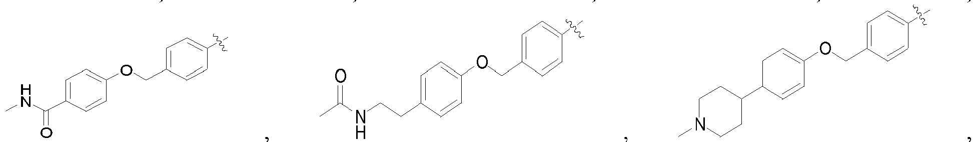

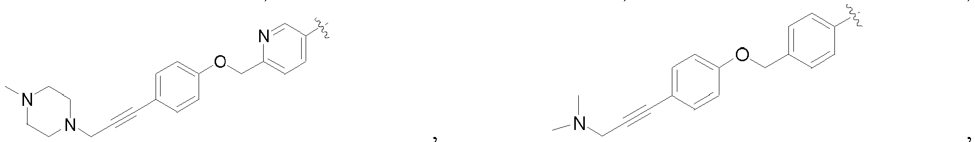

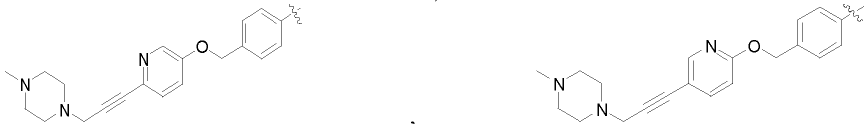

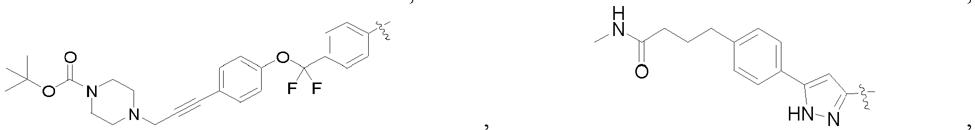

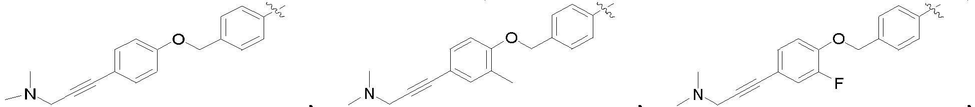

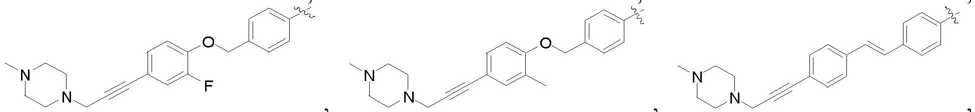

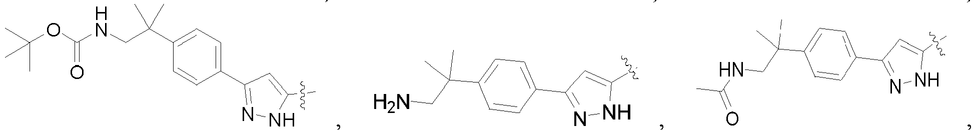

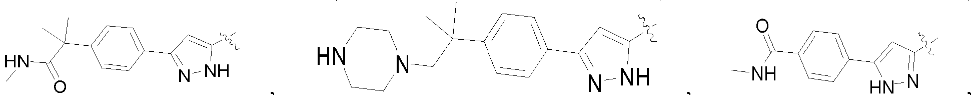

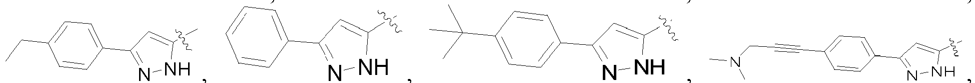

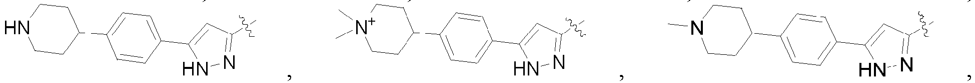

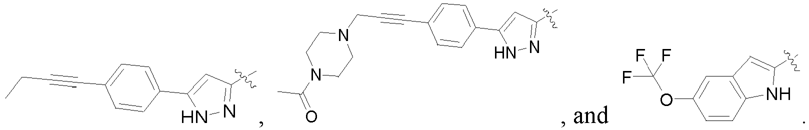

Further, R³¹ is selected from the group consisting of

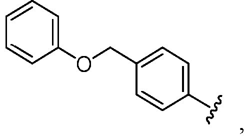

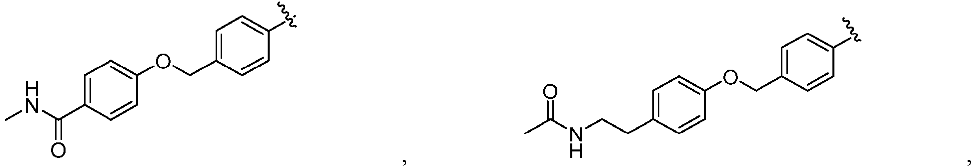

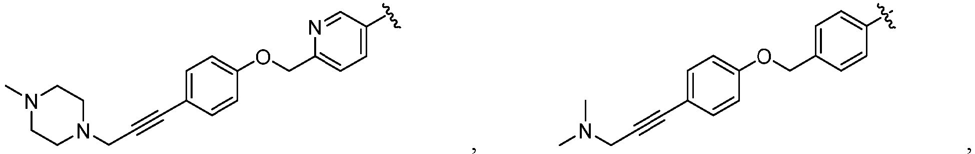

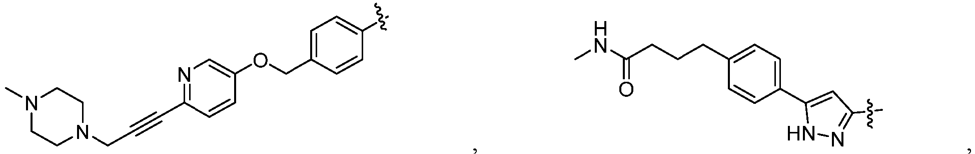

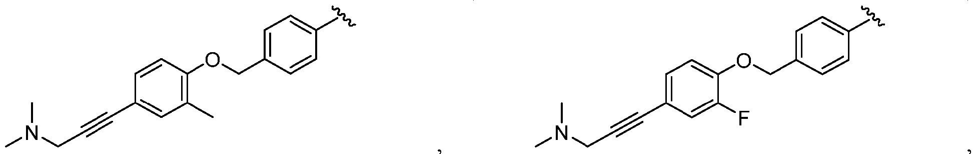

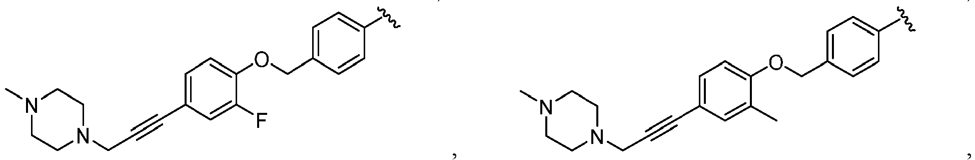

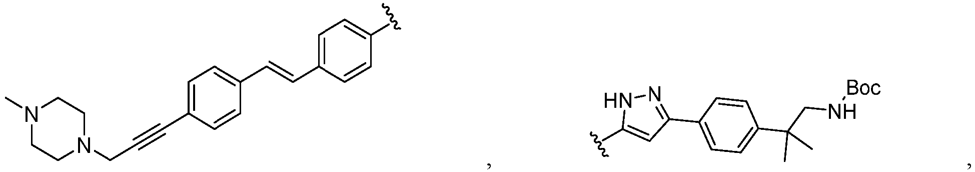

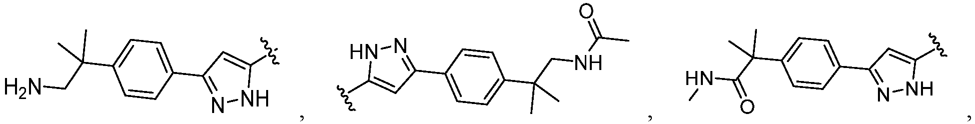

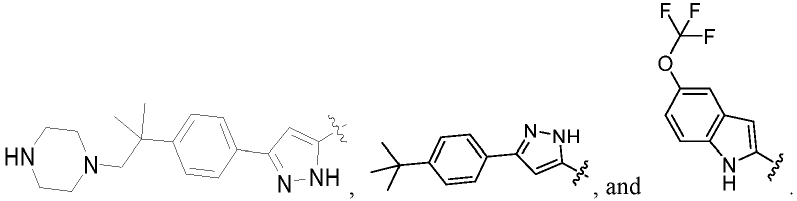

Further, the structure of the compound is selected from the group consisting of

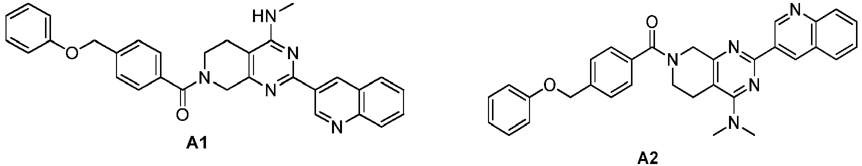

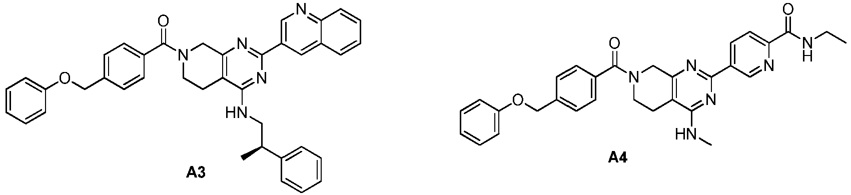

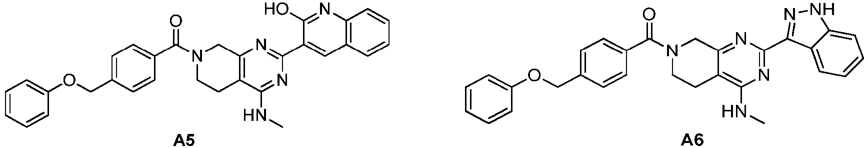

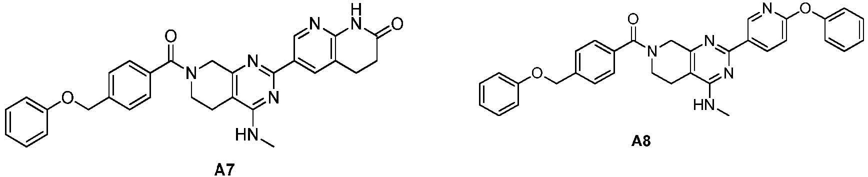

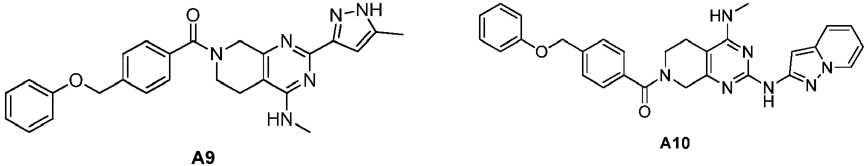

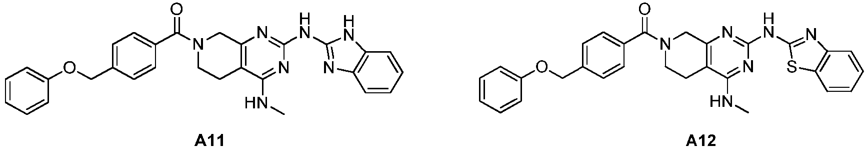

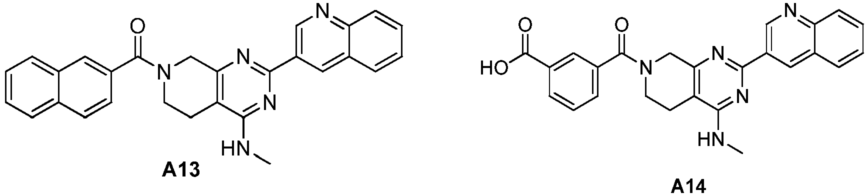

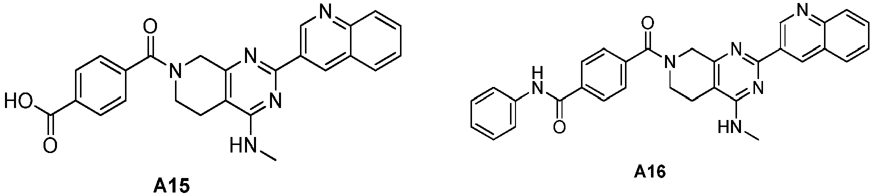

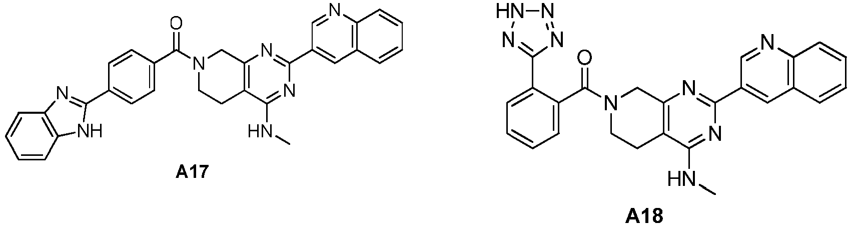

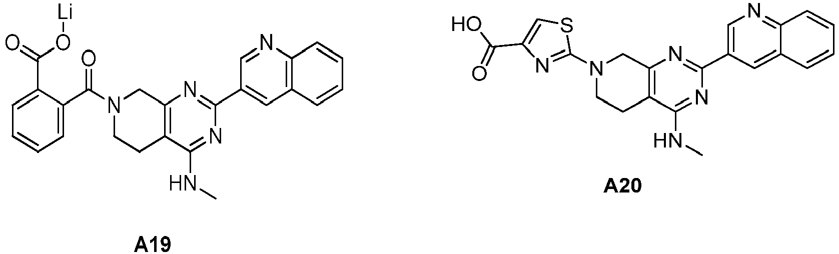

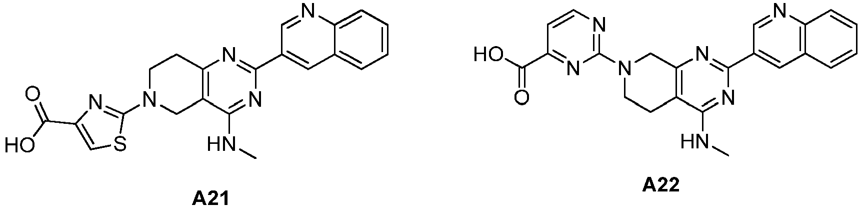

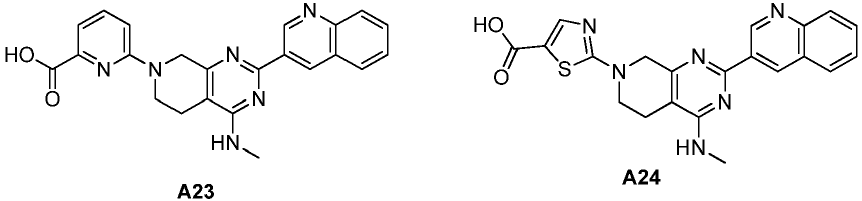

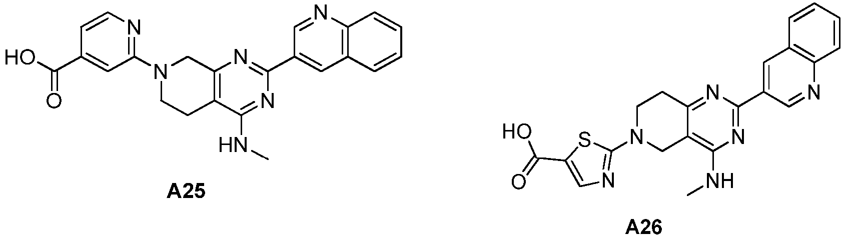

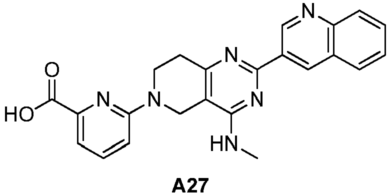

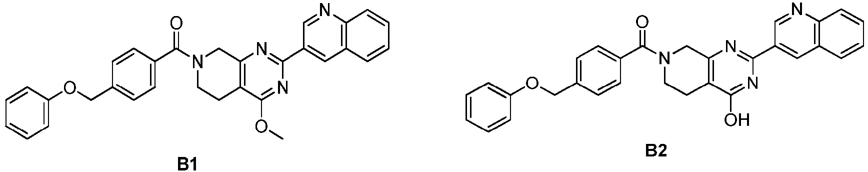

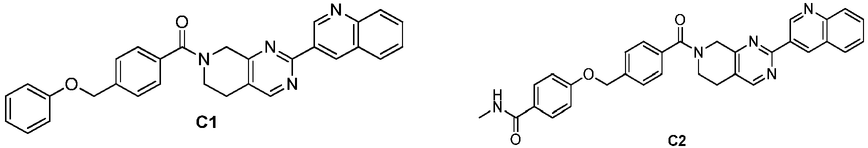

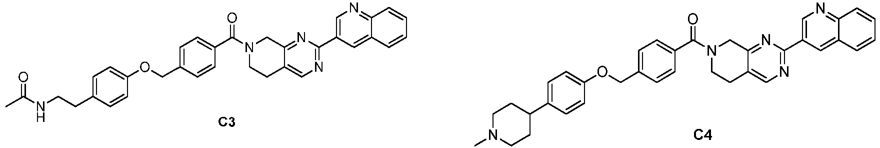

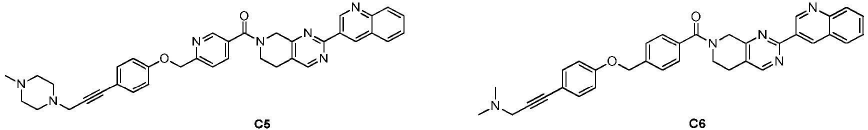

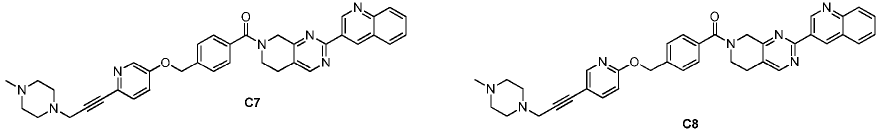

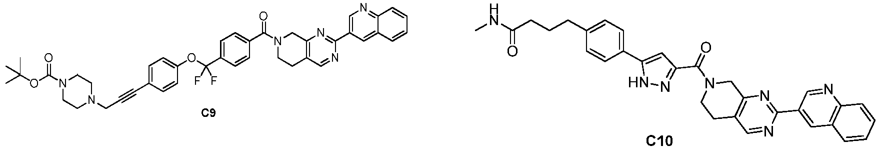

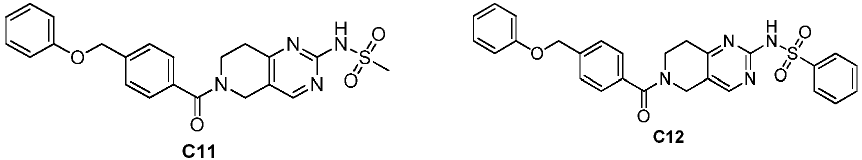

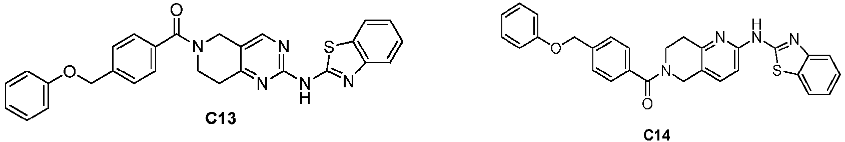

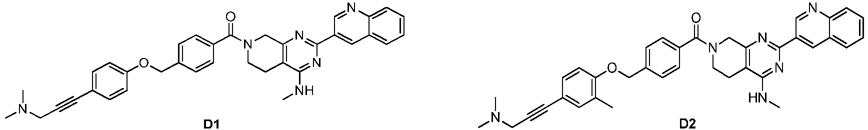

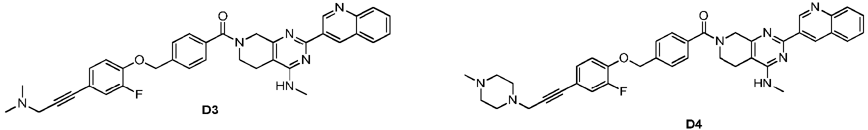

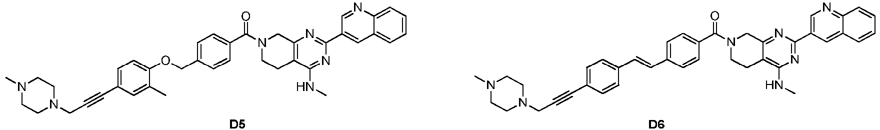

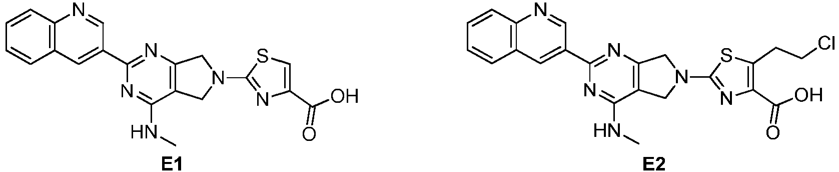

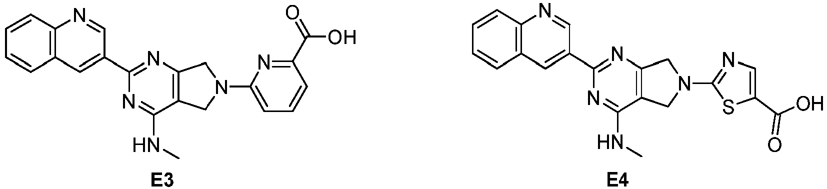

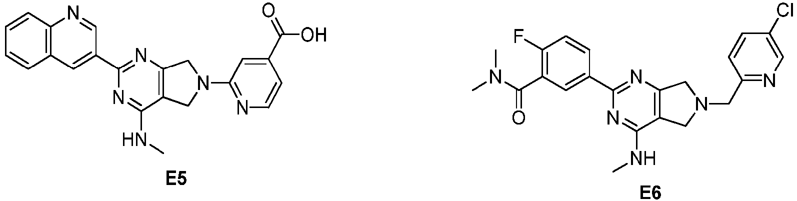

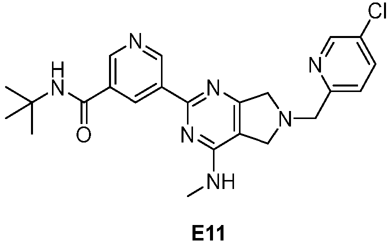

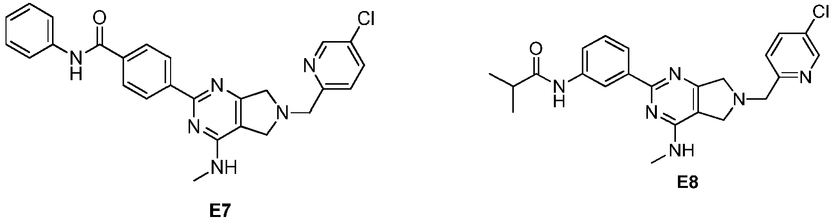

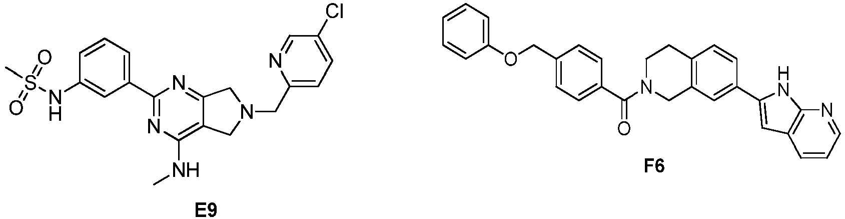

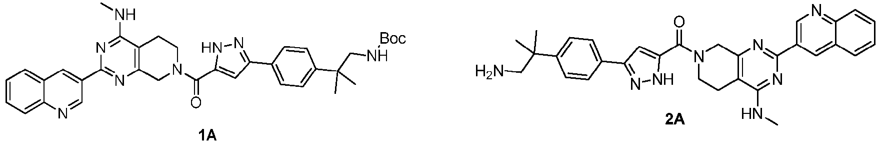

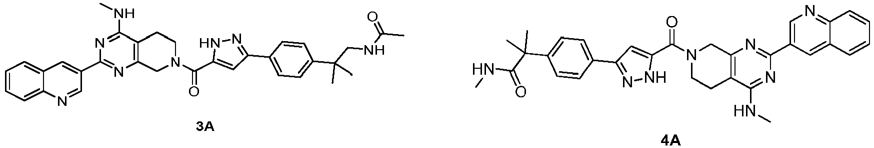

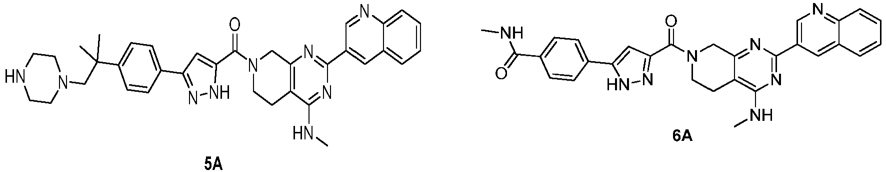

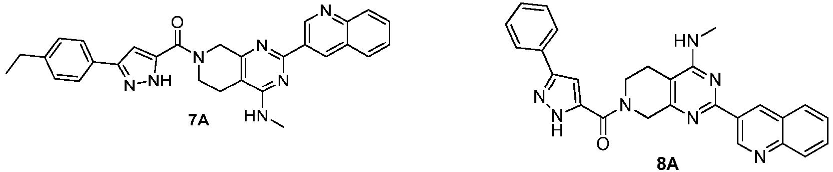

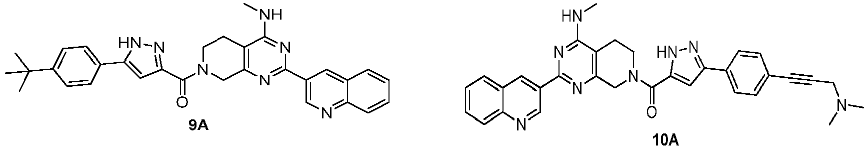

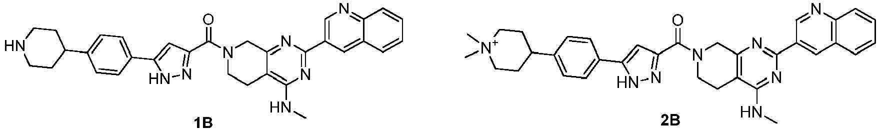

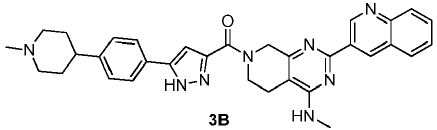

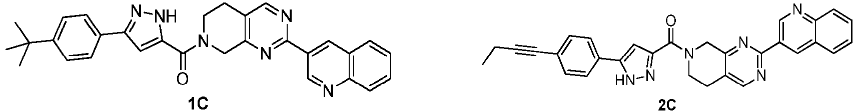

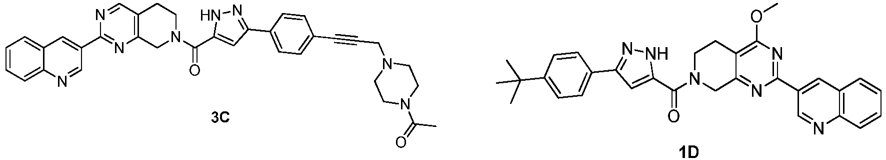

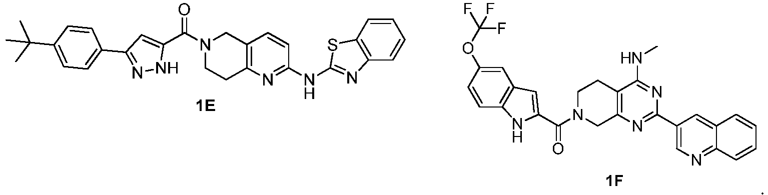

The present invention also provides a pharmaceutical composition, comprising any of the aforementioned compounds, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, and a pharmaceutically acceptable excipient, wherein any of the aforementioned compound, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof is an active ingredient.

The present invention also provides a use of the aforementioned compound, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, or the pharmaceutical composition in the manufacture of a BCL-XL inhibitor.

Further, the BCL-XL inhibitor is capable of binding to a BCL-XL protein.

Further, the BCL-XL inhibitor is a medicament for preventing and/or treating a disease related to BCL-XL protein activity.

Further, the disease related to BCL-XL protein activity is selected from the group consisting of autoimmune disease, bladder cancer, brain cancer, breast cancer, bone marrow cancer, cervical cancer, colorectal cancer, esophageal cancer, hepatocellular carcinoma, follicular lymphoma, lymphoid malignancy of T cell or B cell origin, melanoma, myeloma, oral cancer, ovarian cancer, leukemia, non-small cell lung cancer, prostate cancer, small cell lung cancer or and spleen cancer; the leukemia is preferably a chronic lymphocytic leukemia, a primitive lymphocytic leukemia or a granulocytic leukemia.

The compounds and derivatives provided in the present invention can be named according to the naming system of IUPAC (International Union of Pure and Applied Chemistry) or CAS (Chemical Abstracts Service, Columbus, OH).

Definitions of terms used in the present invention: Unless otherwise stated, the initial definitions provided herein for groups or terms are applied to the groups or terms throughout the description; for terms that are not specifically defined herein, their meanings should be that a person skilled in the art can give them according to the disclosure and context.

"Substitute" means that a hydrogen atom in a molecule is replaced by other different atom or molecule.

The minimum and maximum carbon atom content in a hydrocarbon group is indicated by a prefix, for example, the prefix C_{a~b} alkyl indicates any alkyl containing "a" to "b" carbon atoms. Thus, for example, "C_{1~6} alkyl" refers to an alkyl containing 1 to 6 carbon atoms.

"Alkyl" refers to a saturated hydrocarbon chain with a specified number of member atoms. For example, C_{1~6} alkyl refers to an alkyl group with 1 to 6 member atoms. Alkyl groups can be straight or branched. Representative branched alkyl groups have one, two or three branches. Alkyl groups can be optionally substituted with one or more substituents as defined herein. Alkyl comprises methyl, ethyl, propyl (n-propyl and isopropyl), butyl (n-butyl, isobutyl and tert-butyl), pentyl (n-pentyl, isopentyl and neopentyl) and hexyl. Alkyl groups can also be part of other groups, such as C₁~C₆ alkoxy groups.

"Alkenyl" refers to an unsaturated hydrocarbon chain having at least one carbon-carbon double bond. For example, C_{1~6} alkenyl refers to an alkenyl group having 1 to 6 carbon atoms. Alkenyl groups can be straight or branched. Representative branched alkenyl groups have one, two or three branches. Alkenyl groups can be optionally substituted with one or more substituents as defined herein. C_{1~6} alkenyl comprises ethenyl, propenyl (n-propenyl and isopropenyl), butenyl (n-butenyl, isobutenyl and tert-butenyl), pentenyl (n-pentenyl, isopentenyl and neopentenyl) and hexenyl.

"Alkynyl" refers to an unsaturated hydrocarbon chain having at least one carbon-carbon triple bond. For example, C₁₋₆ alkynyl refers to an alkynyl group having 1 to 6 carbon atoms. Alkynyl groups can be straight or branched. Representative branched alkynyl groups have one, two or three branches. Alkynyl groups may be optionally substituted with one or more substituents as defined herein. C₁₋₆ alkynyl comprises ethynyl, propynyl (n-propynyl and isopropynyl), butynyl (n-butynyl, isobutynyl and tert-butynyl), pentynyl (n-pentynyl, isopentenyl and neopentynyl) and hexynyl.

"Cycloalkyl", "carbocyclic ring" refers to a saturated or partially saturated cyclic group having 3 to 14 carbon atoms and no ring heteroatom and having a single ring or multiple rings (including fused, bridged and spiro ring systems). For polycyclic systems with aromatic and non-aromatic rings without ring heteroatoms, the term "cycloalkyl" applies when the point of attachment is at a non-aromatic carbon atom (e.g., 5,6,7,8,-tetrahydronaphthalen-5-yl). The term "cycloalkyl" comprises cycloalkenyl groups such as cyclohexenyl. Examples of cycloalkyl groups comprise, for example, adamantyl, cyclopropyl, cyclobutyl, cyclohexyl, cyclopentyl, cyclooctyl, cyclopentenyl, and cyclohexenyl. Examples of cycloalkyl groups comprising polybicycloalkyl ring system are bicyclohexyl, bicyclopentyl, bicyclooctyl, etc. Two such bicycloalkyl polycyclic structures are exemplified and named below: 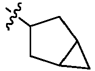 bicyclohexyl and 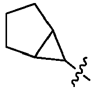 bicyclohexyl.

"Halogen" is fluorine, chlorine, bromine, or iodine.

"Halogen-substituted alkyl" means that hydrogen atoms in alkyl are substituted with one or more halogen atoms. For example, a halogen-substituted C_{1~6} alkyl refers to an alkyl containing 1 to 6 carbon atoms in which the hydrogen atoms are substituted with one or more halogen atoms.

"Heterocyclic ring" and "heterocycloalkyl" refer to a saturated ring or non-aromatic unsaturated ring that contains at least one heteroatom; wherein the heteroatom refers to nitrogen atom, oxygen atom, sulfur atom;

"Aryl" and "aromatic ring" can be used interchangeably, both referring to all-carbon ring with conjugated π electron system.

"Aromatic heterocyclic ring" refers to an aromatic unsaturated ring containing at least one heteroatom; wherein the heteroatom refers to nitrogen atom, oxygen atom, sulfur atom;

"Fused aromatic ring" refers to an aromatic unsaturated fused polycyclic ring. For example,

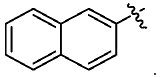

"Fused heteroaromatic ring" refers to an aromatic unsaturated fused polycyclic ring containing at least one heteroatom, such as: 8- to 12-membered fused heteroaromatic ring, the 8-to 12-membered fused heteroaromatic ring can be a fused ring of 6-membered heteroaromatic ring and C5 aliphatic ring, a fused ring of 6-membered heteroaromatic ring and 5-membered aromatic heterocyclic ring, a fused ring of 6-membered heteroaromatic ring and 5-membered heterocyclic ring, a fused ring of C₆ aromatic ring and 5-membered heteroaromatic ring, a fused ring of 6-membered heteroaromatic ring and 5-membered heteroaromatic ring, a fused ring of 6-membered heteroaromatic ring and 6-membered heteroaromatic ring, etc.

"Stereoisomer" comprises enantiomer and diastereomer;
"Tautomer" usually refers to structural isomers of different energies that can be converted into each other through a low energy barrier. For example, proton tautomer (also called prototropic tautomer) comprises interconversion via the migration of proton, such as keto-enol isomerization and imine-enamine isomerization. Valence tautomer comprises interconversion via the reorganization of some of bonding electrons.

"Polymorph" refers to a crystalline form of a compound (or salt, hydrate, or solvate thereof) that is in a specific crystal packing arrangement. All polymorphs have the same elemental composition. Different polymorphs typically have different X-ray diffraction patterns, infrared spectra, melting points, density, hardness, crystal shapes, optical and electrical properties, stability, and solubility. Recrystallization solvents, crystallization rates, storage temperatures, and other factors may cause one polymorph to dominate.

"Solvate" refers to a mixture formed by dissolving a compound in a solvent.

"N-oxide" is also called amine oxide and refers to a class of organic compounds with the general formula R₃N+-O- (also written as R₃N=O or R₃N→O).

"Isotope-labeled compound" refers to a molecule or group in which one or more atoms are replaced by their isotope atoms, for example, hydrogen atoms are replaced by deuterium atoms, in which the proportion of deuterium atoms is greater than the abundance of deuterium in nature; for example, ¹²C is replaced by ¹³C.

"Metabolite" refers to substances generated by chemical structure transformation under the action of body after a drug molecule is absorbed by the body.

"Prodrug" refers to a compound generated by modifying the chemical structure of drug, which is inactive or less active in vitro, and releases the active drug through enzymatic or non-enzymatic conversion in vivo to exert its efficacy.

The term "pharmaceutically acceptable" means that a vehicle, carrier, diluent, excipient, and/or formed salt is usually chemically or physically compatible with other ingredients constituting a drug dosage form, and physiologically compatible with the receptor.

The terms "salt" and "pharmaceutically acceptable salt" refer to an acidic and/or basic salt formed by the aforementioned compound or stereoisomer thereof with an inorganic and/or organic acid or base, including zwitterionic salt (inner salt), and also quaternary ammonium salt, such as alkylammonium salt. These salts can be obtained directly in the final separation and purification of the compound. They can also be obtained by mixing the aforementioned compound or stereoisomer thereof with a certain amount of acid or base appropriately (for example, equivalent amount). These salts may be precipitated in a solution and collected by filtration, or recovered after solvent evaporation, or freeze-dried after reaction in an aqueous medium.

The pharmaceutically acceptable accessory described in the present invention comprises pharmaceutically acceptable excipient, carrier, adjuvant, vehicle or any combination thereof.

In certain embodiments, one or more compounds of the present invention can be used in combination with each other. The compound of the present invention can also be used in combination with any other active agent to prepare a medicament or pharmaceutical composition for regulating cell function or treating a disease. If a group of compounds is used, the compounds can be administered to the subject simultaneously, separately or sequentially.

The compound provided by the present invention can effectively bind to BCL-XL protein, thereby further inhibiting the activity of BCL-XL protein. The compound of the present invention can be used to prepare BCL-XL inhibitor, as well as a medicament for preventing and/or treating a disease related to BCL-XL protein activity (e.g., cancer, autoimmune disease, etc.), and has broad prospects for clinical application.

The compound of the present invention has good pharmacological properties, good stability, high drugability, and the method for preparing the compound is simple with a high yield and low cost, making it suitable for industrial production.

The term "prevention" comprises inhibiting and delaying the onset of a disease, and comprises not only prevention before the development of a disease, but also prevention of recurrence of a disease after treatment.

The term "treatment" means reversing, alleviating or eliminating the progression of a disease or one or more symptoms of such disease or condition to which such term is applied.

Obviously, according to the above content of the present invention, according to the common technical knowledge and customary means in the art, without departing from the above basic technical ideas of the present invention, other forms of modification, replacement or change can also be made.

The following is a further detailed description of the above content of the present invention through specific models in the form of examples. However, this should not be understood as the scope of the above subject matter of the present invention being limited to the following examples. All technologies realized based on the above contents of the present invention belong to the scope of the present invention.

### Specific Models for Carrying Out the Invention

The known starting materials of the present invention could be synthesized by methods known in the art, or could be commercial products purchased from markets.

Unless otherwise specified in the examples, the reaction was carried out under a nitrogen atmosphere. Unless otherwise specified in the examples, the solution referred to an aqueous solution. Unless otherwise specified in the examples, the reaction temperature was room temperature. The room temperature was the most suitable reaction temperature, which was 20°C to 30°C. Unless otherwise specified in the examples, M was moles per liter.

The structure of the compound was determined by nuclear magnetic resonance (NMR) and mass spectrometry (MS). NMR shift (δ) was given in unit of 10⁻⁶ (ppm). NMR was measured using (Brµker AvanceIII 400 and Brµker Avance 600) nuclear magnetometers, and the measurement solvents were deuterated dimethyl sulfoxide (DMSO-d₆), deuterated chloroform (CDCl₃), deuterated methanol (Methol-*d₄*), and the internal standard was tetramethylsilane (TMS). LC-MS determination used Shimadzu LC-MS 2020 (ESI) instrument. HPLC determination used Shimadzu high pressure liquid chromatograph (Shimadzu LC-20A). MPLC (medium pressure preparative chromatography) used Gilson GX-281 reverse phase preparative chromatograph. Thin layer chromatography silica gel plates used were Yantai Huanghai HSGF254 or Qingdao GF254 silica gel plates, and the specifications used for thin layer chromatography separation and purification of products were 0.4 mm to 0.5 mm. Column chromatography generally used Yantai Huanghai silica gel (200~300 mesh silica gel) as the support.

The reagents described in the examples were abbreviated as follows: Pd(dppf)Cl₂: [1,1'-bis(diphenylphosphino)ferrocene]palladium dichloride; Pd(OAc)₃: palladium acetate; Pd₂(dba)₃: tris(dibenzylideneacetone)dipalladium; XantPhos: 4,5-bis(diphenylphosphino)-9,9-dimethylxanthene; S-Phos: 2-dicyclohexylphosphino-2',6'-dimethoxy-1,1'-biphenyl; BINAP: 1,1'-binaphthyl-2,2'-bisdiphenylphosphine; DIPEA: N,N-diisopropylethylamine; HATU: 2-(7-azobenzotriazole)-N,N,N',N'-tetramethyluronium hexafluorophosphate; HOBt: 1-hydroxybenzotriazole; EDCI: 1-(3-dimethylaminopropyl)-3-ethylcarbodiimide hydrochloride; DMAP: 4-dimethylaminopyridine; TCDI: N,N'-thiocarbonyldiimidazole; LiHMDS: lithium bis(trimethylsilyl)amide; DMF: N,N-dimethylamide; THF: tetrahydrofuran; DMSO: N,N-dimethyl sulfoxide; KOAc: potassium acetate; MIBK: methyl isobutyl ketone.

### Example 1, Synthesis of Compound A1 of Series A of the present invention:

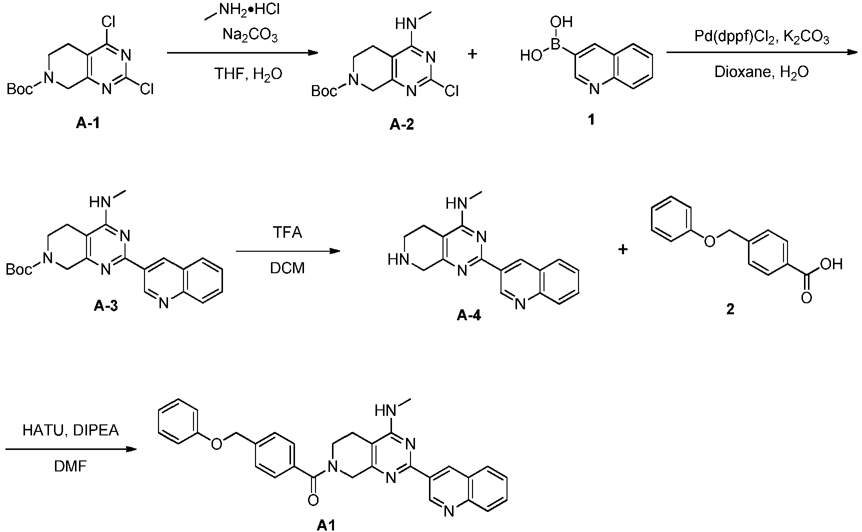

### Step 1, Synthesis of Compound A-2

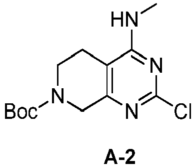

Compound A-1 (304.00 mg, 999.44 µmol), methylamine hydrochloride (80.98 mg, 1.20 mmol), sodium carbonate (317.82 mg, 3.00 mmol) and tetrahydrofuran/water (4 mL/2 mL) were added to a 50 mL reaction bottle, stirred at room temperature until the reaction was completed, and then water was added to quench the reaction (monitored by LC-MS). Saturated sodium chloride solution (10 mL) and ethyl acetate (3 X 15 mL) were used for extraction, the organic phases were combined, the resultant organic phase was dried over anhydrous sodium sulfate, evaporated to remove solvent, purified by MPLC, and concentrated under reduced pressure to remove solvent to obtain Compound A-2 (132.00 mg, 441.81 µmol, 44.21% yield), LCMS: C₁₃H₂₀ClN₄O₂, (ESI) [M+H]: 299.13.

### Step 2, Synthesis of Compound A-3

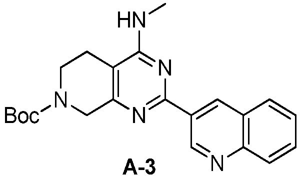

Under nitrogen protection, Compound A-2 (132.00 mg, 441.81 µmol), Compound 1 (91.18 mg, 530.18 µmol), potassium carbonate (182.91 mg, 1.33 mmol), 1,4-dioxane and water (4 mL/1 mL) and Pd(dppf)Cl₂ (6.44 mg, 0.25 mol%) were added to a 50 mL reaction bottle in sequence. The reaction system was sealed and stirred at 80°C for 2 hours, then the reaction was quenched (monitored by LC-MS). Saturated sodium chloride solution (20 mL) and ethyl acetate (3×20 mL) were used to complete the extraction, and the organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure for removal of solvent to obtain Compound A-3 (96.00 mg, 245.85 µmol, 55.65% yield). LCMS: C₂₂H₂₆N₅O₂, (ESI) [M+H]: 392.2.

### Step 3, Synthesis of Compound A-4

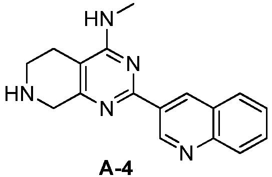

Compound A-3 (96.00 mg, 245.85 µmol) was dissolved in dichloromethane (2 mL) in a 50 mL reaction bottle, and then trifluoroacetic acid (1 mL) was added dropwise, stirred and reacted at room temperature for 3 hours, and then the solvent was removed by concentration under reduced pressure to obtain Compound A-4 (crude product). LCMS: C₁₇H₁₈N₅, (ESI) [M+H]: 292.2.

### Step 4, Synthesis of Compound A1

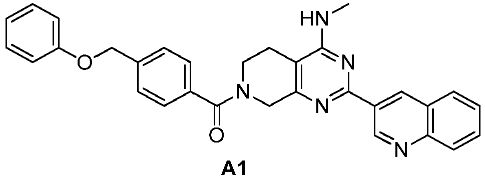

Compound 2 (28.00 mg, 122.68 µmol), DIPEA (47.56 mg, 368.03 µmol, 64.10 µL), HATU (46.62 mg, 122.68 µmol) and DMF (2 mL) were added to a 50 mL reaction bottle in sequence. After the temperature of the reaction system dropped to 0 °C, Compound A-4 (crude product) was added. After stirring for 2 hours under ice bath conditions, the reaction was quenched (monitored by LC-MS). Saturated sodium chloride solution (20 mL) and ethyl acetate (3×20 mL) were used for extraction. The organic phases were combined, dried over anhydrous sodium sulfate, evaporated to remove solvent, purified by MPLC, and concentrated under reduced pressure to remove solvent to obtain Compound A1 (20.00 mg, 39.75 µmol, 32.41% yield, 99.7% purity). LC-MS: C₃₁H₂₈N₅O₂, [M+H]⁺ 502.2; detected value: 502.2. ¹H NMR (400 MHz, DMSO-d₆) δ 9.82-9.11 (m, 2H), 8.16-7.99 (m, 2H), 7.97-7.80 (m, 2H), 7.55 (q, *J =* 8.0 Hz, 4H), 7.32 (t, *J* = 8.0 Hz, 2H), 7.04 (d, *J* = 8.2 Hz, 2H), 6.96 (t, *J* = 7.2 Hz, 1H), 5.18 (s, 2H), 4.71 - 4.51(m, 2H), 3.97 - 3.65 (m, 2H), 3.07 (d, *J* = 4.4 Hz, 3H), 2.56 (s, 2H).

According to the synthetic scheme of Compound **A1,** Compounds **A2** and **A3** could be obtained by replacing the raw material methylamine hydrochloride used in Step **1** with the raw material as listed in Table 1.

**Table 1. Compounds A2 and A3**

| Raw material | Product structure | H-NMR data characterization |
|---|---|---|
| 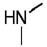 | 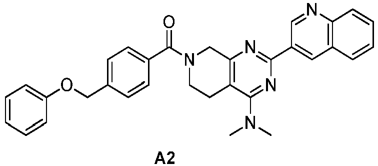 (LC-MS: C₃₂H₃₀N₅O₂, [M+H]⁺ 516.2; detected value: 516.4; purity: 96.4%) | ¹H NMR (400 MHz, Methanol-*d*₄) δ 9.81 (s, 1H), 9.59 (s, 1H), 8.31 (s, 1H), 8.21 (d, *J* = 8.0 Hz, 1H), 8.05 (t, *J* = 8.0 Hz, 1H), 7.98 (d, *J* = 7.6 Hz, 1H), 7.90 (t, *J* = 4.0 Hz, 1H), 7.63-7.58 (m, 4H), 7.57 (d, 4.2 Hz, 1H), 7.30-7.25 (m, 3H), 7.01-6.93 (m, 4H), 5.17 (s, 2H), 3.37 (s, 6H), 3.05 (s, 2H). |
| 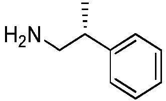 | 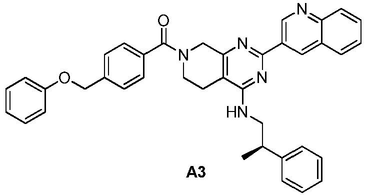 (LC-MS: C₃₉H₃₆N₅O₂, [M+H]⁺ 606.3; detected value: 606.3; purity: 98.3%) | ¹H NMR (400 MHz, Methanol-*d*₄) δ 9.72 (s, 1H), 9.40 (s, 1H), 8.18 (d, *J* = 1.2Hz, 2H), 8.03 (t, *J* = 2.4 Hz, 1H), 7.84 (s, 1H), 7.62-7.53 (m, 4H), 7.33-7.26 (m, 6H), 7.17-7.13 (m, 1H), 7.01-6.92 (m, 3H), 5.17 (s, 2H), 4.84 (s, 2H), 4.67 (s, 1H), 4.09 (s, 1H), 3.96 (dd, *J* = 12.0, 8.0 Hz, 1H), 3.79 (dd, *J* = 12.0, 8.0 Hz, 2H), 2.57 (s, 2H), 1.40 (d, *J* = 6.8 Hz, 3H). |

According to the synthetic scheme of Compound **A1** above, Compounds **A4** to **A12** could be obtained by replacing Compound **1** with the raw material as listed in Table 2.

**Table 2. Compounds A4 to A12**

| Raw material | Product structure | H-NMR data characterization |
|---|---|---|
| 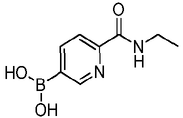 | 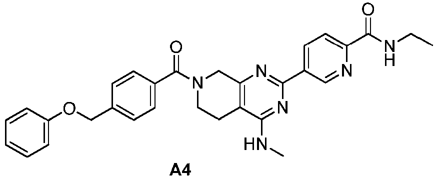 (LC-MS: C₃₀H₃₁N₆O₃, [M+H]⁺ 523.2; detected value: 523.3; purity: 99.9%) | ¹H NMR (400 MHz, Methanol-*d*₄) δ 9.45 (s, 1H), 8.75-8.69 (m, 1H), 8.20 (s, 1H), 7.60 (d, *J* = 8.0 Hz, 2H), 7.53 (d, *J* = 8.0 Hz, 2H), 7.27 (t, *J=* 7.8 Hz, 2H), 7.00 (d, *J* = 8.0 Hz, 2H), 6.94 (t, *J* = 8.0 Hz, 1H), 5.17 (s, 2H), 4.86 (s, 1H), 4.66 (s, 1H), 4.012-4.09 (m, 1H), 3.80 (s, 1H), 3.48 (s, 2H), 3.18 (s, 3H), 2.63 (s, 2H), 1.26 (t, *J* = 7.6 Hz, 3H). |
| 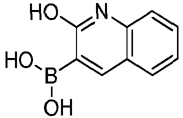 | 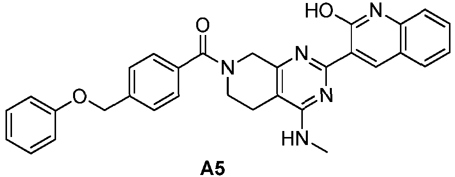 (LC-MS: C₃₁H₂₈N₅O₃, [M+H]⁺ 518.2; detected value: 518.1; purity: 94.4%) | ¹H NMR (400 MHz, Methanol-*d*₄) δ 8.02 (d, *J* = 8.0 Hz, 1H), 7.80 (t, *J =* 7.9 Hz, 1H), 7.62 (d, *J* = 7.9 Hz, 2H), 7.56 (d, *J* = 8.0 Hz, 2H), 7.51-7.42 (m, 3H), 7.28 (t, *J* = 7.8 Hz, 2H), 7.01-6.92 (m, 3H), 5.18 (s, 2H),3.48 (s, 2H), 2.13 (s, 2H), 2.67 (s, 3H), 2.03 (d, *J* = 4.0 Hz, 2H). |
| 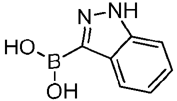 | 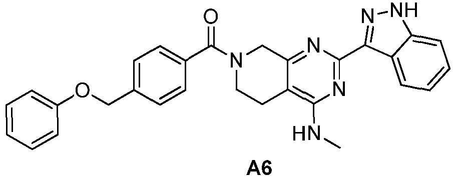 (LC-MS: C₂₉H₂₇N₆O₂, [M+H]⁺ 491.2; detected value: 491.2; purity: 90.4%) | ¹H NMR (400 MHz, DMSO-d₆) δ 8.62-8.58 (m, 1H), 7.58-7.51 (m, 5H), 7.40-7.24 (m, 4H), 7.04 (d, *J* = 8.0 Hz, 2H), 6.97 (t, *J* = 6.8 Hz,1H), 5.18 (s, 2H), 4.71-4.49 (m, 4H),3.94 (s, 2H), 3.07 (s, 3H). |
| 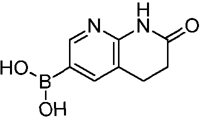 | 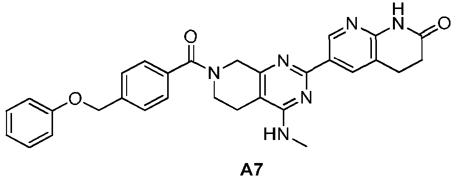 (LC-MS: C₃₀H₂₉N₆O₃, [M+H]⁺ 521.2; detected value: 521.2; purity: 95.6%) | ¹H NMR (400 MHz, Methanol-*d*₄) δ 8.97 (s, 1H), 8.37 (s, 1H), 7.61 (d, *J* = 7.8 Hz, 2H), 7.55 (d, *J* = 7.9 Hz, 2H), 7.27 (t, *J =* 7.8 Hz, 2H), 7.03-6.92 (m, 3H), 5.17 (s, 2H), 4.79 (s, 2H), 4.08-3.81 (m, 2H), 3.22 (s, 3H), 3.13-3.04 (m, 2H), 2.71-2.63 (m, 4H). |
| 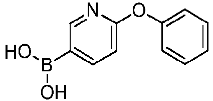 | 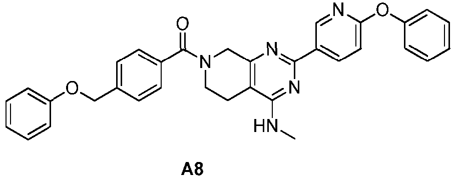 (LC-MS: C₃₃H₃₀N₅O₃, [M+H]⁺ 544.2; detected value: 544.5; purity: 99.9%) | ¹H NMR (400 MHz, Methanol-*d*₄) δ 9.08-8.97 (m, 1H), 8.69-8.60 (m, 1H), 7.58 (d, *J* = 7.2 Hz, 2H), 7.50 (d, *J* = 8.0 Hz, 2H), 7.43 (t, *J* = 4.4 Hz, 2H), 7.27 (t, *J* = 8.0 Hz, 3H), 7.15 (s, 2H), 7.00-6.91 (m 4H), 5.15 (s, 2H), 4.75 (s, 1H), 4.58-4.52 (m, 1H), 4.07 (s, 1H), 3.73 (s, 1H), 3.07 (s, 3H), 2.59-2.53 (m, 2H). |
| 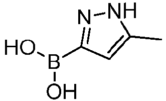 | 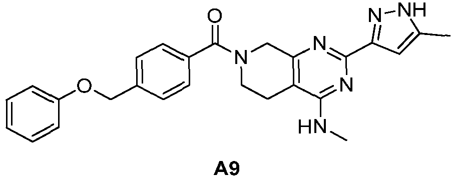 (LC-MS: C₂₆H₂₇N₆O₂, [M+H]⁺ 455.2; detected value: 455.3; purity: 99.4%) | ¹H NMR (400 MHz, Methanol-*d*₄) δ 7.60 (d, *J* = 4.2 Hz, 2H), 7.54 (d, *J* = 8.2 Hz, 2H), 7.27 (t, *J* = 4.4 Hz, 2H), 6.99 (d, *J* = 4.0 Hz, 2H), 6.96 (t, *J* = 7.2 Hz, 1H), 6.86 (s, 1H), 5.16 (s, 2H), 4.86 (s, 2H), 4.06-3.79 (m, 2H), 3.23 (s, 3H), 2.61 (s, 2H), 2.68 (s, 3H). |
| 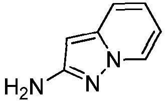 | 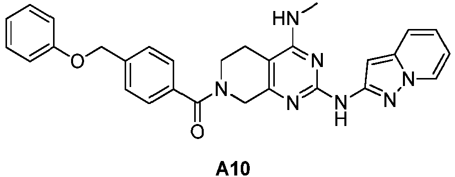 (LC-MS: C₂₉H₂₈N₇O₂, [M+H]⁺ 506.2; detected value: 506.2; purity: 99.6%) | ¹H NMR (400 MHz, Methanol-*d*₄) δ 8.62 (s, 1H), 7.62-7.60 (m, 2H), 7.57-7.54 (m, 3H), 7.27 (td, *J* = 7.2, 2.0 Hz, 3H), 7.00 (d, *J* = 8.1 Hz, 2H), 6.96-6.89 (m, 2H), 6.37 (s, 1H), 5.17(s, 2H), 4.85 (s, 2H), 3.79-3.73 (m, 2H), 3.13 (s, 3H), 2.54 (s, 2H). |
| 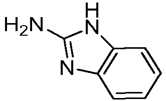 | 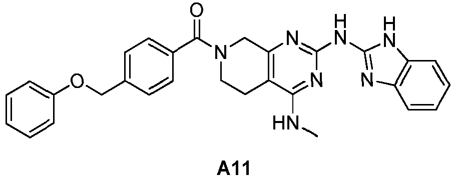 (LC-MS: C₂₉H₂₈N₇O₂, [M+H]⁺ 506.2; detected value: 506.4; purity: 92.0%) | ¹H NMR (400 MHz, Methanol-*d*₄) δ 7.60 (d, *J* = 8.0 Hz, 2H), 7.53 (t, *J* = 4.2 Hz, 2H), 7.43-7.36 (m, 3H), 7.35-7.23 (m, 2H), 6.99 (d, *J* = 8.0 Hz, 2H), 6.93 (t, *J* = 8.0 Hz, 1H), 5.16 (s, 2H), 4.75-4.63 (m, 2H), 4.08-3.78 (m, 2H), 3.14 (s, 3H), 2.58 (s, 2H). |
| 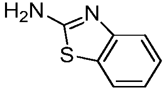 | 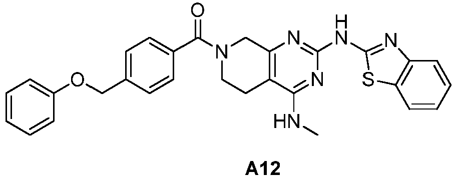 (LC-MS: C₂₉H₂₇N₆O₂S, [M+H]⁺ 523.2; detected value: 523.4; purity: 96.3%) | ¹H NMR (400 MHz, Methanol-*d*₄) δ 7.87 (d, *J* = 8.0 Hz, 1H), 7.60-7.44 (m, 6H), 7.37-7.23 (m, 3H), 7.05-6.99 (m, 3H), 5.17 (s, 2H), 4.68-4.50 (m, 2H), 3.64 (s, 2H), 3.19 (s, 3H), 2.54 (s, 2H). |

According to the synthetic scheme of Compound **A1** above, Compounds **A13** to **A19** could be obtained by replacing Compound **2** with the raw material as listed in Table 3.

**Table 3. Compounds A13 to A19**

| Raw material | Product structure | H-NMR data characterization |
|---|---|---|
| 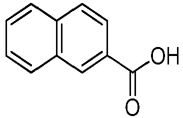 | 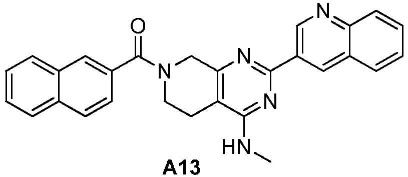 (LC-MS: C₂₈H₂₄N₅O, [M+H]⁺ 446.2; detected value: 446.2; purity: 99.3%) | ¹H NMR (400 MHz, Methanol-*d*₄) δ 9.82 (s, 1H), 9.48 (s, 1H), 8.45-8.19 (m, 2H), 8.11-7.91 (m, 5H), 7.85 (s, 1H), 7.65-7.54 (m, 3H), 4.92 (s, 1H), 4.65 (s, 1H), 4.25 (s, 1H), 3.86 (s, 1H), 3.24 (s, 3H), 2.67 (s, 2H). |
| 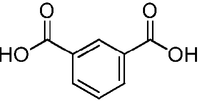 | 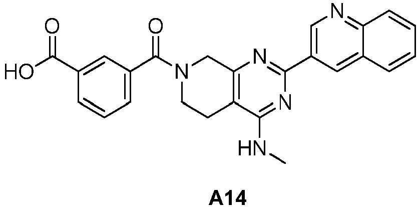 (LC-MS: C₂₅H₂₂N₅O₃, [M+H]⁺ 440.2; detected value: 440.3; purity: 99.0%) | ¹H NMR (400 MHz, Methanol-*d*₄) δ 9.75 (s, 1H), 9.50-9.48 (m, 1H), 8.25-8.23 (m, 1H), 8.19-8.16 (m, 3H), 8.05 (t, *J* = 8.0 Hz, 1H), 7.87-7.85 (m, 1H), 7.83-7.76 (m, 1H), 7.64 (t, *J* = 7.6 Hz, 1H), 4.89 (s, 1H), 4.68-4.62 (m, 1H), 4.14 (s, 1H), 3.78 (s, 1H), 3.23 (s, 3H), 2.64 (s, 2H). |
| 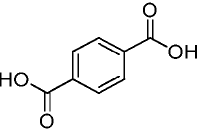 | 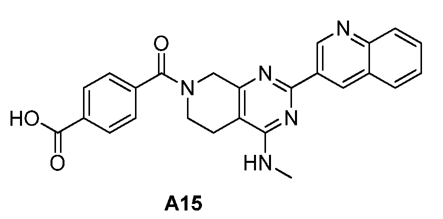 (LC-MS: C₂₅H₂₂N₅O₃, [M+H]⁺ 440.2; detected value: 440.2; purity: 98.8%) | ¹H NMR (400 MHz, Methanol-*d*₄) δ 9.77-9.72 (m, 1H), 9.52-9.48 (s, 1H), 8.27-8.22 (m, 1H),8.16 (d, *J* = 8.0 Hz, 3H), 8.04 (s, 1H), 7.86-7.81 (m, 1H), 7.62 (d, *J* = 4.0 Hz, 2H), 4.92 (s, 1H), 4.63 (s, 1H), 4.14 (s, 1H), 3.76 (s, 1H), 3.23 (s, 3H), 2.68-2.61 (m, 2H). |
| 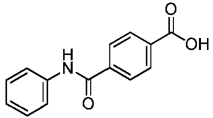 | 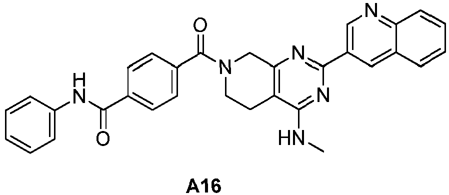 (LC-MS: C₃₁H₂₇N₆O₂, [M+H]⁺ 515.2; detected value: 515.4; purity: 95.5%) | ¹H NMR (400 MHz, DMSO-d₆) δ 10.37 (s, 1H), 9.84-9.72 (m, 1H), 9.24-9.12 (m, 1H), 8.20-8.15 (m, 1H), 8.12 (d, *J* = 8.0 Hz, 3H), 8.05 (d, *J* = 8.0 Hz, 3H), 7.86-7.78 (m, 3H), 7.65 (d, *J* = 8.0 Hz, 3H), 7.37 (t, *J* = 8.0 Hz, 2H), 7.29-7.22 (m, 1H), 7.12 (t, *J* = 8.0 Hz, 1H), 4.76 (s, 1H), 4.51 (s, 1H), 4.00 (s, 1H), 3.66 (s, 1H), 3.08 (d, *J* = 4.0 Hz, 3H), 2.56 (s, 2H). |
| 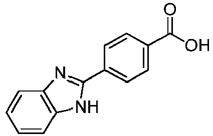 | 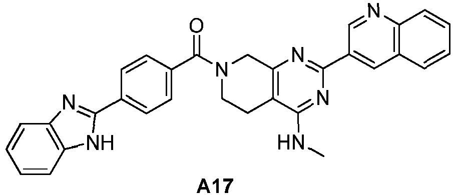 (LC-MS: C₃₁H₂₆N₇O, [M+H]⁺ 512.2; detected value: 512.4; purity: 98.3%) | ¹H NMR (400 MHz, Methanol-*d*₄) δ 9.82-9.73 (m, 1H), 9.73-9.41 (m, 1H), 8.28-8.19 (m, 4H), 8.03-7.95 (m, 1H), 7.87-7.82 (m, 5H), 7.61-7.59 (m, 2H), 4.94 (s, 1H), 4.67 (s, 1H), 4.17 (s, 1H), 3.93 (s, 1H), 3.23 (s, 3H), 2.69 (s, 2H). |
| 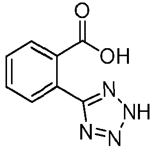 | 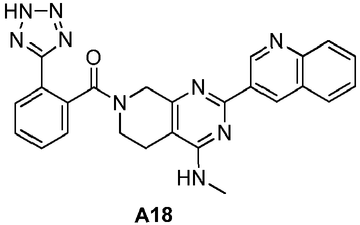 (LC-MS: C₂₅H₂₂N₉O, [M+H]⁺ 464.2; detected value: 464.3; purity: 99.1%) | ¹H NMR (400 MHz, Methanol-*d*₄) δ 9.76-9.66 (m, 1H), 9.48-9.39 (m, 1H), 8.28-8.17 (m, 2H), 8.15-7.97 (m, 2H), 7.95-7.78 (m, 1H), 7.79-7.65 (m, 2H), 7.63-7.60 (m, 1H), 4.95 (s, 1H), 4.45 (s, 1H), 3.68 (s, 1H), 3.26-3.23 (m, 3H), 2.75-2.49 (m, 2H). |
| 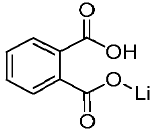 | 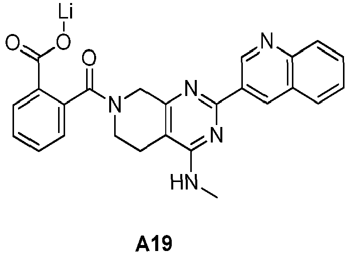 (LC-MS: C₂₅H₂₁LiN₅O₃, [M+H]⁺ 446.2; detected value: 440.3; purity: 83.8%) | ¹H NMR (400 MHz, DMSO-d₆) δ 9.83-9.70 (m, 1H), 9.18-9.07 (m, 1H), 8.18-8.02 (m, 2H), 7.94-7.52 (m, 4H), 7.34 (td, *J* = 8.0, 2.8 Hz, 2H), 7.09 (dq, *J* = 12.0, 6.2 Hz, 1H), 4.72-4.67 (m, 1H), 4.31-4.02 (m, 2H), 3.67-3.65 (m, 1H), 3.05 (d, *J* = 5.7 Hz, 3H), 2.67-2.57 (m, 1H), 2.34-2.25 (m, 1H). |

### Synthesis method of Compound A20 of Series A of the present invention:

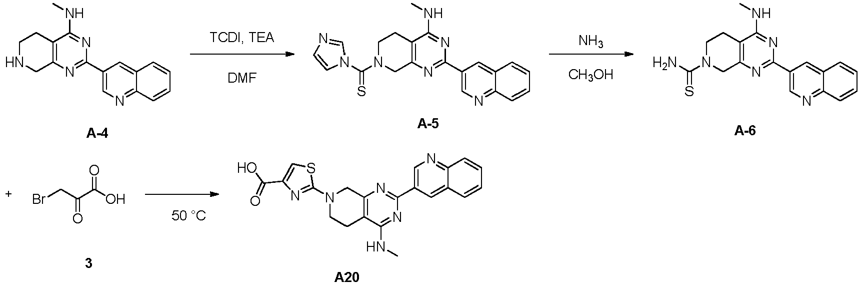

### Step 1, Synthesis of Compound A-5

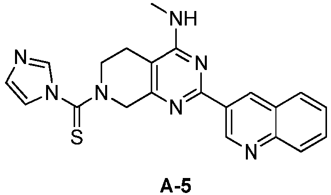

Compound A-4 (80.00 mg, 274.58 µmol), TCDI (58.72 mg, 329.50 µmol) and DMF (5 mL) were added to a 50 mL reaction bottle in sequence. After stirring for 10 minutes, triethylamine (111.14 mg, 1.10 mmol, 153.19 µL) was added. The reaction was kept at room temperature for 1 hour and then quenched (monitored by LC-MS). The extraction was completed with saturated sodium chloride solution (20.0 mL) and dichloromethane (3×15 mL). The organic phases were combined and dried over anhydrous sodium sulfate. After the solvent was removed by concentration under reduced pressure, Compound A-5 (crude product) was obtained. LCMS: C₂₁H₂₀N₇S, (ESI) [M+H]: 402.2.

### Step 2, Synthesis of Compound A-6

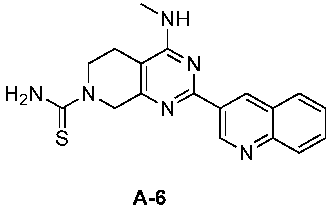

Compound **A-5** (crude product) and methylamine alcohol solution (7 N, 1 mL) were added to a 50 mL reaction bottle, reacted at room temperature overnight and then quenched (monitored by LCMS). After the excess methylamine alcohol solution was removed by concentration under reduced pressure, Compound A-6 (crude product) was obtained. LCMS: C₁₈H₁₉N₆S, (ESI) [M+H]: 351.1.

### Step 3, Synthesis of Compound A20

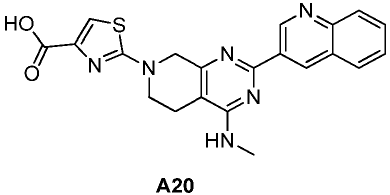

Compound **A-6** (crude product), Compound **3** (38.11 mg, 228.28 µmol) and ethanol (2 mL) were added to a 50 mL reaction bottle, the reaction was maintained at 50 °C for about 2 hours until the reaction was completed (monitored by LC-MS), the reaction solution was removed by concentration under reduced pressure, and purified by MPLC to obtain Compound **A20** (27.00 mg, 64.52 µmol, 28.26% yield, 92.0% purity). LC-MS: C₂₁H₁₉N₆O₂S, [M+H]⁺ 419.1; detected value: 419.2. ¹H NMR (400 MHz, DMSO-*d₆*) δ 13.17 (s, 1H), 9.81 (dd, *J* = 9.3, 2.1 Hz, 1H), 9.19 (dd, *J* = 15.5, 2.1 Hz, 1H), 8.17 (d, *J* = 8.2 Hz, 1H), 8.08 (d, *J* = 8.5 Hz, 1H), 7.84-7.79 (m, 1H), 7.74- 7.58 (m, 3H), 7.22-7.16 (m, 1H), 4.89 (s, 1H), 4.57 (s, 1H), 4.08 (s, 1H), 3.87 (t, *J* = 5.8 Hz, 1H), 3.07 (d, *J* = 4.4 Hz, 3H), 2.65 (t, *J* = 5.8 Hz, 2H).

According to the synthetic scheme of Compound **A20** above, Compound **A21** could be obtained by replacing Compound **A-4** with the raw material as listed in Table 4.

**Table 4. Compound A21**

| Raw material | Product structure | H-NMR data characterization |
|---|---|---|
| 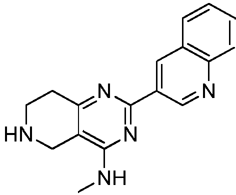 | 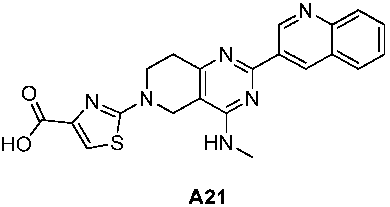 (LC-MS: C₂₁H₁₉N₆O₂S, [M+H]⁺ 419.1; detected value: 419.2; purity: 94.9%) | ¹H NMR (400 MHz, Methanol-*d*₄) δ 9.76 (s, 1H), 9.20 (s, 1H), 8.06 (d, *J* = 8.4 Hz, 2H), 7.81 (t, *J* = 7.8 Hz, 1H), 7.65 (t, *J* = 7.6 Hz, 1H), 7.52 (s, 1H), 4.42 (s, 2H), 3.89 (t, *J* = 5.9 Hz, 2H), 3.19 (s, 3H), 3.02 (t, *J* = 5.9 Hz, 2H). |

### Synthesis method of Compound A22 of Series A of the present invention:

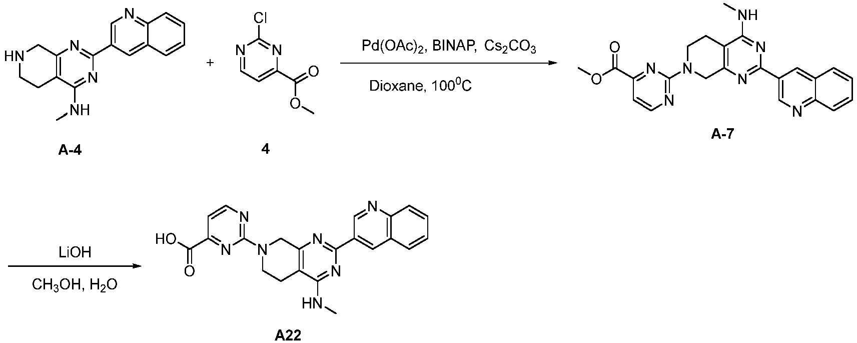

### Step 1, Synthesis of Compound A-7

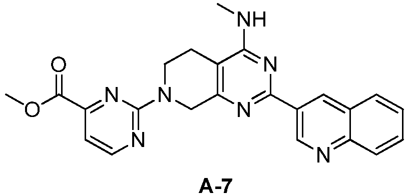

Under nitrogen protection, Compound A-4 (20.00 mg, 68.65 µmol), Compound 4 (11.85 mg, 68.65 µmol), BINAP (42.74 mg, 68.65 µmol), cesium carbonate (22.36 mg, 68.65 µmol), 1,4-dioxane and water (4 mL/1 mL) and Pd(OAc)₂ (15.41 mg, 0.25 mol%) were added to a 50 mL reaction bottle in sequence. The reaction system was sealed, stirred at 100 °C for 2 h, and then quenched (monitored by LC-MS). Saturated sodium chloride solution (10 mL) and ethyl acetate (3 × 10 mL) were used to complete the extraction. The organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure to remove solvent to obtain Compound **A-7** (25.00 mg, 58.49 µmol, 85.20% yield), LCMS: C₂₃H₂₂N₇O₂, (ESI) [M +H]: 428.2.

### Step 2. Synthesis of Compound A22

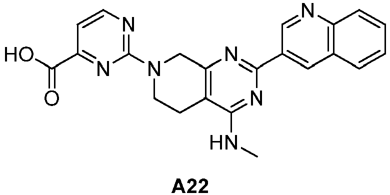

Compound **A-7** (25.00 mg, 58.49 µmol), lithium hydroxide (560.25 µg, 23.39 µmol), methanol (2 mL) and water (2 mL) were added to a 50 mL reaction bottle in sequence, stirred at room temperature for 5 hours, and then the reaction was quenched (monitored by LC-MS). The pH value of the system was adjusted to 6-7 with 1N hydrochloric acid solution, and the reaction solution was concentrated, and purified by MPLC to obtain Compound **A22** (5.00 mg, 12.09 µmol, 51.70% yield, 95.0% purity). LC-MS: C₂₂H₂₀N₇O₂, [M+H]⁺ 414.2; detected value: 414.3. ¹H NMR (400 MHz, DMSO-d₆) δ 9.81 (d, *J* = 2.4 Hz, 1H), 9.79 (s, 1H), 8.69 (d, *J* = 4.8 Hz, 1H), 8.34 - 8.28 (m, 2H), 8.03 (t, *J* = 8.0 Hz, 1H), 7.84 (t, *J* = 5.6 Hz, 1H), 7.18 (d, *J* = 4.8 Hz, 1H), 5.08 (s, 2H), 4.18 (t, *J* = 5.6 Hz, 2H), 3.12 (s, 3H), 2.72 (s, 2H).

According to the synthetic scheme of Compound **A22,** Compounds **A23** to **A25** could be obtained by replacing Compound **4** with the raw material as listed in Table 5.

**Table 5. Compounds A23 to A25**

| Raw material | Product structure | H-NMR data characterization |
|---|---|---|
| 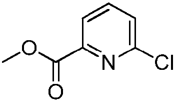 | 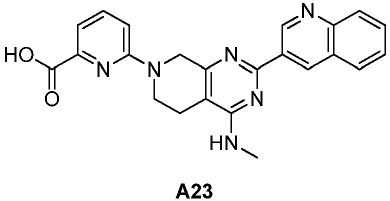 (LC-MS: C₂₃H₂₁N₆O₂, [M+H]⁺ 413.2; detected value: 413.3; purity: 98.9%) | ¹H NMR (400 MHz, Methanol-*d*₄) δ 9.68 (d, *J* = 2.0 Hz, 1H), 9.35 (s, 1H), 8.22-8.16 (m, 2H), 8.00 (t, *J* = 8.4 Hz, 1H), 7.83-7.77 (m, 2H), 7.52 (d, *J* = 7.2 Hz, 1H), 7.24 (d, *J* = 8.6 Hz, 1H), 4.90 (s, 2H), 4.07 (t, *J* = 5.7 Hz, 2H), 3.26 (s, 3H), 2.67 (t, *J* = 5.8 Hz, 2H). |
| 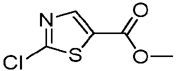 | 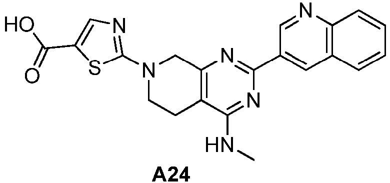 (LC-MS: C₂₁H₁₉N₆O₂S, [M+H]⁺ 419.1; detected value: 419.0; purity: 99.0%) | ¹H NMR (400 MHz, DMSO-d₆) δ 9.76 (s, 1H), 9.50 (s, 1H), 8.26 (dd, *J* = 24.2, 8.3 Hz, 2H), 7.98 (s, 1H), 7.91 (s, 1H), 7.85 (s, 1H), 7.79 (s,), 4.68 (s, 2H), 3.91 (s, 2H), 3.09 (s, 3H), 2.65 (s, 2H). |
| 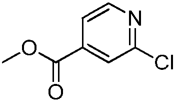 | 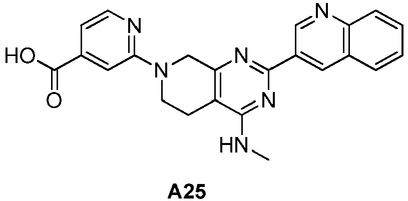 (LC-MS: C₂₃H₂₁N₆O₂, [M+H]⁺ 413.2; detected value: 413.2; purity: 98.2%) | ¹H NMR (400 MHz, DMSO-d₆) δ 9.74 (s, 1H), 9.26 (d, *J* = 2.0 Hz, 1H), 8.34 (d, *J* = 5.2 Hz, 1H), 8.22 (d, *J* = 7.8 Hz, 1H), 8.14 (d, *J* = 8.4 Hz, 1H), 7.91 (t, *J* = 7.6 Hz, 1H), 7.74 (t, *J* = 7.5 Hz, 1H), 7.48 (s, 1H), 7.13 (d, *J* = 5.2 Hz, 1H), 4.45 (s, 2H), 4.06 (t, *J* = 5.7 Hz, 2H), 3.18 (d, *J* = 4.2 Hz, 3H), 2.97 (t, *J =* 5.8 Hz, 2H). |

### Synthesis method of Compound A26 of Series A of the present invention:

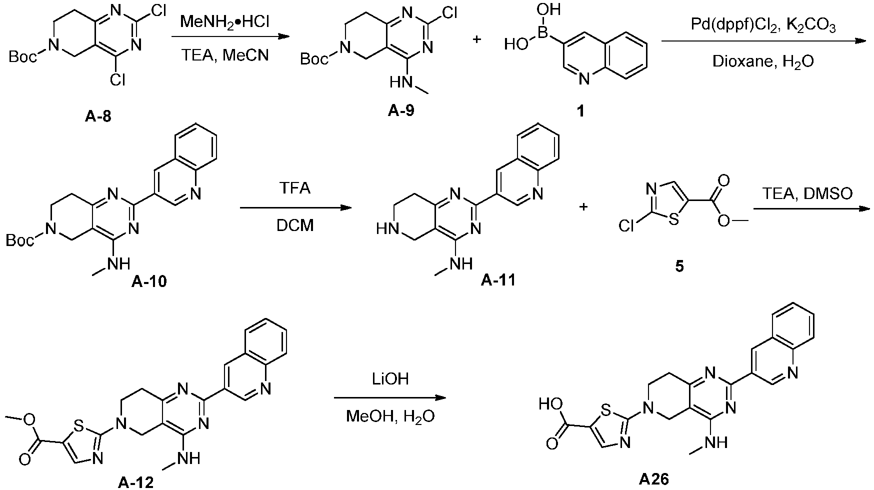

### Step 1, Synthesis of Compound A-9

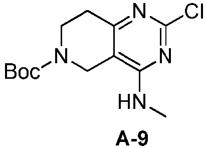

Compound A-8 (500.00 mg, 1.64 mmol), methylamine hydrochloride (61.26 mg, 1.97 mmol), triethylamine (499.01 mg, 4.93 mmol, 687.82 µL) and acetonitrile (10 mL) were added into a 50 mL reaction bottle, reacted at 80 °C for 2 hours, then water was added to quench the reaction (monitored by LC-MS). Saturated sodium chloride solution (10 mL) and ethyl acetate (3 × 15 mL) were used for extraction, and the organic phases were combined and dried over anhydrous sodium sulfate, evaporated to remove solvent, purified by MPLC, and concentrated under reduced pressure to remove solvent to obtain Compound A-9 (490.00 mg, 1.64 mmol, 99.77% yield), LCMS: C₁₃H₂₀ClN₄O₂, (ESI) [M+H]: 299.2.

### Step 2, Synthesis of Compound A-10

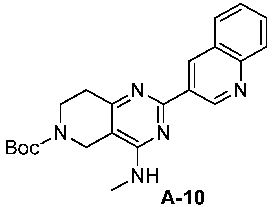

Under nitrogen protection, Compound **A-9** (490.00 mg, 1.64 mmol), Compound 1 (91.18 mg, 530.18 µmol), potassium carbonate (182.91 mg, 1.33 mmol), 1,4-dioxane and water (4 mL/1 mL) and Pd(dppf)Cl₂ (6.44 mg, 0.25 mol%) were added to a 50 mL reaction bottle in sequence. The reaction system was sealed and stirred at 80°C for 2 hours, then the reaction was quenched (monitored by LC-MS). Saturated sodium chloride solution (20 mL) and ethyl acetate (3×20 mL) were used to complete extraction, and the organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure to remove solvent to obtain Compound **A-10** (96.00 mg, 245.85 µmol, 55.65% yield). LCMS (ESI): C₂₂H₂₆N₅O₂, [M+H]: 392.2.

### Step 3, Synthesis of Compound A-11

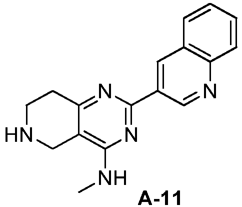

Compound A-10 (96.00 mg, 245.85 µmol) was dissolved in dichloromethane (2 mL) in a 50 mL reaction bottle, and trifluoroacetic acid (1 mL) was then added dropwise, stirred and reacted at room temperature for 3 hours, and the solvent was removed by concentration under reduced pressure to obtain Compound A-11 (crude product). LCMS: C₁₇H₁₈N₅, (ESI) [M+H]: 292.2.

### Step 4, Synthesis of Compound A-12

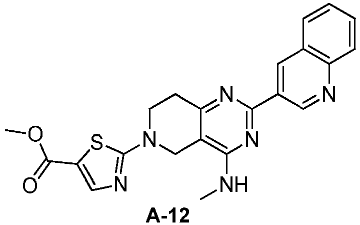

Compound A-11 (crude product), Compound 5 (24.38 mg, 137.29 µmol), triethylamine (41.68 mg, 411.88 µmol, 57.45 µL) and DMSO (5 mL) were added to a 50 mL reaction bottle, heated to 80 °C, stirred and reacted for 2 hours, and then water was added to quench the reaction (monitored by LC-MS). Saturated sodium chloride solution (25 mL) and ethyl acetate (3 × 25 mL) were used for extraction, and the organic phases were combined and dried over anhydrous sodium sulfate, evaporated to remove solvent, purified by MPLC, and concentrated under reduced pressure to remove solvent to obtain Compound A-12 (35.00 mg, 80.93 µmol, 58.94% yield) was obtained. LCMS: C₂₂H₂₁N₆O₂S, (ESI) [M+H]: 433.1.

### Step 5, Synthesis of Compound A26

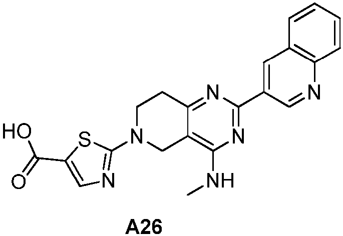

Compound **A-12** (35.00 mg, 80.93 µmol), lithium hydroxide (560.25 µg, 23.39 µmol), methanol (2 mL) and water (2 mL) were added to a 50 mL reaction bottle in sequence, stirred and reacted at room temperature for 5 hours, and then the reaction was quenched (monitored by LC-MS). The pH value of the system was adjusted to 6-7 with 1N hydrochloric acid solution. The reaction solution was concentrated and purified by MPLC to obtain Compound A26 (10.80 mg, 25.81 µmol, 31.89% yield, 98.0% purity). LC-MS: C₂₁H₁₉N₆O₂S, [M+H]⁺ 419.1; detected value: 419.1. ¹H NMR (400 MHz, DMSO-d₆) δ 9.77 (d, *J* = 2.1 Hz, 1H), 9.56 (s, 1H), 8.60 (s, 1H), 8.27 (d, *J =* 8.4 Hz, 1H), 8.22 (d, *J =* 8.4 Hz, 1H), 7.99 (t, *J* = 7.7 Hz, 1H), 7.86 (s, 1H), 7.80 (t, *J* = 7.6 Hz, 1H), 4.48 (s, 2H), 3.96 (t, *J* = 5.8 Hz, 2H), 3.17 -3.14 (m, 5H).

According to the synthetic scheme of Compound **A26,** Compound **A27** could be obtained by replacing Compound **5** with the raw material as listed in Table 6.

**Table 6. Compound A27**

| Raw material | Product structure | H-NMR data characterization |
|---|---|---|
| 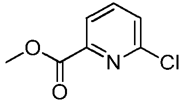 | 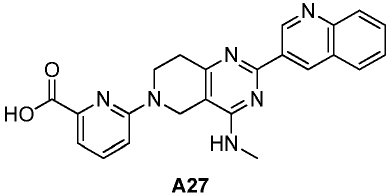 (LC-MS: C₂₃H₂₁N₆O₂, [M+H]⁺ 413.2; detected value: 413.3; purity: 96.4%) | ¹H NMR (400 MHz, DMSO-d₆) δ 9.75 (s, 1H), 9.26 (s, 1H), 8.21 (d, *J* = 8.0 Hz, 1H), 8.13 (d, *J* = 8.0 Hz, 1H), 7.90 (t, *J* = 7.8 Hz, 1H), 7.83 (t, *J* = 8.0 Hz, 1H), 7.74 (t, *J* = 7.5 Hz, 1H), 7.41 (d, *J* = 7.2 Hz, 1H), 7.26 (d, *J* = 8.5 Hz, 1H), 4.46 (s, 2H), 4.08 (t, *J* = 5.7 Hz, 2H), 3.19 (d, *J* = 4.2 Hz, 3H), 2.96 (t, *J* = 4.0 Hz, 2H). |

### Example 2, Synthesis of Compounds B1 and B2 of Series B of the Present Invention:

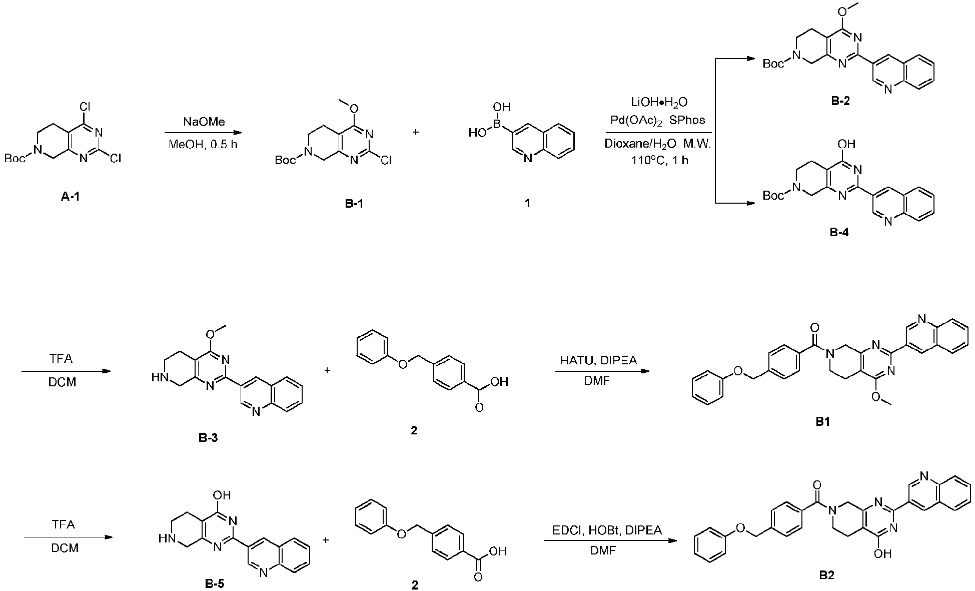

### Step 1, Synthesis of Compound B-1

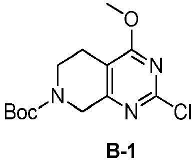

Compound **A-1** (400.00 mg, 1.32 mmol), sodium ethoxide (71.01 mg, 1.32 mmol) and methanol (5 mL) were added to a 50 mL reaction bottle in sequence, stirred and reacted at room temperature for 0.5 hours, and then water was added to quench the reaction (monitored by LC-MS). Saturated sodium chloride solution (25 mL) and ethyl acetate (3 × 25 mL) were used for extraction, and the organic phases were combined and dried over anhydrous sodium sulfate, and evaporated to remove solvent to obtain Compound **B-1** (crude product), LCMS: C₁₃H₁₉ClN₃O₃, (ESI) [M+H]: 300.1.

### Step 2, Synthesis of Compound B-2

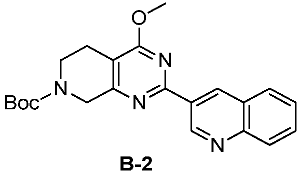

Under nitrogen protection, Compound **B-1** (crude product), Compound **1** (219.28 mg, 1.27 mmol), hydrated lithium hydroxide (191.59 mg, 8.00 mmol), Pd(OAc)₂ (2.85 mg, 0.25 mol%), S-Phos (10.40 mg, 25.35 µmol), 1,4-dioxane and water (8 mL/2 mL) were added to a microwave reaction tube in sequence. The reaction system was sealed, and the reaction was carried under stirring at 110 °C for 1 hour and then quenched (monitored by LC-MS). Saturated sodium chloride solution (20 mL) and ethyl acetate (3×20 mL) were used to complete the extraction, and the organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure to remove solvent to obtain Compound **B-2** (140.00 mg, 369.96 µmol, 29.18% yield). LCMS: C₂₂H₂₅N₄O₃, (ESI) [M+H]: 393.2.

### Step 3, Synthesis of Compound B-3

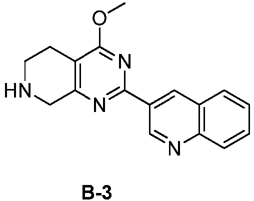

Compound B-2 (140.00 mg, 369.96 µmol) was dissolved in dichloromethane (4 mL) in a 50 mL reaction bottle, and then TFA (1 mL) was added dropwise. The mixture was stirred at room temperature for 0.5 hours, and the solvent was removed by concentration under reduced pressure to obtain Compound **B-3** (crude product). LCMS: C₁₇H₁₇N₄O, (ESI) [M+H]: 293.1.

### Step 4, Synthesis of Compound B1

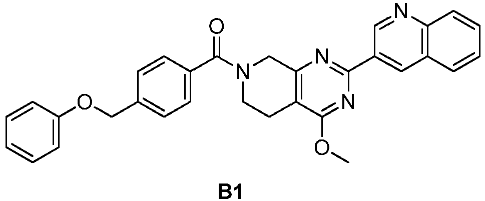

Compound 2 (91.30 mg, 400.00 µmol), DIPEA (129.24 mg, 1.00 mmol, 174.18 µL), HATU (182.40 mg, 480.00 µmol) and DMF (4 mL) were added to a 50 mL reaction bottle in sequence. After the temperature of the reaction system dropped to 0 °C, Compound **B-3** (crude product) was added. After stirring for 1.5 hours under ice bath conditions, the reaction was quenched (monitored by LC-MS). Saturated sodium chloride solution (20 mL) and ethyl acetate (3×20 mL) were used to complete extraction. The organic phases were combined, dried over anhydrous sodium sulfate, evaporated to remove solvent, purified by MPLC, and concentrated under reduced pressure to remove solvent to obtain Compound **B1** (28.40 mg, 52.55 µmol, 13.14% yield, 93.0% purity). LC-MS: C₃₁H₂₇N₄O₃, [M+H]⁺ 503.2; detected value: 503.2. ¹H NMR (400 MHz, Methanol-d4) δ 9.88-9.78 (m, 1H), 9.63-9.53 (m, 1H), 8.27-8.16 (m, 2H), 8.02 (s, 1H), 7.84 (s, 1H), 7.61-7.53 (m, 4H), 7.37-7.24 (m, 2H), 7.01-6.96 (m, 2H), 6.95-6.92 (m, 1H), 5.16 (s, 2H), 4.86 (s, 1H), 4.72 (s, 1H), 4.19 (s, 3H), 4.07-4.01 (m, 1H), 3.75 (s, 1H), 2.80 (s, 2H). Purity> 90%.

### Step 5, Synthesis of Compound B-4

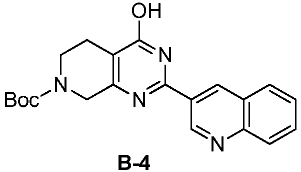

Under nitrogen protection, Compound **B-1** (crude), Compound 1 (219.28 mg, 1.27 mmol), hydrated lithium hydroxide (191.59 mg, 8.00 mmol), Pd(OAc)₂ (2.85 mg, 0.25 mol%), S-Phos (10.40 mg, 25.35 µmol), 1,4-dioxane and water (8 mL/2 mL) were added to a microwave reaction tube in sequence. The reaction system was sealed and stirred at 110 °C for 1 hour before quenching the reaction (monitored by LC-MS). Saturated sodium chloride solution (20.0 mL) and ethyl acetate (3×20 mL) were used to complete the extraction. The organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure to obtain Compound **B-4** (140.29 mg, 369.96 µmol, 29.18% yield) was obtained. LCMS: C₂₁H₂₃N₄O₃, (ESI) [M+H]: 379.2.

### Step 6, Synthesis of Compound B-5

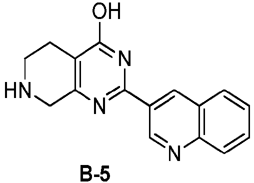

Compound **B-4** (140.29 mg, 369.96 µmol) was dissolved in dichloromethane (4 mL) in a 50 mL reaction bottle, and then TFA (1 mL) was added dropwise. The reaction was carried out under stirring at room temperature for 0.5 hours, and the solvent was removed by concentration under reduced pressure to obtain Compound **B-5** (crude product). LCMS: C₁₆H₁₅N₄O, (ESI) [M+H]: 279.1.

### Step 7, Synthesis of Compound B2

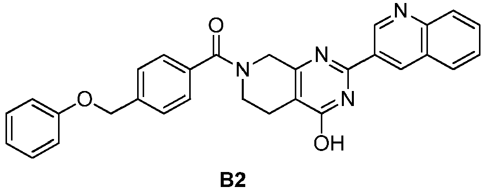

Compound **2** (91.30 mg, 0.4 mmol), EDCI (91.68 mg, 480.00 µmol), HOBt (64.86 mg, 480.00 µmol), DIPEA (62.04 mg, 480.00 µmol) and DMF (4 mL) were added to a 50 mL reaction bottle in sequence. After the temperature of the reaction system dropped to 0 °C, Compound **B-5** (crude product) was added. The reaction was carried out under stirring and ice bath conditions for 1 hour, and then quenched (monitored by LC-MS). The mixture was extracted with saturated sodium chloride solution (10 mL) and ethyl acetate (3×10 mL). The organic phases were combined and dried over anhydrous sodium sulfate, evaporated to remove solvent, and purified by MPLC, and the solvent was removed by concentration under reduced pressure to obtain Compound **B2** (2.60 mg, 5.24 µmol, 1.31% yield, 98.5% purity). LC-MS: C₃₀H₂₅N₄O₃, [M+H]⁺ 489.2; detected value: 489.4. ¹H NMR (400 MHz, DMSO-d₆) δ 13.06 (s, 1H), 9.53-9.42 (m, 1H), 9.11-9.05 (m, 1H), 8.10 (s, 2H), 7. 89 (s, 1H), 7.72 (s, 1H), 7.55 (q, *J* = 8.0 Hz, 4H), 7.31 (t, *J =* 8.0 Hz, 2H), 7.04 (d, *J =* 8.0 Hz, 2H), 7.03-6.96 (m, 1H), 5.18 (s, 2H), 4.67-4.47 (m, 2H), 3.75-3.15 (m, 2H), 2.50 (s, 2H). Purity> 95%.

### Example 3, Synthesis of Compound C1 of Series C of the present invention:

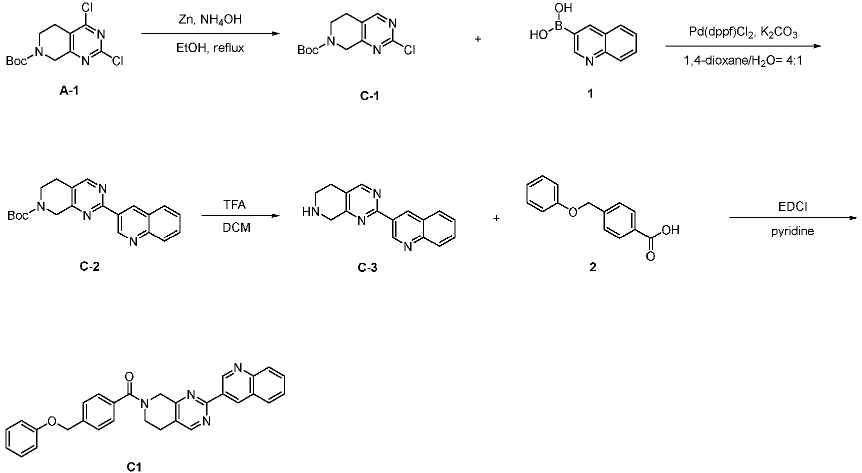

### Step 1, Synthesis of Compound C-1

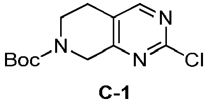

Compound **A-1** (250.00 mg, 821.90 µmol), ammonium hydroxide (4.11 mmol), zinc powder (6.58 mmol), and ethanol (2 mL) were added to a 50 mL reaction bottle in sequence, heated to 80 °C and refluxed, and reacted for 4 hours (monitored by LC-MS). Zinc powder was removed by filtration and washed with methanol (2×20 mL), the filtrate was collected, concentrated under reduced pressure to remove solvent, and purified by MPLC to obtain Compound **C-1** (175.00 mg, 648.80 µmol, 78.94% yield), LCMS: C₁₂H₁₇ClN₃O₂, (ESI) [M+H]: 270.1.

### Step 2, Synthesis of Compound C-2

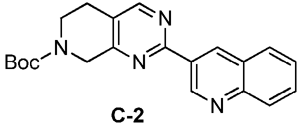

Under nitrogen protection, Compound **C-1** (175.00 mg, 648.80 µmol), Compound 1 (134.67 mg, 778.57 µmol), potassium carbonate (224.18 mg, 1.62 mmol), 1,4-dioxane and water (4 mL/1 mL) and Pd(dppf)Cl₂ (2.46 mg, 0.25 mol%) were added to a 50 mL reaction bottle in sequence. The reaction system was sealed, stirred and reacted at 80 °C for 2 hours, then the reaction was quenched (monitored by LC-MS). Saturated sodium chloride solution (20 mL) and ethyl acetate (3×20 mL) were used to complete the extraction, and the organic phases were combined and dried over anhydrous sodium sulfate, evaporated to remove solvent, and separated by column chromatography with an eluent having a volume ratio of petroleum ether/ethyl acetate = 1:100~1:5, and the solvent was removed by concentration under reduced pressure to obtain Compound **C-2** (235.00 mg, 648.41 µmol, 99.94% yield). LCMS: C₂₁H₂₃N₄O₂, (ESI) [M+H]: 363.2.

### Step 3, Synthesis of Compound C-3

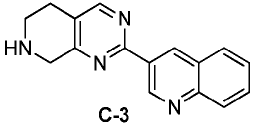

Compound **C-2** (235.00 mg, 648.41 µmol) was dissolved in dichloromethane (4 mL) in a 50 mL reaction bottle, and then trifluoroacetic acid (1 mL) was added dropwise. The reaction was carried out under stirring at room temperature for 0.5 hours, and the solvent was removed by concentration under reduced pressure to obtain Compound **C-3** (crude product). LCMS: C₁₆H₁₅N₄, (ESI) [M+H]: 263.1.

### Step 4, Synthesis of compound C1

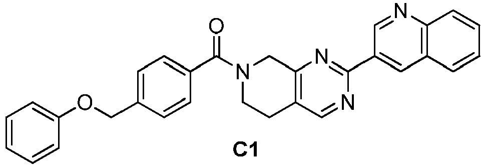

Compound **2** (170.00 mg, 648.09 µmol), EDCI (246.27 mg, 1.30 mmol) and piperidine solution (1 mL) were added to a 50 mL reaction bottle in sequence. After the temperature of the reaction system dropped to 0 °C, Compound **C-3** (crude product) was added. The reaction was carried out under stirring and ice bath conditions for 1 hour and then quenched. Extraction was completed with saturated sodium chloride solution (20 mL) and dichloromethane (3×15 mL). The organic phases were combined and dried over anhydrous sodium sulfate, evaporated to remove solvent, and purified by MPLC, and the solvent was removed by concentration under reduced pressure to obtain Compound **C1** (10.00 mg, 20.32 µmol, 3.13% yield, 96.0% purity). LC-MS: C₃₀H₂₅N₄O₂, [M+H]⁺ 473.2; detected value: 473.1. ¹H NMR (400 MHz, Chloroform-d) δ 8.69 (s, 1H), 8.25 (s, 1H), 7.99 (s, 1H), 7.81 (t, *J* = 8.0 Hz, 1H), 7.63 (t, *J* = 8.0 Hz, 1H), 7.58-7.52 (m, 5H), 7.34-7.30 (m, 2H), 7.01-6.98 (m, 3H), 5.14 (s, 2H), 5.09-4.78 (m, 2H), 4.10-3.73 (m, 2H), 3.03-3.01 (m, 2H).

According to the synthetic scheme of Compound **C1** above, Compounds **C2** to **C10** could be obtained by replacing Compound **2** with the raw materials as listed in Table 7.

**Table 7. Compounds C2 to C10**

| Raw material | Product structure | H-NMR data characterization |
|---|---|---|
| 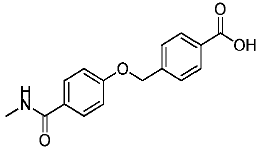 | 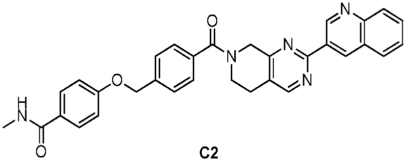 (LC-MS: C₃₂H₂₈N₅O₃, [M+H]⁺ 530.2; detected value: 530.3; purity: 94.7%) | ¹H NMR (400 MHz, Methanol-*d*₄) δ 9.93 (s, 1H), 9.60 (s, 1H), 8.80 (s, 1H), 8.36-8.24 (m, 1H), 8.16 (d, *J* = 4.0 Hz, 1H), 8.02 (t, *J* = 8.0 Hz, 1H), 7.79 (d, *J* = 8.7 Hz, 3H), 7.64-7.56 (m, 4H), 7.08 (d, *J* = 8.0 Hz, 1H), 7.06 (d, *J* = 8.0 Hz, 1H), 5.25 (s, 2H), 5.06 (s, 2H), 3.80 (s, 2H), 3.04 (d, *J* = 4.0 Hz, 2H), 2.90 (s, 3H). |
| 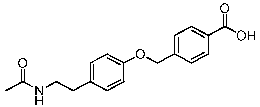 | 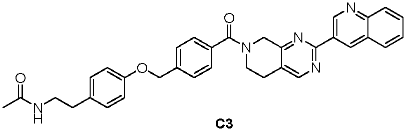 (LC-MS: C₃₄H₃₂N₅O₃, [M+H]⁺ 558.2; detected value: 558.3; purity: 99.1%) | ¹H NMR (400 MHz, DMSO-*d*₆) δ 9.82-9.77 (m, 1H), 9.29 (s, 1H), 8.42 (s, 1H), 8.21-8.08 (m, 2H), 7.91 (t, *J* = 4.0 Hz, 1H), 7.85 (t, *J* = 8.0 Hz, 1H), 7.68 (s, 1H), 7.59-7.54 (m, 4H), 7.13 (d, *J* = 8.0 Hz, 2H), 6.96 (d, *J* = 8.0 Hz, 2H), 5.16 (s, 2H), 4.94-4.77 (m, 2H), 3.67-3.53 (m, 2H), 3.24-3.12 (m, 2H), 3.11 (s, 2H), 2.62 (t, *J* = 8.0 Hz, 2H), 1.78 (s, 3H). |
| 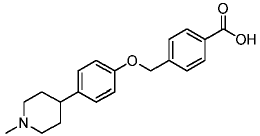 | 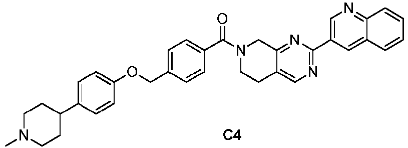 (LC-MS: C₃₆H₃₆N₅O₂, [M+H]⁺ 570.3; detected value: 570.3; purity: 99.9%) | ¹H NMR (400 MHz, Methanol-*d*₄) δ9.77-9.68 (m, 1H), 9.20-9.11 (m, 1H), 8.77 (s, 1H), 8.11-8.06 (m, 2H), 7.87 (s, 1H), 7.70 (s, 1H), 7.61-7.54 (m, 4H), 7.22-7.18 (m, 2H), 7.03-6.96 (m, 2H), 5.18 (s, 2H), 3.90 (d, *J* = 4.4 Hz, 1H), 3.80 (d, *J* = 5.6 Hz, 1H), 3.72 (t, *J* = 8.0 Hz, 1H), 3.60-3.48 (m, 3H), 3.16-3.13 (m, 1H), 3.10-3.03 (m, 2H), 3.03-2.94 (m, 2H), 2.91 (s, 3H), 2.11-2.03 (m, 2H), 1.94-1.88 (m, 2H). |
| 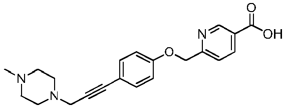 | 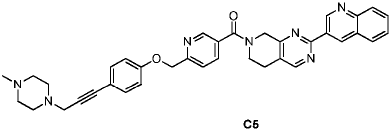 (LC-MS: C₃₇H₃₆N₇O₂, [M+H]⁺ 610.3; detected value: 610.2; purity: 99.7%) | ¹H NMR (400 MHz, Methanol-*d*₄) δ 9.80-9.71 (m, 1H), 9.23-9.21 (m, 1H), 8.73 (d, *J* = 9.6 Hz, 2H), 8.04 (d, *J* = 8.0 Hz, 3H), 7.84-7.67 (m, 3H), 7.37 (t, *J* = 8.8 Hz, 2H), 7.01 (d, *J* = 8.0 Hz, 2H), 5.27 (s, 2H), 4.02-3.32 (m, 8H), 3.14-2.77 (m, 11H). |
| 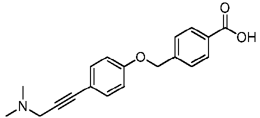 | 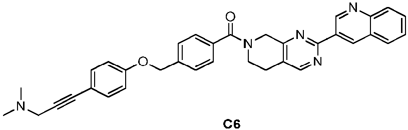 (LC-MS: C₃₅H₃₂N₅O₂, [M+H]⁺ 554.3; detected value: 554.3; purity: 99.1%) | ¹H NMR (400 MHz, Chloroform-*d*) δ 9.91 (s, 1H), 9.10 (s, 1H), 8.61 (s, 1H), 8.09 (d, *J* = 4.0 Hz, 1H), 7.88 (s, 1H), 7.71 (t, *J* = 7.6 Hz, 1H), 7.54-7.45 (m, 5H), 7.34 (d, *J* = 8.0 Hz, 2H), 6.87 (d, *J =* 8.6 Hz, 2H), 5.08 (s, 2H), 4.09-4.00 (m, 2H), 3.78-3.64 (m, 4H), 2.93-2.87 (m, 2H), 2.54-2.43 (m, 6H). |
| 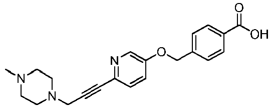 | 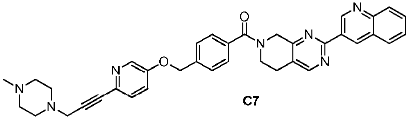 (LC-MS: C₃₇H₃₆N₇O₂, [M+H]⁺ 610.3; detected value: 610.4; purity: 96.7%) | ¹H NMR (400 MHz, Methanol-*d*₄) δ 9.98-9.79 (m, 2H), 8.78 (s, 1H), 8.36-8.34 (m, 2H), 8.26 (d, *J =* 8.7 Hz, 1H), 8.13 (t, *J* = 7.8 Hz, 1H), 7.93 (s, 1H), 7.64-7.58 (m, 6H), 5.30 (s, 2H), 5.03 (s, 1H), 4.84 (s, 1H), 4.12 (s, 1H) , 3.82 (s, 3H), 3.40 (s, 4H), 3.06 (s, 6H), 2.93 (s, 3H). |
| 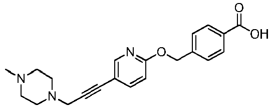 | 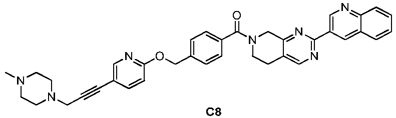 (LC-MS: C₃₇H₃₆N₇O₂, [M+H]⁺ 610.3; detected value: 610.4; purity: 99.8%) | ¹H NMR (400 MHz, Methanol-*d*₄) δ 9.97-9.73 (m, 2H), 8.77 (s, 1H), 8.36-8.23 (m, 2H), 8.11 (s, 1H), 8.04 (d, *J* = 2.4 Hz, 1H), 7.92 (s, 1H), 7.55-7.46 (m, 5H), 6.56 (d, *J* = 9.4 Hz, 1H), 5.25 (s, 2H), 5.01 (s, 1H), 4.87 (s, 1H), 4.10 (s, 1H), 3.74 (s, 3H), 3.37 (s, 4H), 3.01 (s, 6H), 2.90 (s, 3H). |
| 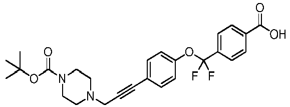 | 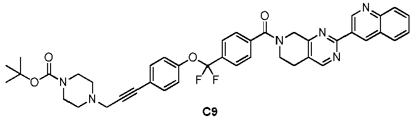 (LC-MS: C₄₂H₄₁F₂N₆O₄, [M+H]⁺731.3; detected value: 731.3; purity: 90.6%) | ¹H NMR (400 MHz, Methanol-*d*₄) δ 8.50 (s, 1H), 8.15 (s, 1H), 7.90 (d, *J* = 8.1 Hz, 1H), 7.81-7.66 (m, 7H),7.59-7.52 (m, 3H), 7.39-7.20 (m, 2H), 4.29 (t, *J* = 6.6 Hz, 4H), 3.96-3.95 (m, 3H), 3.77-3.60 (m, 3H), 3.03-2.99 (m, 3H), 2.46-2.38 (m, 3H), 1.48-1.45 (m, 9H). |
| 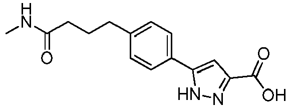 | 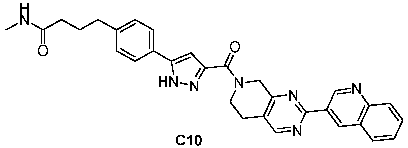 (LC-MS: C₄₁H₃₀N₇O₂, [M/2+H]⁺ 266.6; detected value: 266.7; purity: 96.9%) | ¹H NMR (400 MHz, DMSO-*d*₆) δ 9.82 (d, *J* = 13.4 Hz, 1H), 9.24 (d, *J* = 12.4 Hz, 1H), 8.80 (s, 1H), 8.11 (s, 2H), 7.85 (d, *J* = 8.5 Hz, 1H), 7.76-7.68 (m, 3H), 7.29 (d, *J* = 7.8 Hz, 2H), 6.97 (s, 1H), 5.48-5.41 (m, 1H), 5.01-4.97 (m, 1H), 4.36-4.03 (m, 2H), 3.11-3.04 (m, 2H), 2.64 (d, *J* = 4.9 Hz, 3H), 2.16 (dd, *J =* 16.1, 8.7 Hz, 2H), 1.92-1.85 (m, 2H), 0.86 (d, *J* = 9.3 Hz, 2H). |

### Synthesis method of Compound C11 of Series C of the present invention:

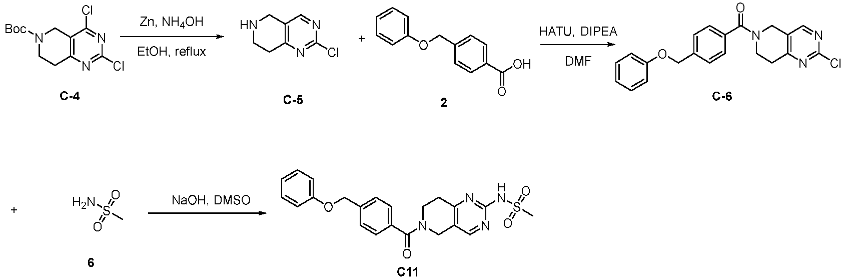

### Step 1, Synthesis of Compound C-5

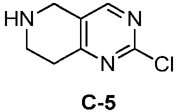

Compound **C-4** (400 mg, 1.32 mmol), ammonium hydroxide (4.11 mmol), zinc powder (3.95 mmol), and ethanol (5 mL) were added to a 50 mL reaction bottle in sequence, heated to 80 °C and refluxed, and reacted for 3 hours (monitored by LC-MS). Zinc powder was removed by filtration, washed with methanol (2×20 mL), the filtrate was collected, and concentrated under reduced pressure, then the concentrate was dissolved in a mixed solution of 6 N hydrochloric acid and ethyl acetate (10 mL), stirred and reacted at room temperature for 1 hour, and concentrated under reduced pressure to remove solvent to obtain Compound C-5 (crude product), LCMS: C₇H₉ClN₃, (ESI) [M+H]: 170.0.

### Step 2, Synthesis of Compound C-6

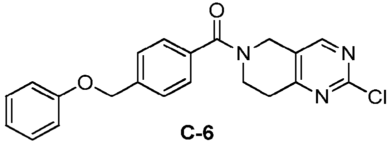

Compound **2** (150.00 mg, 657.19 µmol), DIPEA (254.81 mg, 1.97 mmol, 343.41 µL), HATU (274.71 mg, 722.91 µmol) and DMF (5 mL) were added to a 50 mL reaction bottle in sequence. After the temperature of the reaction system dropped to 0 °C, Compound **C-5** (crude product) was added. After stirring for 1 hour under ice bath conditions, the reaction was quenched (monitored by LC-MS). Saturated sodium chloride solution (20 mL) and ethyl acetate (3×20 mL) were used for extraction. The organic phases were combined, dried over anhydrous sodium sulfate, evaporated to remove solvent, purified by MPLC, and concentrated under reduced pressure to remove solvent to obtain Compound **C-6** (210.00 mg, 552.87 µmol, 84.13% yield), LCMS: C₂₁H₁₉ClN₃O₂, (ESI) [M+H]: 380.1.

### Step 3, Synthesis of Compound C11

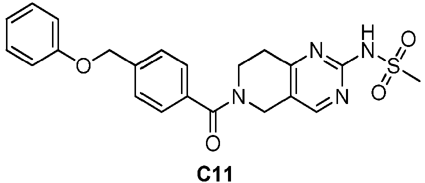

Compound **C-6** (50.00 mg, 131.63 µmol), Compound **6** (12.52 mg, 131.63 µmol), sodium hydroxide (15.80 mg, 394.90 µmol) and DMSO (5 mL) were added to a 50 mL reaction bottle, heated to 120 °C and stirred and reacted for 2 hours, and then water was added to quench the reaction (monitored by LC-MS). Saturated sodium chloride solution (25 mL) and ethyl acetate (3 X 25 mL) were used for extraction, the organic phases were combined, dried over anhydrous sodium sulfate, evaporated to remove solvent, purified by MPLC, and concentrated under reduced pressure to remove solvent to obtain Compound **C11** (30.00 mg, 61.71 µmol, 46.88% yield, 90.2% purity). LC-MS: C₂₂H₂₃N₄O₄S, [M+H]⁺ 439.1; detected value: 439.1. ¹H NMR (400 MHz, DMSO-*d₆*) δ 8.19-7.93 (m, 1H), 7.56 (d, *J =* 7.8 Hz, 2H), 7.48 (d, *J =* 7.8 Hz, 2H), 7.32 (t, *J =* 7.8 Hz, 2H), 7.04 (d, *J =* 8.2 Hz, 2H), 6.97 (t, *J =* 7.4 Hz, 1H), 5.16 (s, 2H), 4.62-4.44 (m, 2H), 3.60 (s, 2H), 2.94 (s, 3H), 2.72 (s, 2H).

According to the synthetic scheme of Compound **11**, Compounds **C12** and **C13** could be obtained by replacing Compound **6** with the raw material as listed in Table 8.

**Table 8. Compounds C12 and C13**

| Raw material | Product structure | H-NMR data characterization |
|---|---|---|
| 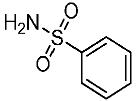 | 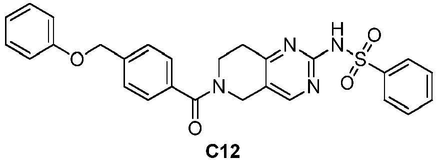 (LC-MS: C₂₇H₂₅N₄O₄S , [M+H]⁺ 501.2; detected value: 501.1; purity: 95.5%) | ¹H NMR (400 MHz, DMSO-*d₆*) δ 7.96 (d, *J* = 8.0 Hz, 2H), 7.56-7.50 (m, 4H), 7.43 (d, *J* = 5.6 Hz, 2H), 7.28 (t, *J* = 3.6 Hz, 3H), 7.00-6.94 (m, 4H), 5.11 (s, 2H), 4.63-4.48 (m, 2H), 3.56 (s, 2H), 2.76 (s, 2H). |
| 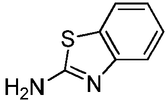 | 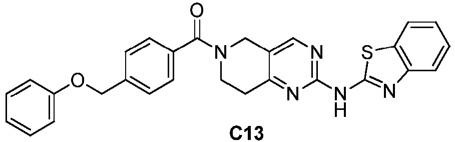 (LC-MS: C₂₈H₂₄N₅O₂S , [M+H]⁺ 494.2; detected value: 494.2; purity: 98.0%) | ¹H NMR (400 MHz, DMSO-*d₆*) δ 8.78 - 8.58 (m, 1H), 7.88 (d, *J* = 5.6 Hz, 1H), 7.62 (d, *J* = 8.0 Hz, 1H), 7.51 (t, *J* = 7.7 Hz, 3H), 7.37-7.19 (m, 4H), 7.17-6.91 (m, 4H), 5.13 (s, 2H), 4.73-4.60 (m, 2H), 3.84-3.81 (m, 2H), 2.95-2.89 (m, 2H). |

### Synthesis method of Compound C14 of series C of the present invention:

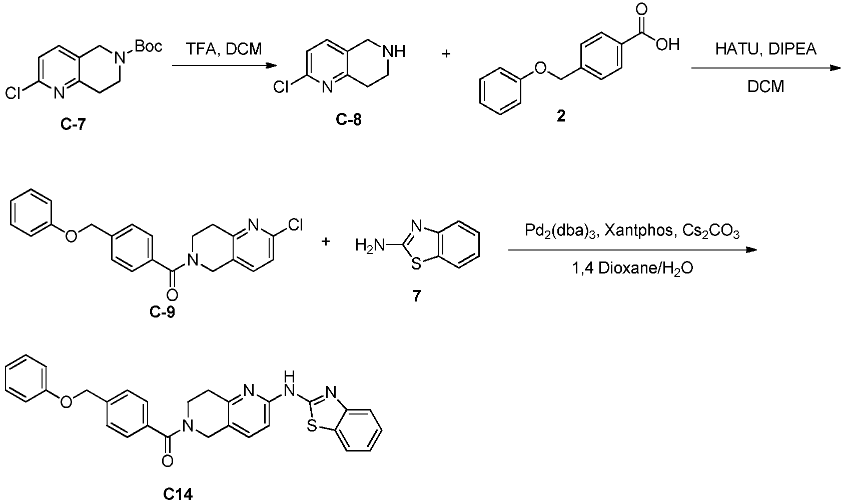

### Step 1, Synthesis of Compound C-8

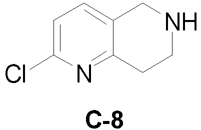

Compound **C-7** (300.00 mg, 1.12 mmol) was dissolved in dichloromethane (8 mL) in a 50 mL reaction bottle, then trifluoroacetic acid (1.5 mL) was added dropwise, the reaction was carried under stirring at room temperature for 0.5 hours, and the solvent was removed by concentration under reduced pressure to obtain Compound **C-8** (crude product), LCMS: C₈H₁₀ClN₂, (ESI) [M+H]: 169.0.

### Step 2. Synthesis of Compound C-9

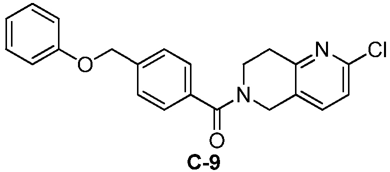

Compound **2** (228.00 mg, 998.94 µmol), DIPEA (322.76 mg, 2.50 mmol, 434.99 µL), HATU (455.51 mg, 1.20 mmol) and dichloromethane (10 mL) were added to a 50 mL reaction bottle in sequence. After the temperature of the reaction system dropped to 0 °C, Compound **C-8** (crude product) was added, and the reaction was carried out under stirring and ice bath conditions for 12 hours, and then quenched (monitored by LC-MS). Saturated sodium chloride solution (20 mL) and ethyl acetate (3×20 mL) were used to complete the extraction, and the organic phases were combined and dried over anhydrous sodium sulfate, evaporated to remove solvent, purified by MPLC, and concentrated under reduced pressure to remove solvent to obtain Compound **C-9** (300.00 mg, 791.87 µmol, 79.27% yield), LCMS: C₂₂H₂₀ClN₂O₂, (ESI) [M+H]: 379.1.

### Step 4, Synthesis of Compound C14

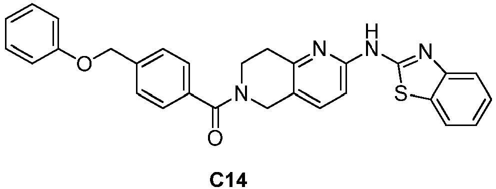

Under nitrogen protection, Compound **C-9** (300.00 mg, 791.87 µmol), Compound 7 (59.47 mg, 395.93 µmol), cesium carbonate (258.15 mg, 791.87 µmol), Xantphos (76.36 mg, 131.98 µmol), Pd₂(dba)₃ (48.34 mg, 52.79 µmol) and 1,4-dioxane and water (4 mL/1 mL) were added to a microwave reaction tube in sequence. The reaction system was sealed and reacted under microwaves at 150 °C for 1 hour, then the reaction was quenched (monitored by LC-MS). Saturated sodium chloride solution (20 mL) and ethyl acetate (3 × 20 mL) were used to complete the extraction. The organic phases were combined and dried over anhydrous sodium sulfate, evaporated to remove solvent, and separated by column chromatography with an eluent having a volume ratio of petroleum ether/ethyl acetate = 1:100~1:5, and the solvent was removed by concentration under reduced pressure to obtain Compound **C14** (46.50 mg, 94.40 µmol, 35.76% yield, 96.6% purity). LC-MS: C₂₀H₂₅N₄O₅S, [M+H]⁺ 493.2; detected value: 439.1. ¹H NMR (400 MHz, DMSO-*d₆*) δ 7.89 (d, *J =* 7.7 Hz, 1H), 7.62-7.55 (m, 6H), 7.37-7.30 (m, 3H), 7.19 (t, *J =* 7.5 Hz, 1H), 7.04 (d, *J =* 8.1 Hz, 3H), 6.96 (t, *J =* 7.3 Hz, 1H), 5.18 (s, 2H), 4.74-4.57 (m, 2H), 3.70 (s, 3H), 3.01 (s, 2H).

### Example 4, Synthesis method of Series D of the present invention:

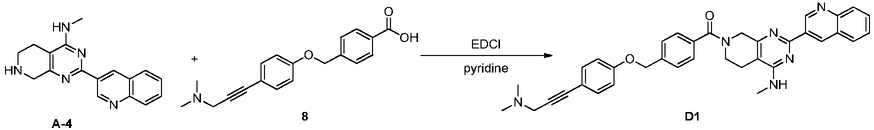

### Step 1, Synthesis of Compound D1

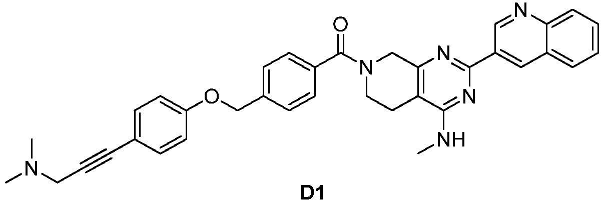

Compound 8 (40.00 mg, 129.30 µmol), EDCI (74.48 mg, 387.90 µmol) and piperidine solution (5 mL) were added to a 50 mL reaction bottle in sequence, and after the temperature of the reaction system dropped to 0 °C, Compound **A-4** (39.56 mg, 135.77 µmol) was added. After the reaction was carried out under stirring and ice bath conditions for 0.5 hours, the reaction was quenched. Saturated sodium chloride solution (20 mL) and dichloromethane (3×15 mL) were used to complete extraction. The organic phases were combined and dried over anhydrous sodium sulfate, evaporated to remove solvent, purified by MPLC, and concentrated under reduced pressure to remove the solvent to obtain Compound **D1** (25.20 mg, 42.43 µmol, 32.81% yield, 98.1% purity). LC-MS: C₃₆H₃₅N₆O₂, [M+H]⁺ 583.3; detected value: 583.3. ¹H NMR (400 MHz, Methanol-d4) δ 9.78 (s, 1H), 9.52 (s, 1H), 8.28-8.20 (m, 2H), 8.04 (t, *J =* 4.0 Hz, 1H), 7.86 (s, 1H), 7.62-7.55 (m, 4H), 7.48 (d, *J =* 8.4 Hz, 2H), 7.05 (d, *J =* 8.4 Hz, 2H), 5.21 (s, 2H), 4.86 (s, 1H), 4.70 (s, 1H), 4.28 (s, 2H), 4.12-4.09 (m, 1H), 3.85 (s, 1H), 3.25 (s, 3H), 3.00 (s, 6H), 2.66 (s, 2H).

According to the synthetic scheme of Compound **D1** above, Compounds **D2** to **D6** could be obtained by replacing Compound **8** with the raw materials as listed in Table 9.

**Table 9. Compounds D2 to D6**

| Raw material | Product structure | H-NMR data characterization |
|---|---|---|
| 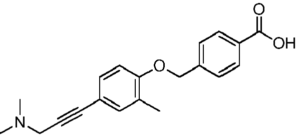 | 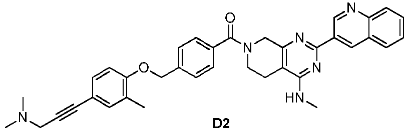 (LC-MS: C₃₇H₃₇N₆O₂, [M+H]⁺ 597.3; detected value: 597.3; purity: 98.6%) | ¹H NMR (400 MHz, DMSO-*d₆*) δ 9.60 (s, 1H), 9.33 (s, 1H), 8.20-8.12 (m, 2H), 8.00-7.79 (m, 2H), 7.54-7.32 (m, 6H), 7.03 (s, 1H), 5.18 (s, 2H), 4.76-4.61 (m, 2H), 4.21-3.66 (m, 4H), 3.08 (s, 3H), 2.85 (s, 6H), 2.17 (s, 5H). |
| 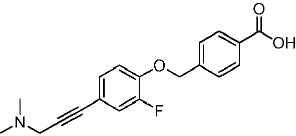 | 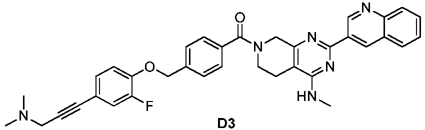 (LC-MS: C₃₆H₃₄FN₆O₂, [M+H]⁺ 601.3; detected value: 604.3; purity: 98.1%) | ¹H NMR (400 MHz, DMSO-*d₆*) δ 9.74-9.67 (m, 1H), 9.45-9.38 (m, 1H), 8.31-8.21 (m, 2H), 8.06-7.87 (m, 2H), 7.63-7.58 (m, 4H), 7.49 (d, *J =* 11.7 Hz, 1H), 7.38-7.34 (m, 2H), 5.30 (s, 2H), 4.86-4.66 (m, 2H), 4.27 (s, 2H), 4.02 (s, 1H), 3.72 (s, 1H), 3.16 (s, 3H), 2.90 (s, 6H), 2.56 (s, 2H). |
| 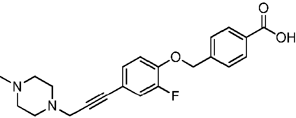 | 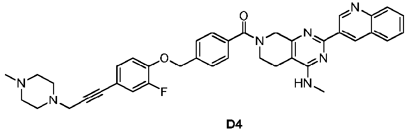 (LC-MS: C₃₉H₃₉FN₇O₂, [M+H]⁺ 656.3; detected value: 656.3; purity: 95.9%) | ¹H NMR (400 MHz, DMSO-*d₆*) δ 9.82-9.74 (m, 1H), 9.34-9.25 (m, 1H), 8.24 (s, 1H), 8.13 (s, 1H), 7.90 (s, 1H), 7.74 (s, 1H), 7.57-7.56 (m, 3H), 7.39-7.36 (m, 1H), 7.28 (s, 2H), 7.16-7.03 (m, 1H), 5.30 (s, 2H), 4.77-4.56 (m, 2H), 3.98 (s, 1H), 3.70 (s, 3H), 3.26 (S, 2H ), 3.11 (d, *J* = 3.8 Hz, 7H), 2.81 (s, 3H), 2.68-2.57 (m, 4H), 2.33 (s, 1H). |
| 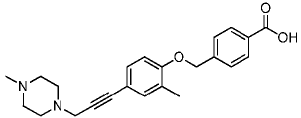 | 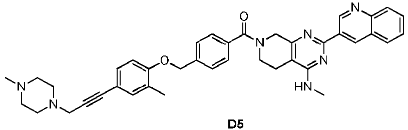 (LC-MS: C₄₀H₄₂N₇O₂, [M+H]⁺ 652.3; detected value: 652.2; purity: 95.1%) | ¹H NMR (400 MHz, DMSO-*d₆*) δ 9.74-9.64 (m, 1H), 9.16-9.05 (m, 1H), 8.11-8.05 (m, 2H), 7.81 (s, 1H), 7.65-7.48 (m, 5H), 7.23 (s, 2H), 6.98 (d, *J =* 7.6 Hz, 1H), 5.17 (s, 2H), 4.69-4.48 (m, 3H), 4.19 (s, 2H), 3.64 (s, 2H), 3.45 (s, 2H), 3.03 (s, 3H), 2.67-2.50 (m, 8H), 2.36 (s, 3H), 2.17 (s, 3H). |
| 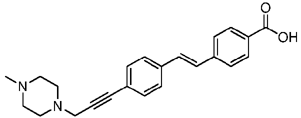 | 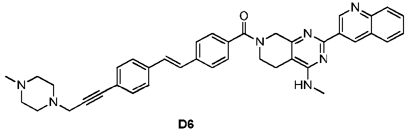 (LC-MS: C₄₀H₄₀N₇O, [M+H]⁺ 634.3; detected value: 634.6; purity: 94.9%) | ¹H NMR (400 MHz, Methanol-*d*₄) δ 9.77-9.68 (m, 1H), 9.20-9.11 (m, 1H), 8.10-8.04 (m, 2H), 7.80 (s, 1H), 7.72-7.64 (m, 3H), 7.62-7.50 (m, 4H), 7.43-7.41 (m, 2H), 7.27 (s, 2H), 4.86 (s, 1H), 4.52 (s, 1H), 4.09 (s, 1H), 3.79 (s, 1H), 3.69 (s, 2H), 3.28 (s, 4H), 3.15 (s, 3H), 2.93 (s, 4H), 2.84 (s, 3H), 2.59 (s, 2H). |

### Example 5, Synthesis of Compound E1 of Series E of the present invention:

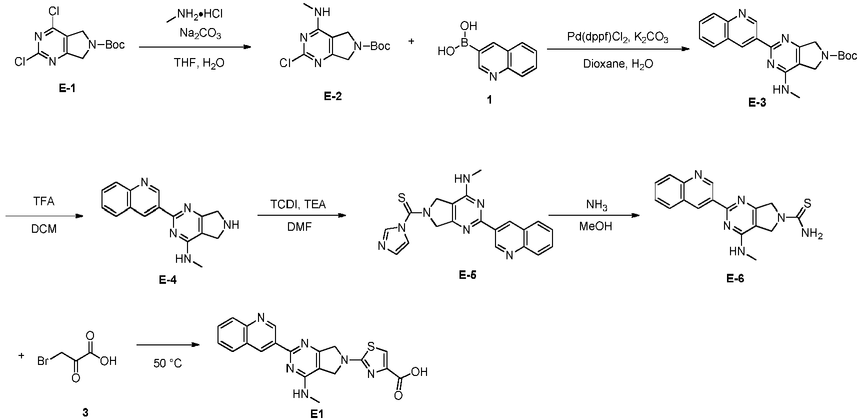

### Step 1, Synthesis of Compound E-2

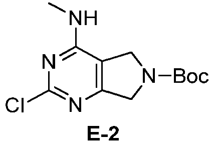

Compound **E-1** (260.14 mg, 999.44 µmol), methylamine hydrochloride (80.98 mg, 1.20 mmol), sodium carbonate (317.82 mg, 3.00 mmol) and tetrahydrofuran/water (4 mL/ 2mL) were added to a 50 mL reaction bottle, stirred at room temperature until the reaction was completed, and then water was added to quench the reaction (monitored by LC-MS). Saturated sodium chloride solution (10 mL) and ethyl acetate (3 × 15 mL) were used for extraction, and the organic phases were combined and dried over anhydrous sodium sulfate, evaporated to remove solvent, purified by MPLC, and concentrated under reduced pressure to remove solvent to obtain Compound **E-2** (113.64 mg, 441.81 µmol, 44.21% yield), LCMS: C₁₂H₁₈ClN₄O₂, (ESI) [M+H]: 285.1.

### Step 2, Synthesis of Compound E-3

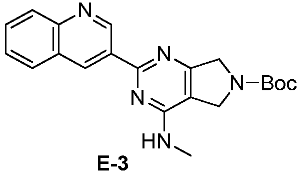

Under nitrogen protection, Compound **E-2** (113.64 mg, 441.81 µmol), Compound 1 (91.18 mg, 530.18 µmol), potassium carbonate (182.91 mg, 1.33 mmol), 1,4-dioxane and water (4 mL/1 mL) and Pd(dppf)Cl₂ (6.44 mg, 0.25 mol%) were added to a 50 mL reaction bottle in sequence. The reaction system was sealed and reacted under stirring at 80 °C for 2 hours, then the reaction was quenched (monitored by LC-MS). Saturated sodium chloride solution (20 mL) and ethyl acetate (3×20 mL) were used to complete the extraction, and the organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure to remove solvent to obtain Compound E-3 (92.41 mg, 245.85 µmol, 55.65% yield). LCMS (ESI): C₂₁H₂₄N₅O₂, [M+H]: 378.2.

### Step 3, Synthesis of Compound E-4

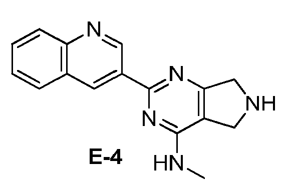

Compound **E-3** (92.41 mg, 245.85 µmol) was dissolved in dichloromethane (2 mL) in a 50 mL reaction bottle, and trifluoroacetic acid (1 mL) was then added dropwise, reacted under stirring at room temperature for 1 hour, and the solvent was removed by concentration under reduced pressure to obtain Compound **E-4** (crude product). LCMS: C₁₆H₁₆N₅, (ESI) [M+H]: 278.1.

### Step 4, Synthesis of Compound E-5

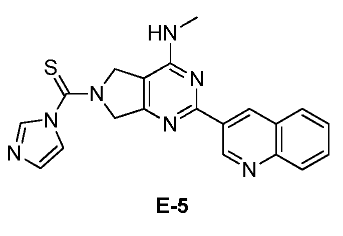

Compound **E-4** (crude product), TCDI (42.41 mg, 237.99 µmol) and DMF (5 mL) were added to a 50 mL reaction bottle in sequence. After stirring for 10 minutes, triethylamine (60.21 mg, 594.97 µmol, 82.98 µL) was added. After keeping the reaction at room temperature for 1 hour, the reaction was quenched (monitored by LC-MS), and saturated sodium chloride solution (20 mL) and dichloromethane (3 × 15 mL) were used to complete the extraction. The organic phases were combined, dried over anhydrous sodium sulfate, and concentrated under reduced pressure to remove solvent to obtain Compound E-5 (crude product), LCMS: C₂₀H₁₈N₇S, (ESI) [M+H]: 388.1.

### Step 5, Synthesis of Compound E-6

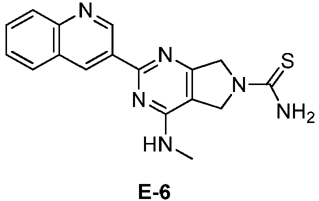

Compound E-5 (crude product) and methylamine alcohol solution (7 N, 1 mL) were added to a 50 mL reaction bottle, the reaction was carried out overnight at room temperature, and then quenched (monitored by LCMS), and the excess methylamine alcohol solution was removed by concentration under reduced pressure to obtain Compound **E-6** (crude product), LCMS: C₁₇H₁₇N₆S, (ESI) [M+H]: 337.1.

### Step 6, Synthesis of Compound E1

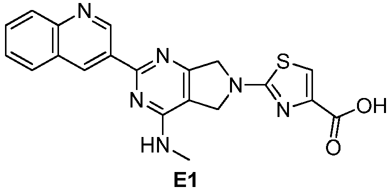

Compound **E-6** (crude product), Compound **3** (67.28 mg, 0.2 mmol) and ethanol (2 mL) were added to a 50 mL reaction bottle, and the reaction temperature was maintained at 50 °C for about 2 hours until the reaction was completed (monitored by LC-MS). The reaction solution was removed by concentration under reduced pressure and purified by MPLC to obtain Compound **E1** (7.50 mg, 18.43 µmol, 9.22% yield, 99.4% purity). LC-MS: C₂₀H₁₇N₆O₂S, [M+H]⁺ 405.1; detected value: 405.0. ¹H NMR (400 MHz, Methanol-d4) δ 9.73 (s, 1H), 9.43 (s, 1H), 8.10 (d, *J =* 2.0 Hz, 1H), 8.01 (d, *J =* 7.2 Hz, 1H), 7.92 (s, 1H), 7.74 (s, 1H), 7.22 (s, 1H), 4.44 (s, 2H), 4.23 (s, 2H), 3.16 (s, 3H).

According to the synthetic scheme of Compound **E1** above, Compound **E2** could be obtained by replacing Compound **3** with the raw material as listed in Table 10.

**Table 10. Compound E2**

| Raw material | Product structure | H-NMR data characterization |
|---|---|---|
| 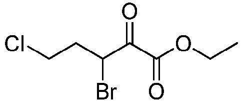 | 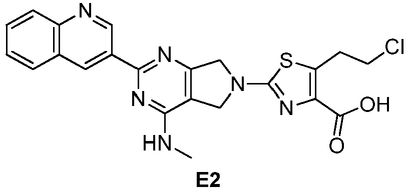 (LC-MS: C₂₂H₂₀ClN₆O₂S, [M+H]⁺ 467.1; detected value: 467.3; purity: 95.6%) | ¹H NMR (400 MHz, DMSO-*d₆*) δ 9.82 (d, *J =* 2.1 Hz, 1H), 9.25 (s, 1H), 8.20 (d, *J* = 8.0 Hz, 1H), 8.09 (d, *J* = 8.4 Hz, 1H), 7.84 (t, *J* = 8.0 Hz, 1H), 7.67 (d, *J* = 7.6 Hz, 1H), 7.65 (t, *J* = 4.0 Hz, 1H), 4.63 (s, 2H), 4.59 (s, 2H), 3.83 (t, *J* = 6.7 Hz, 2H), 3.50 (t, *J* = 6.7 Hz, 2H), 3.09 (d, *J =* 4.5 Hz, 3H). |

### Synthesis method of Compound E3 of Series E of the present invention:

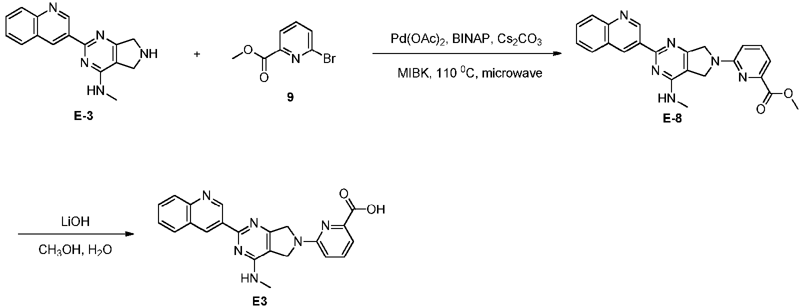

### Step 1, Synthesis of Compound E-8

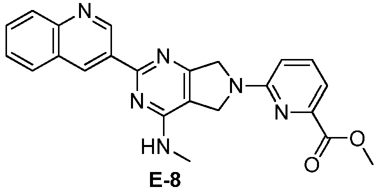

Under nitrogen protection, Compound **E-3** (42.50 mg, 153.25 µmol), Compound 9 (33.11 mg, 153.25 µmol), BINAP (12.06 mg, 30.65 µmol), cesium carbonate (151.18 mg, 459.75 µmol), and Pd₂(dba)₃ (15.41 mg, 0.25 mol%) were added to a 50 mL reaction bottle in sequence and dissolved in 10 mL MIBK. The reaction system was sealed and the reaction was carried out under microwaves at 110 °C for 2 hours (monitored by LC-MS). Saturated sodium chloride solution (10 mL) and ethyl acetate (3×10 mL) were used to complete extraction, and the organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure to remove solvent to obtain Compound **E-8** (24.11 mg, 58.49 µmol, 85.20% yield), LCMS: C₂₃H₂₁N₆O₂, (ESI) [M+H]: 413.2.

### Step 2, Synthesis of Compound E3

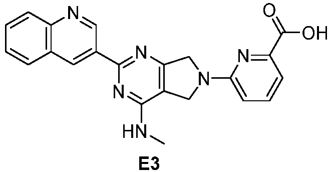

Compound **E-8** (24.11 mg, 58.49 µmol), lithium hydroxide (560.25 µg, 23.39 µmol), methanol (2 mL) and water (2 mL) were added to a 50 mL reaction bottle in sequence. The reaction was carried out under stirring at room temperature for 5 hours and then quenched (monitored by LC-MS). The pH value of the system was adjusted to 6-7 with 1N hydrochloric acid solution. The reaction solution was concentrated and purified by MPLC to obtain Compound **E3** (5.00 mg, 12.55 µmol, 10.35% yield, 98.1% purity). LC-MS: C₂₂H₁₉N₆O₂, [M+H]⁺ 399.2; detected value: 399.3. ¹H NMR (400 MHz, DMSO-*d₆*) δ 9.83 (s, 1H), 9.28 (s, 1H), 8.20 (d, *J =* 8.0 Hz, 1H), 8.09 (d, *J =* 8.3 Hz, 1H), 7.85 (t, *J =* 8.0 Hz, 1H), 7.76-7.67 (m, 3H), 7.33 (d, *J =* 7.2 Hz, 1H),, 4.68-4.64 (m, 4H), 3.09 (d, *J =* 4.2 Hz, 3H). Purity > 95%.

According to the synthetic scheme of Compound **E3** above, Compounds **E4** and **E5** could be obtained by replacing Compound **9** with the raw materials as listed in Table 11.

**Table 11. Compounds E4 and E5**

| Raw material | Product structure | H-NMR data characterization |
|---|---|---|
| 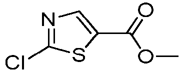 | 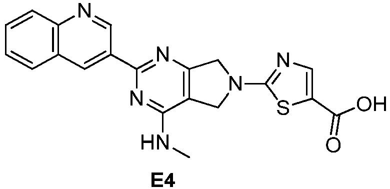 (LC-MS: C₂₀H₁₇N₆O₂S, [M+H]⁺ 405.1; detected value: 405.1; purity: 90.6%) | ¹H NMR (400 MHz, DMSO-*d₆*) δ 9.78 (s, 1H), 9.27 (s, 1H), 8.20 (d, *J =* 8.0 Hz, 1H), 8.09 (d, *J =* 8.3 Hz, 1H), 7.94 - 7.86 (m, 1H), 7.77 - 7.70 (m, 2H), 4.64 (s, 2H), 4.52 (s, 2H), 3.08 (s, 3H). |
| 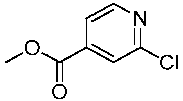 | 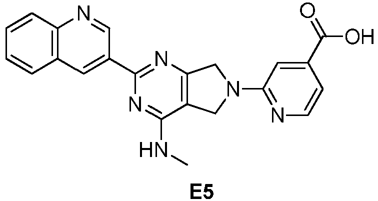 (LC-MS: C₂₂H₁₉N₆O₂, [M+H]⁺ 399.2; detected value: 399.3; purity: 96.0%) | ¹H NMR (400 MHz, DMSO-*d₆*) δ 9.85 (s, 1H), 9.25 (s, 1H), 8.32 (d, *J =* 5.2 Hz, 1H), 8.20 (d, *J =* 8.1 Hz, 1H), 8.09 (d, *J =* 8.4 Hz, 1H), 7.83 (t, *J* = 7.6 Hz, 1H), 7.67 (t, *J =* 4.4 Hz, 1H), 7.58 (d, *J* = 5.2 Hz, 1H), 7.12-7.05 (m, 1H), 6.99 (s, 1H) , 4.73 (s, 2H), 4.65 (s, 2H), 3.11 (d, *J =* 4.4 Hz, 3H). |

### Synthesis of Compound E6 of Series E of the present invention:

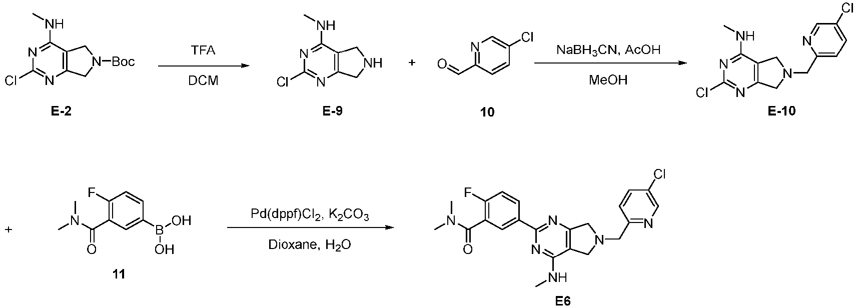

### Step 1, Synthesis of Compound E-9

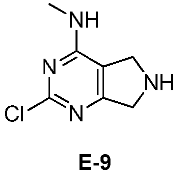

In a 50 mL reaction bottle, E-2 (113.64 mg, 441.81 µmol) was dissolved in dichloromethane (4 mL), then trifluoroacetic acid (1.5 mL) was added dropwise, stirred and reacted at room temperature for 0.5 hours, and concentrated under reduced pressure to remove solvent to obtain Compound **E-9** (crude product), LCMS: C₇H₁₀ClN₄, (ESI) [M+H]: 185.1.

### Step 2, Synthesis of Compound E-10

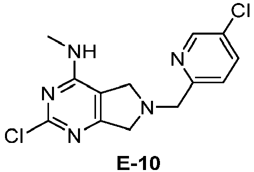

Compound **E-9** (crude), Compound 10 (79.91 mg, 0.51 mmol), NaBH₃CN (32.05 mg, 0.51 mmol), acetic acid (0.5 mL) and methanol (5 mL) were added to a 50 mL reaction bottle in sequence. After stirring at room temperature for 2 hours, the reaction was quenched (monitored by LC-MS), the mixture was evaporated to remove solvent, purified by MPLC, and concentrated under reduced pressure to remove solvent to obtain Compound **E-10** (142.16 mg, 0.46 mmol, 90.02% yield), LCMS: C₁₃H₁₄C₁₂N₅, (ESI) [M+H]: 310.1.

### Step 3, Synthesis of Compound E6

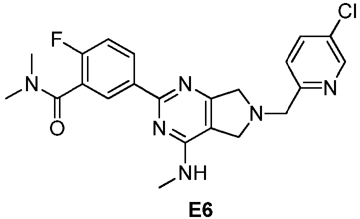

Under nitrogen protection, Compound **E-10** (47.00 mg, 151.52 µmol), Compound **11** (31.97 mg, 151.52 µmol), potassium carbonate (41.82 mg, 303.05 µmol), and Pd(dppf)Cl₂ (5.55 mg, 0.25 mol%) were added to a 50 mL reaction bottle in sequence. The reaction system was sealed and reacted under stirring at 80 °C for 8 hours, and then the reaction was quenched (monitored by LC-MS). Extraction was completed with saturated sodium chloride solution (20 mL) and ethyl acetate (3 × 25 mL). The organic phases were combined and dried over anhydrous sodium sulfate, evaporated to remove solvent, separated by column chromatography with an eluent having a volume ratio of petroleum ether/ethyl acetate = 1:100 to 1:5, and concentrated under reduced pressure to remove solvent to obtain Compound **E6** (6.90 mg, 15.74 µmol, 10.39% yield, 99.9% purity). LC-MS: C₂₂H₂₃ClFN₆O, [M+H]⁺ 441.1; detected value: 441.3. ¹H NMR (400 MHz, Methanol-d4) δ 8.56 (d, *J =* 2.4 Hz, 1H), 8.40-8.37 (m, 1H), 8.29 (dd, *J =* 6.0, 2.4 Hz, 1H), 7.88 (dd, *J =* 4.0, 2.4 Hz, 1H), 7.74 (d, *J =* 2.0 Hz, 1H), 7.49 (t, *J =* 8.0 Hz, 1H), 7.39-738 (m, 2H), 5.56 (s, 2H), 3.32 (s, 3H), 3.17 (s, 3H), 3.05 (s, 3H). Purity> 95%.

According to the synthetic scheme of Compound **E6** above, Compounds **E7** to **E9** and **E11** could be obtained by replacing Compound **11** with the raw materials as listed in Table 12.

**Table 12. Compounds E7, E8, E9, E11**

| Raw material | Product structure | H-NMR data characterization |
|---|---|---|
| 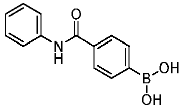 | 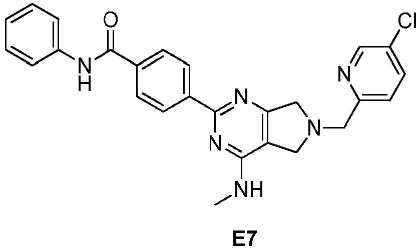 (LC-MS: C₂₆H₂₄ClN₆O, [M+H]⁺ 471.2; detected value: 471.2; purity: 93.1%) | ¹H NMR (400 MHz, Methanol-*d*₄) δ 8.72 (d, *J* = 2.5 Hz, 1H), 8.50 (d, *J* = 8.4 Hz, 2H), 8.06-7.95 (m, 3H), 7.71 (d, *J = 8.0 Hz,* 2H), 7.55 (d, *J =* 8.3 Hz, 1H), 7.37 (t, *J* = 7.9 Hz, 2H), 7.17 (t, *J* = 7.4 Hz, 1H), 4.72-4.66 (m, 6H), 3.14 (s, 3H). |
| 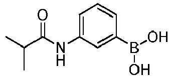 | 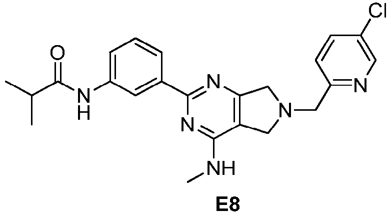 (LC-MS: C₂₃H₂₆ClN₆O, [M+H]⁺ 437.2; detected value: 437.1; purity: 91.2%) | ¹H NMR (400 MHz, Methanol-*d*₄) δ 8.70 (d, *J =* 6.0 Hz, 1H), 8.63 (d, *J* = 2.0 Hz, 1H), 8.06 (dt, *J =* 7.8, 1.4 Hz, 1H), 7.94 (dt, *J =* 8.4, 2.4 Hz, 1H), 7.69-7.67 (m, 1H), 7.55 (t, *J =* 8.3 Hz, 1H), 7.42 (t, *J = 7.9* Hz, 1H), 4.80 (s, 2H), 4.68 (d, *J* = 7.0 Hz, 2H), 4.60 (s, 2H), 3.15 (s, 3H), 2.66 (q, *J* = 6.8 Hz, 1H), 1.22 (d, *J* = 6.8 Hz, 6H). |
| 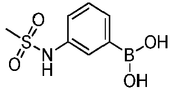 | 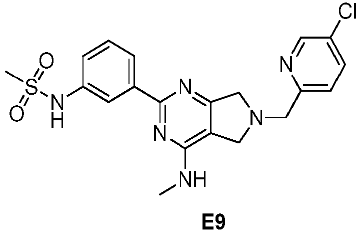 (LC-MS: C₂₀H₂₂ClN₆O₂S, [M+H]⁺ 445.1; detected value: 445.0; purity: 95.4%) | ¹H NMR (400 MHz, Methanol-*d*₄) δ 8.71 (d, *J* = 2.0 Hz, 1H), 8.31 (s, 1H), 8.12 (d, *J* = 5.6 Hz, 1H), 7.97 (dd, *J* = 8.0, 2.4 Hz, 1H), 7.4 (d, *J* = 8.0 Hz, 1H), 7.45 (t, *J* = 8.0 Hz, 1H), 7.37 (d, *J* = 8.0 Hz, 1H), 4.83 (s, 2H), 4.69-4.63 (m, 4H), 3.13 (s, 3H), 2.96 (s, 3H). |
| 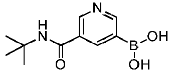 | 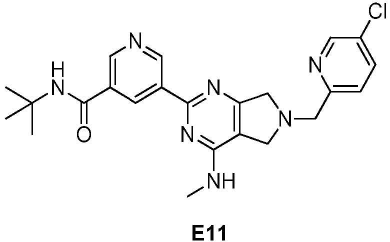 (LC-MS: chemical formula: C₂₃H₂₇ClN₇O, [M+H]⁺452.2; detected value: 452.3; purity: 99.4%) | H NMR (400 MHz, Methanol-*d*₄) δ 9.47 (d, *J* = 2.4 Hz, 1H), 9.13 (d, *J* = 2.0 Hz, 1H), 8.93-8.83 (m, 1H), 8.56 (d, *J* = 2.4 Hz, 1H), 7.89 (dd, *J =* 8.3, 2.5 Hz, 1H), 7.78 (d, *J =* 2.0 Hz, 1H), 7.46-7.37 (m, 2H), 5.60 (d, *J* = 15.5 Hz, 4H), 3.36 (s, 3H), 1.50 (s, 9H). |

### Example 6, Synthesis method of Compound F6 of Series F of the present invention:

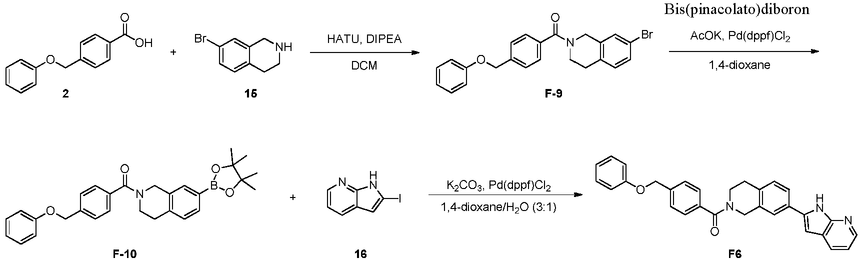

### Step 1, Synthesis of Compound F-9

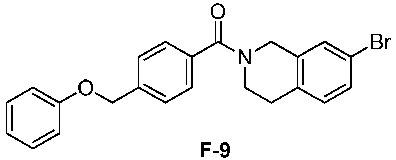

Compound **2** (100.00 mg, 438.13 µmol), DIPEA (141.56 mg, 1.10 mmol, 190.78 µL), HATU (183.14 mg, 481.94 µmol) and DCM (2 mL) were added to a 50 mL reaction bottle in sequence. After the temperature of the reaction system dropped to 0 °C, Compound **15** (92.92 mg, 438.13 µmol) was added. The reaction was carried out under stirring and ice bath conditions for 0.5 hours, and then quenched (monitored by LC-MS). Saturated sodium chloride solution (20 mL) and ethyl acetate (3×20 mL) were used to complete the extraction, and the organic phases were combined and dried over anhydrous sodium sulfate, evaporated to remove solvent, purified by MPLC, and the solvent was removed by concentration under reduced pressure to obtain Compound **F-9** (182.00 mg, 430.96 µmol, 98.36% yield). LCMS: C₂₃H₂₁BrNO₂, (ESI) [M+H]: 422.1.

### Step 2, Synthesis of Compound F-10

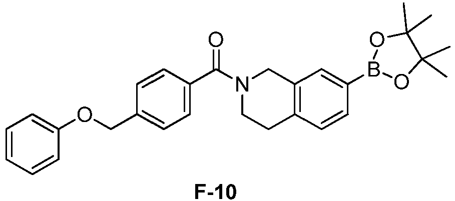

Under nitrogen protection, Compound **F-9** (182.00 mg, 430.96 µmol), bis(pinacolato)diboron (240.76 mg, 948.11 µmol), potassium acetate (105.74 mg, 1.08 mmol), 1,4-dioxane and water (4 mL/1 mL) and Pd(dppf)Cl₂ (31.98 mg, 0.25 mol%) were added to a 50 mL reaction bottle in sequence. The reaction system was sealed, stirred and reacted at 100 °C for 1 hour, and then the reaction was quenched (monitored by LC-MS). Saturated sodium chloride solution (20 mL) and ethyl acetate (3 × 20 mL) were used to complete the extraction, and the organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure to remove solvent to obtain Compound **F-10** (202.00 mg, 430.36 µmol, 99.86% yield), LCMS: C₂₉H₃₃BNO₄, (ESI) [M+H]: 470.2.

### Step 3, Synthesis of Compound F6

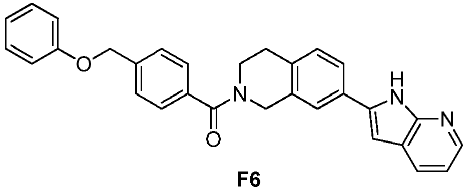

Under nitrogen protection, Compound **F-10** (202.00 mg, 430.36 µmol), Compound **16** (126.02 mg, 516.43 µmol), potassium carbonate (148.70 mg, 1.08 mmol), and Pd(dppf)Cl₂ (31.93 mg, 0.25 mol%) were added in a 50 mL reaction bottle in sequence and dissolved in a mixed solvent of 1,4-dioxane (3 mL) and water (1 mL). The reaction system was sealed and stirred at 80 °C for 1 hour, and then the reaction was quenched (monitored by LC-MS). Saturated sodium chloride solution (20 mL) and ethyl acetate (3×20 mL) were used to complete extraction. The organic phases were combined and dried over anhydrous sodium sulfate, evaporated to remove solvent, separated by column chromatography with an eluent having a volume ratio of petroleum ether/ethyl acetate = 1:100 to 1:5, and concentrated under reduced pressure to remove solvent to obtain Compound **F6** (25.00 mg, 54.40 µmol, 12.64% yield, 97.7% purity). LC-MS: C₃₀H₂₆N₃O₂, [M+H]⁺ 460.2; detected value: 460.2. ¹H NMR (400 MHz, Chloroform-*d*) δ 8.23 (s, 2H), 7.78-7.72 (m, 2H), 7.60-7.57 (m, 2H), 7.53-7.51 (m, 2H), 7.35-7.26 (m, 4H), 7.05-6.92 (m, 4H), 5.16 (s, 2H), 4.03 (s, 1H), 3.98-3.87 (m, 2H), 3.73 (s, 1H), 3.05-2.96 (m, 2H). Purity > 95%.

### Example 7, Synthesis of Compound 1A of Series A of the present invention:

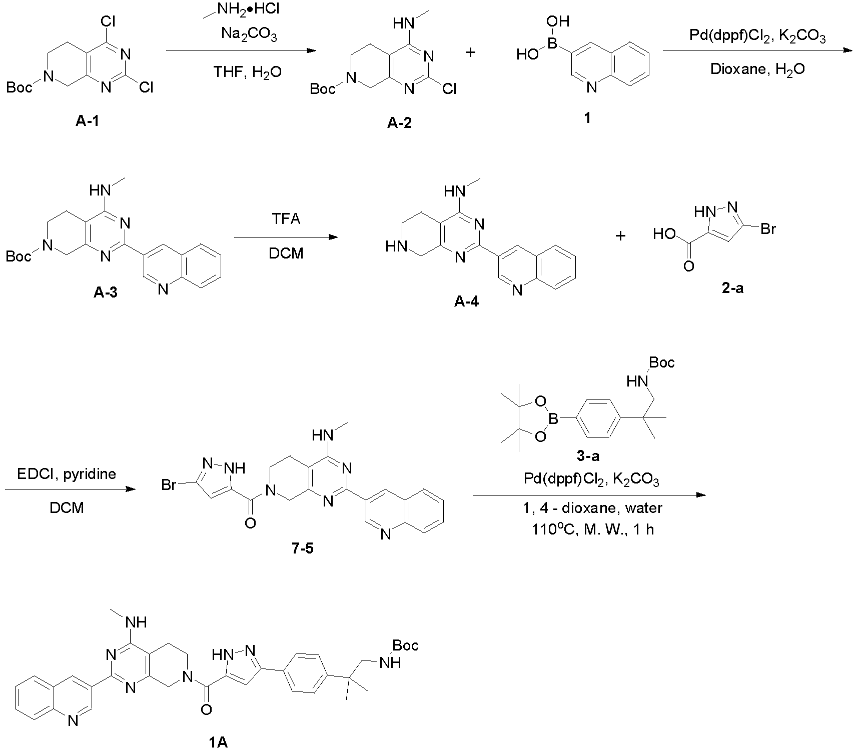

### Step 1, Synthesis of Compound A-2

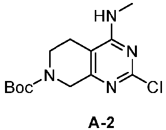

Compound **A-1** (304.00 mg, 999.44 µmol), methylamine hydrochloride (80.98 mg, 1.20 mmol), sodium carbonate (317.82 mg, 3.00 mmol) and tetrahydrofuran/water (4 mL/2 mL) were added to a 50 mL reaction bottle, stirred at room temperature until the reaction was completed, and then water was added to quench the reaction (monitored by LC-MS). Saturated sodium chloride solution (10 mL) and ethyl acetate (3 × 15 mL) were used for extraction, and the organic phases were combined and dried over anhydrous sodium sulfate, evaporated to remove solvent, purified by MPLC, and concentrated under reduced pressure to remove solvent to obtain Compound **A-2** (132.00 mg, 441.81 µmol, 44.21% yield). The obtained compound was characterized: MS (ESI) m/z = 299.1 (M+1)⁺

### Step 2, Synthesis of Compound A-3

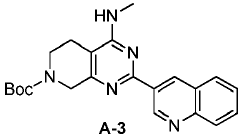

Under nitrogen protection, Compound **A-2** (132.00 mg, 441.81 µmol), Compound 1 (91.18 mg, 530.18 µmol), potassium carbonate (182.91 mg, 1.33 mmol), 1,4-dioxane and water (4 mL/1 mL) and Pd(dppf)Cl₂ (6.44 mg, 0.25 mol%) were added to a 50 mL reaction bottle in sequence. The reaction system was sealed, the reaction was carried out under stirring at 80 °C for 2 h, and then quenched (monitored by LC-MS). Saturated sodium chloride solution (20 mL) and ethyl acetate (3×20 mL) were used to complete extraction, and the organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure to remove solvent to obtain Compound **A-3** (96.00 mg, 245.85 µmol, 55.65% yield). The obtained compound was characterized: MS (ESI) m/z = 392.2 (M+1)⁺

### Step 3, Synthesis of Compound A-4

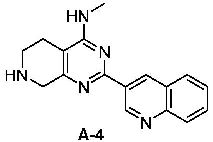

Compound **A-3** (96.00 mg, 245.85 µmol) was dissolved in dichloromethane (2 mL) in a 50 mL reaction bottle, and trifluoroacetic acid (1 mL) was then added dropwise. The reaction was carried out under stirring at room temperature for 3 hours, and the solvent was removed by concentration under reduced pressure to obtain Compound **A-4** (crude product). The obtained compound was characterized: MS (ESI) m/z = 292.2 (M+1)⁺.

### Step 4, Synthesis of Compound 7-5

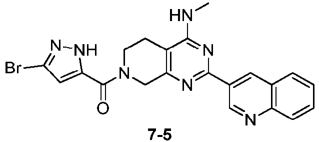

DCM (2 mL), Compound **2-a** (191 mg, 1.00 mmol), EDCI (229 mg, 1.20 mmol) and pyridine (159 mg, 2.00 mmol, 161 uL) were added to a 10 mL reaction bottle in sequence. After the temperature of the reaction system dropped to 0°C, Compound **A-4** (292 mg, 1.00 mmol) was added. The reaction was carried out under stirring and ice bath conditions for 0.5 hours, and then quenched. After purification by MPLC, the solvent was removed by concentration under reduced pressure to obtain Compound 7-5 (199 mg, 457.1 µmol, 45.71% yield). The obtained compound was characterized: MS (ESI) m/z = 464.1 (M+1)⁺.

### Step 5.1, Synthesis of Compound 3-a

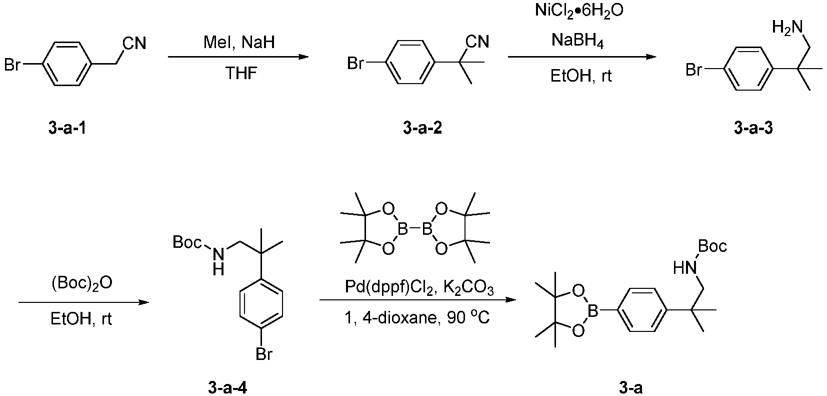

### Step 5.1.1, Synthesis of Compound 3-a-2

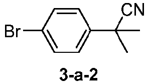

Under nitrogen protection, 10 mL of THF, Compound **3-a-1** (392 mg, 2.00 mmol) and iodomethane (709.84 mg, 5.00 mmol) were added to a 50 mL flask. After cooling to 0 °C, sodium hydride (119.96 mg, 5.00 mmol) was added. The reaction system was warmed to room temperature, stirred and reacted at room temperature for 3 hours. When LCMS detection showed the reaction was completed, water was added to quench the reaction. Saturated NaCl solution (20.0 mL) and ethyl acetate (3×20 mL) were used to complete extraction. The organic phases were combined and dried over anhydrous sodium sulfate, evaporated to remove solvent to obtain Compound **3-a-2** (380 mg, 1.70 mmol, 84.80% yield). The obtained compound was characterized: MS (ESI) m/z = 224.0 (M+1)⁺.

### Step 5.1.2, Synthesis of Compound 3-a-3

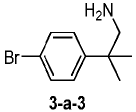

Compound **3-a-2** (380 mg, 1.70 mmol), 20 mL of anhydrous ethanol, and nickel dichloride hexahydrate (403.03 mg, 1.70 mmol) were added to a 50 mL flask. Sodium borohydride (320.76 mg, 8.48 mmol) was added after cooling to 0 °C. The reaction system was heated to room temperature, stirred and reacted at room temperature for 1 hour. When LCMS detection showed that the reaction was completed, 5 mL of 1 N HCl solution was added, and 1 N NaOH solution was added to adjust pH value to 10. The obtained mixture was filtered to obtain a filtrate containing Compound **3-a-3**, which was used directly in the next reaction.

The obtained compound was characterized: MS (ESI) m/z = 228.0 (M+1)⁺.

### Step 5.1.3, Synthesis of Compound 3-a-4

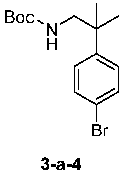

Di-tert-butyl dicarbonate (370.60 mg, 1.70 mmol) was added to the filtrate containing Compound **3-a-3** obtained in the previous step. The obtained mixture was stirred at room temperature for 1 hour. When LCMS detection showed that the reaction was completed, ethyl acetate (3×20 mL) was used to complete extraction. The organic phases were combined, dried over anhydrous sodium sulfate, evaporated to remove solvent, and separated by column chromatography to obtain Compound **3-a-4** (500 mg, crude product). The obtained compound was characterized: MS (ESI) m/z = 328.1 (M+1)⁺.

### Step 5.1.4, Synthesis of Compound 3-a

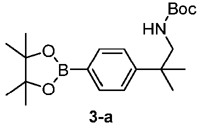

Under nitrogen protection, Compound **3-a-4** (500 mg, crude product), bis(pinacolato)diboron (676.92 mg, 2.67 mmol), K₂CO₃ (294.29 mg, 2.13 mmol), 1,4-dioxane (10 mL) and Pd(dppf)Cl₂ (38.97 mg, 53.31 µmol) were added to a 50 mL reaction bottle in sequence. The reaction was carried out under stirring and nitrogen protection at 90°C for 2 hours, and then quenched (monitored by LC-MS). Saturated NaCl solution (20.0 mL) and ethyl acetate (3×20 mL) were used to complete extraction, and the organic phases were combined, dried over anhydrous sodium sulfate, evaporated to remove solvent, separated by column chromatography with an eluent having a volume ratio of petroleum ether/ethyl acetate = 30:1, and concentrated under reduced pressure to remove solvent to obtain Compound **3-a** (143 mg, 381.02 µmol, 35.73% yield). The obtained compound was characterized: MS (ESI) m/z = 376.3 (M+1)⁺.

### Step 5.2, Synthesis of Compound 1A

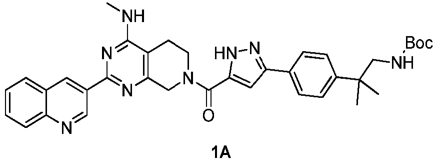

Under nitrogen protection, Compound **3-a** (48.50 mg, 129.23 µmol), Compound **7-5** (50 mg, 107.69 µmol), K₂CO₃ (29.72 mg, 215.38 µmol), 1,4-dioxane and water (4 mL/1 mL) and Pd(dppf)Cl₂ (15.74 mg, 21.54 µmol) were added to a 50 mL reaction bottle in sequence. The reaction system was sealed, and the reaction was carried out under stirring at 110 °C for 1 hour, and then quenched (monitored by LC-MS). Saturated NaCl solution (20.0 mL) and ethyl acetate (3×20 mL) were used for extraction. The organic phases were combined and dried over anhydrous sodium sulfate, evaporated to remove solvent, separated by MPLC, and concentrated under reduced pressure to remove solvent to obtain Compound **1A** (30 mg, 43.81 µmol, 40.68% yield, 92.4% purity). The obtained compound was characterized: MS (ESI) m/z = 633.4 (M+1)⁺.

¹H NMR (400 MHz, DMSO-*d₆*) δ 9.74-9.71 (m, 1H), 9.36-9.31 (m, 1H), 8.28 (t, *J =* 4.8 Hz, 1H), 8.26 (t, *J =* 4.8 Hz, 1H), 7.98 (d, *J =* 7.2 Hz, 1H), 7.82-7.71 (m, 3H), 7.47 (d, *J =* 7.2 Hz, 2H), 7.15 (s, 1H), 5.17 (s, 1H), 4.84 (s, 1H), 4.27 (s, 1H), 4.03 (s, 1H), 3.14 (s, 5H), 2.72-2.66 (m, 2H), 1.33 (d, *J =* 6.8 Hz, 9H), 1.25 (s, 6H).

### Example 8. Synthesis of Compound 2A of Series A of the present invention:

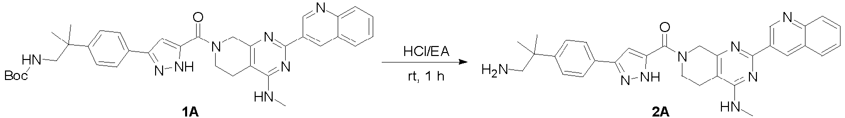

Compound **1A** (30 mg, 43.81 µmol) was dissolved in a mixed solution of 6 N HCl and ethyl acetate (5 mL) in a 50 mL reaction bottle, stirred and reacted at room temperature for 0.5 hours, and concentrated under reduced pressure to remove the solvent to obtain Compound **2A** (24 mg, 37.95 µmol, 80.05% yield, 90% purity). The obtained compound was characterized: MS (ESI) m/z =533.5 (M+1)⁺

### Example 9, Synthesis of Compound 3A of Series A of the present invention:

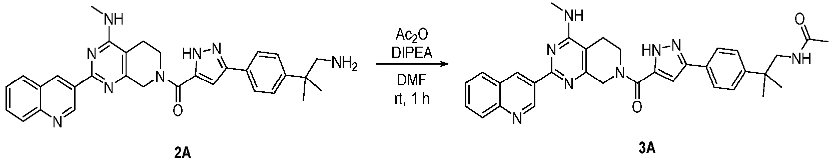

Compound **2A** (20 mg, 37.55 µmol), DMF (1 mL), N,N-diisopropylethylamine (9.71 mg, 75.10 µmol, 13.08 uL) and acetic anhydride (3.83 mg, 37.55 µmol) were added to a 10 mL flask at room temperature. After stirring at room temperature for 1 hour, LC-MS showed that the reaction was completed, and then the solvent was evaporated. After MPLC separation and purification, the solvent was removed by concentration under reduced pressure to obtain Compound **3A** (2.5 mg, 4.29 µmol, 11.41% yield, 98.5% purity). The obtained compound was characterized: MS (ESI) m/z =575.3 (M+1)⁺. ¹H NMR (400 MHz, Methanol -d4) δ 9.77-9.75 (m, 1H), 9.49-9.48 (m, 1H), 8.27 (t, *J =* 4.8 Hz, 1H), 8.20 (t, *J =* 4.8 Hz, 1H), 8.05 (t, *J =* 7.2 Hz, 1H), 7.86 (s, 1H), 7.69 (d, *J =* 8.0 Hz, 2H), 7.51 (d, *J =* 8.0 Hz, 2H), 7.02 (s, 1H), 5.30 (s, 1H), 5.08 (s, 1H), 4.39 (s, 1H), 4.21 (s, 1H), 3.41 (s, 2H), 3.34 (s, 2H), 3.26 (s, 3H), 1.88 (s, 3H), 1.35 (s, 6H).

### Example 10, Synthesis of Compounds 4A, 5A, 6A of Series A of the present invention:

According to the synthesis scheme of Compound **1A**, Compounds **4A**, **5A**, **6A** could be obtained by replacing the raw material **3-a** used in Step **1** with the raw materials as listed in Table 13.

**Table 13. Compounds 4A to 6A**

| Raw material | Product structure | H-NMR data characterization |
|---|---|---|
| 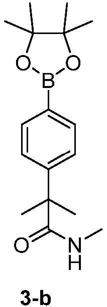 | 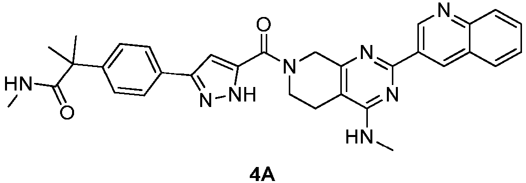 (LC-MS: C₃₂H₃₂N₈O₂, [M+H]⁺ 561.3; detected value: 561.5; purity: 99.9%) | ¹H NMR (400 MHz, Methanol-*d*₄) δ 9.76-9.75 (m, 1H), 9.49-9.48 (m, 1H), 8.26 (t, *J* = 4.8 Hz, 1H), 8.19 (t, *J* = 4.8 Hz, 1H), 8.04 (t, *J* = 7.2 Hz, 1H), 7.86 (s, 1H), 7.70 (d, *J* = 8.0 Hz, 2H), 7.44 (d, *J* = 8.0 Hz, 2H), 7.03 (s, 1H), 5.29 (s, 1H), 5.08 (s, 1H), 4.38 (s, 1H), 4.13 (s, 1H), 3.26 (s, 3H), 2.71 (s, 5H), 1.56 (s, 6H). |
| 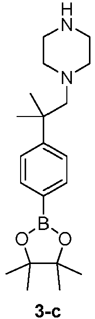 | 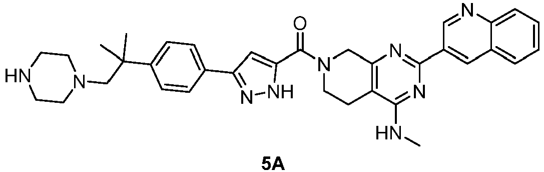 (LC-MS: C₃₅H₃₉N₉O, [M+H]⁺ 602.3; detected value: 602.4; purity: 94.9%) | ¹H NMR (400 MHz, Methanol-*d*₄) δ 9.79-9.72 (m, 1H), 9.51-9.41 (m, 1H), 8.32-8.20 (m, 2H), 8.13-7.86 (m, 2H), 7.76 (d, *J* = 8.0 Hz, 2H), 7.44 (d, *J* = 8.0 Hz, 2H), 7.03 (s, 1H), 5.28 (s, 1H), 5.08 (s, 1H), 4.70 (s, 2H), 4.14 (s, 1H),3.19-2.94 (m, 5H), 2.87-2.77 (m, 3H), 2.71 (s, 3H), 2.52 (t, *J* = 7.2 Hz, 4H), 1.35 (s, 6H). |
| 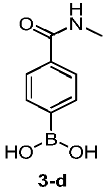 | 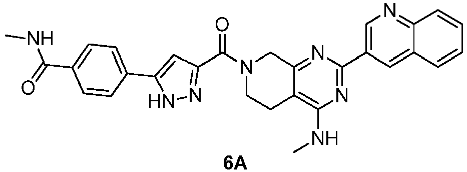 (LC-MS: C₂₉H₂₆N₈O₂, [M+H]⁺ 519.2; detected value: 519.4; purity: 99.8%) | ¹H NMR (400 MHz, DMSO-*d₆*) δ 9.73-9.70 (m, 1H), 9.37-9.33 (m, 1H), 8.26 (s, 1H), 8.17 (s, 1H), 8.00-7.99 (m,1H), 7.92 (s, 4H), 7.81 (d, *J* = 6.0 Hz, 1H), 7.22 (s, 1H), 5.13 (s, 1H), 4.85 (s, 1H), 4.24 (s, 1H), 4.01 (s, 1H), 3.13 (s, 3H), 2.83 (s, 3H), 2.66-2.55 (m, 2H). |

### Synthesis of Compound 3-b

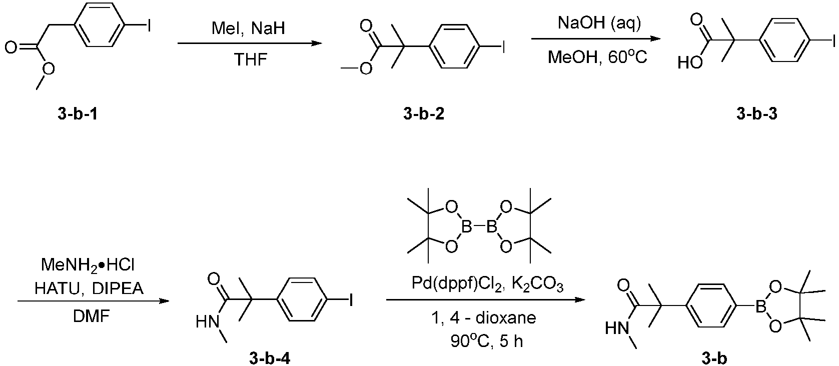

### Step 1, Synthesis of Compound 3-b-2

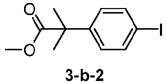

Compound **3-b-1** (1 g, 3.62 mmol) and THF (15 mL) were added to a dry 50 mL flask at room temperature in sequence. After cooling to 0°C, sodium hydride (260 mg, 14.49 mmol) was added. After stirring at 0°C for 1 hour, iodomethane (220 g, 14.49 mmol) was added and the temperature was gradually raised to room temperature. When LCMS showed that the reaction was completed, water was added to quench the reaction. The extraction was completed with ethyl acetate (3×15 mL), the organic phases were combined, dried over anhydrous sodium sulfate, and evaporated to remove solvent to obtain 1.1 g of crude product **3-b-2** , which was directly used in the next step. The obtained compound was characterized: MS (ESI) m/z = 305.0 (M+1)⁺.

### Step 2, Synthesis of Compound 3-b-3

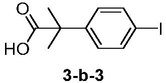

Compound **3-b-2** (1.1 g, crude), sodium hydroxide (104.23 mg, 2.48 mmol), methanol (5 mL) and water (5 mL) were added to a 50 mL reaction bottle in sequence. The reaction was carried out under stirring at 60°C for 12 hours, and then quenched (monitored by LC-MS). The pH value of the system was adjusted to 4 with 1N hydrochloric acid solution. The reaction solution was concentrated to obtain Compound **3-b-3** (150.00 mg, crude), which was directly used in the next step. The obtained compound was characterized: MS (ESI) m/z = 291.0 (M+1)⁺.

### Step 3, Synthesis of Compound 3-b-4

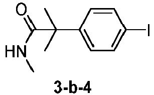

Compound **3-b-3** (1 g, 3.62 mmol), DIPEA (300.47 mg, 2.32 mmol, 404.94 uL), HATU (353.38 mg, 929.95 µmol) and DMF (15 mL) were added to a 50 mL reaction bottle in sequence. After the temperature of the reaction system dropped to 0°C, methylamine hydrochloride (135.00 mg, 774.96 µmol) was added. After the reaction was carried out under stirring and ice bath conditions for 1 hour, the reaction was quenched (monitored by LC-MS). Saturated NaCl solution (20.0 mL) and ethyl acetate (3×20.0 mL) were used for extraction. The organic phases were combined, dried over anhydrous sodium sulfate, evaporated to remove solvent, purified by MPLC, and concentrated under reduced pressure to remove solvent to obtain Compound **3-b-4** (230.00 mg, 495.14 µmol, yield 63.89%). The obtained compound was characterized: MS (ESI) m/z = 304.0 (M+1)⁺.

### Step 4, Synthesis of Compound 3-b

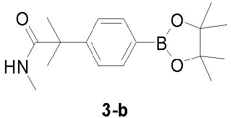

Under nitrogen protection, Compound **3-b-4** (303.14 mg, 1 mmol), bis(pinacolato)diboron (634.85 mg, 2.50 mmol), K₂CO₃ (276.00 mg, 2.00 mmol), 1,4-dioxane (10 mL) and Pd(dppf)Cl₂ (73.10 mg, 100.00 µmol) were added to a 50 mL reaction bottle in sequence. The reaction system was sealed, the reaction was carried out under stirring at 90°C for 5 hours (monitored by LC-MS), and then the resulting mixture was separated by MPLC, and concentrated under reduced pressure to remove solvent to obtain Compound 3-b (20 mg, 65.96 µmol, 6.60% yield). The obtained compound was characterized: MS (ESI) m/z = 304.2 (M+1)⁺.

### Synthesis of Compound 3-c

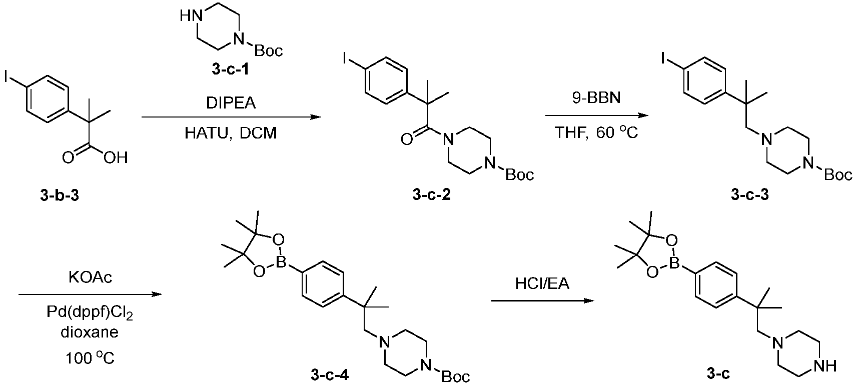

### Step 1, Synthesis of Compound 3-c-2

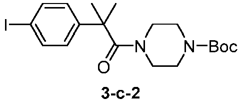

Compound **3-b-3** (1 g, crude, 3.45 mmol), DIPEA (1.34 mg, 10.34 mmol, 1.80 mL), HATU (1.44 g, 3.79 mmol) and anhydrous DCM (15 mL) were added to a 50 mL reaction bottle in sequence. After the temperature of the reaction system dropped to 0°C, Compound 3-c-1 (642.03 mg, 3.45 mmol) was added. After the reaction was carried out under stirring and ice bath conditions for 1 hour, the reaction was quenched (monitored by LC-MS). Saturated NaCl solution (20.0 mL) and ethyl acetate (3×20.0 mL) were used to complete extraction. The organic phases were combined, dried over anhydrous sodium sulfate, evaporated to remove solvent, purified by MPLC, and concentrated under reduced pressure to remove solvent to obtain Compound **3-c-2** (1.3 g, crude). The obtained compound was characterized: MS (ESI) m/z = 459.1 (M+1)⁺

### Step 2, Synthesis of Compound 3-c-3

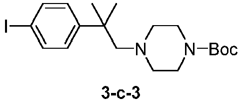

Compound **3-c-2** (1.3 g, crude) and 9-BBN (10 mL) were added to a 50 mL reaction bottle and dissolved in THF (20 mL). After heating to 60 °C and stirring for 5 hours (monitored by LC-MS) under nitrogen protection, 20 mL of water was added to quench the reaction. Ethyl acetate (3×15 mL) was used for extraction, the organic phases were combined, dried over anhydrous sodium sulfate, evaporated to remove solvent, purified by MPLC, and concentrated under reduced pressure to remove solvent to obtain Compound **3-c-3** (300 mg, 675.15 µmol, 23.80% yield). The obtained compound was characterized: MS (ESI) m/z = 459.1 (M+1)⁺

### Step 3, Synthesis of Compound 3-c-4

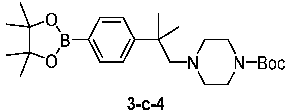

Under nitrogen protection, Compound 3-c-3 (335.49 mg, 755.00 µmol), bis(pinacolato)diboron (230.07 mg, 906.01 µmol), KOAc (369.95 mg, 3.78 mmol), 1,4-dioxane and water (4 mL/1 mL) and Pd(dppf)Cl₂ (55.27 mg, 75.50 µmol) were added to a 50 mL reaction bottle in sequence. The reaction system was sealed, and the reaction was carried out under stirring at 100°C for 2 hours, and then quenched (monitored by LC-MS). Saturated NaCl solution (20.0 mL) and ethyl acetate (3×20 mL) were used to complete extraction. The organic phases were combined and dried over anhydrous sodium sulfate, evaporated to remove solvent, separated by MPLC, and concentrated under reduced pressure to remove solvent to obtain Compound **3-c-4** (100 mg, 290.45 µmol, 38.47% yield). The obtained compound was characterized: MS (ESI) m/z = 445.3 (M+1)⁺

### Step 4, Synthesis of Compound 3-c

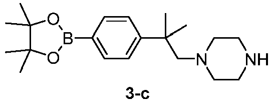

Compound **3-c-4** (100 mg, 290.45 µmol) was dissolved in a mixed solution of 6 N HCl and ethyl acetate (10 mL) in a 50 mL reaction bottle, stirred and reacted at room temperature for 0.5 hours, and concentrated under reduced pressure to remove solvent to obtain Compound 3-c (90 mg, the crude product was directly used in the next step). The obtained compound was characterized: MS (ESI) m/z = 345.3 (M+1)⁺

### Synthesis of Compound 3-d

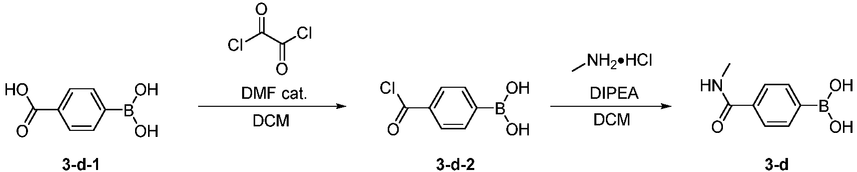

### Step 1, Synthesis of Compound 3-d-2

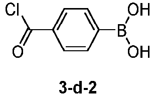

Compound **3-d-1** (252 mg, 1.52 mmol), DCM (20 mL) and 3 drops of DMF (i.e., catalytic amount of DMF) were added to a 100 mL flask. The mixture was cooled to 0 °C, and oxalyl chloride (964.59 mg, 7.6 mmol) was added. The reaction system was slowly heated to room temperature. After reacting at room temperature for 1 hour, the solvent was spin-dried to obtain the crude product **3-d-2** (300 mg) which was directly used in the next reaction.

### Step 2, Synthesis of Compound 3-d

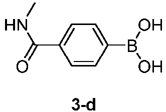

DCM (10 mL), DIPEA (500 mg, 4.56 mmol), methylamine hydrochloride (103 mg, 1.52 mmol) were added to the crude product **3-d-2** obtained in the previous step. The reaction was stopped after reacting at room temperature for 1 hour (monitored by LC-MS). Saturated NaCl solution (20 mL) and ethyl acetate (3×20 mL) were used to complete extraction, and the organic phases were combined and dried over anhydrous sodium sulfate, evaporated to remove solvent to obtain Compound **3-d** (223 mg, 82% yield). The obtained compound was characterized: MS (ESI) m/z = 179.1 (M+1)⁺

### Example 11, Synthesis of Compound 1A of Series A of the present invention

According to the synthesis scheme of Compound **7-5** in the synthesis scheme of Compound **1A** (i.e., Steps **1** to **4**), Compounds **7A**, **8A**, **9A** and **10A** could be obtained by replacing the raw material **2-a** used in the reaction with the raw material as listed in Table 14.

**Table 14. Compounds 7A to 10A**

| Raw material | Product structure | H-NMR data characterization |
|---|---|---|
| 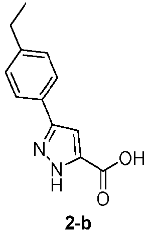 | 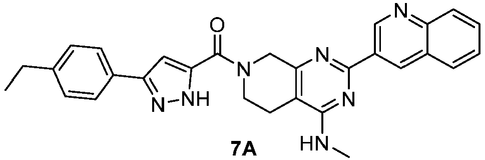 (LC-MS: C₂₉H₂₇N₇O, [M+H]⁺ 490.2; detected value: 490.3; purity: 99.9%) | ¹H NMR (400 MHz, Methanol-*d*₄) δ 9.77-9.76 (m, 1H), 9.50-9.49 (m, 1H), 8.27-8.20 (m, 2H), 8.04 (t, *J* = 7.9 Hz, 1H), 7.86 (s, 1H), 7.64 (d, *J* = 8.4 Hz, 2H), 7.30 (t, *J* = 7.2 Hz, 2H), 6.99 (s, 1H), 5.31 (s, 1H), 4.97 (s, 1H), 4.38 (s, 1H), 4.14 (s, 1H), 3.26 (s, 3H), 2.74-2.66 (m, 4H), 1.33-1.24 (m, 3H). |
| 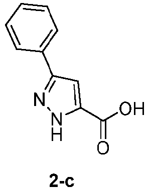 | 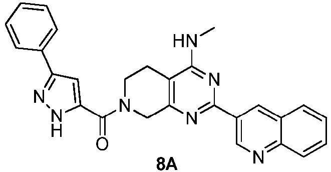 (LC-MS: C₂₇H₂₃N₇O, [M+H]⁺ 462.2; detected value: 462.3; purity: 98.1%) | ¹H NMR (400 MHz, Methanol-*d*₄) δ 9.74-9.72 (m, 1H), 9.45-9.43 (m, 1H), 8.24-8.18 (m, 2H), 8.04 (t, *J* = 7.9 Hz, 1H), 7.84 (s, 1H), 7.75 (d, *J* = 8.4 Hz, 2H), 7.46 (t, *J* = 7.2 Hz, 2H), 7.39 (t, *J* = 7.2 Hz, 1H), 7.03 (s, 1H), 5.28 (s, 1H), 4.93 (s, 1H), 4.37 (s, 1H), 4.13 (s, 1H), 3.25 (s, 3H), 2.73-2.67 (m, 2H). |
| 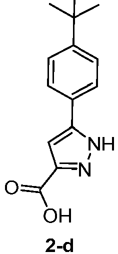 | 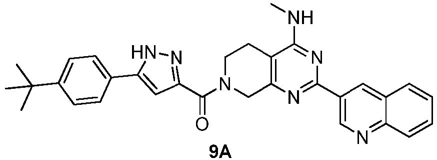 (LC-MS: C₃₁H₃₁N₇O, [M+H]⁺ 518.3; detected value: 518.5; purity: 99.4%) | ¹H NMR (400 MHz, Methanol-*d*₄) δ 9.77-9.74 (m, 1H), 9.44-9.42 (m, 1H), 8.25-8.18 (m, 2H), 8.02 (s,1H), 7.84 (s, 1H), 7.67 (d, *J =* 8.4 Hz, 2H), 7.52 (t, *J =* 7.2 Hz, 2H), 6.99 (s, 1H), 5.28 (s, 1H), 4.95 (s, 1H), 4.38 (s, 1H), 4.14 (s, 1H), 3.25 (s, 3H), 2.73-2.68 (m, 2H), 1.35 (s, 9H). |
| 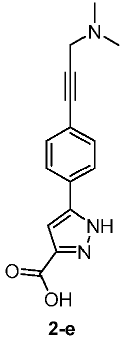 | 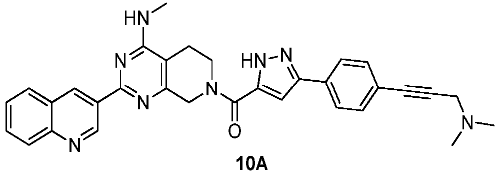 (LC-MS: C₃₂H₃₀N₈O, [M+H]⁺ 543.3; detected value: 543.4; purity: 97.6%) | ¹H NMR (400 MHz, Methanol-*d*₄) δ 9.76 (s, 1H), 9.48 (s, 1H), 8.25-8.19 (m, 2H), 8.03 (s, 1H), 7.82 (d, *J* = 8.1 Hz, 3H), 7.63 (d, *J =* 8.0 Hz, 2H), 7.13 (s, 1H), 5.26 (s, 1H), 4.94 (s, 1H), 4.37 (s, 1H), 4.34 (s, 2H), 4.14 (s, 1H), 3.25 (s, 3H), 3.03 (s, 6H), 2.74-2.70 (m, 2H). |

### Synthesis of Compound 2-d

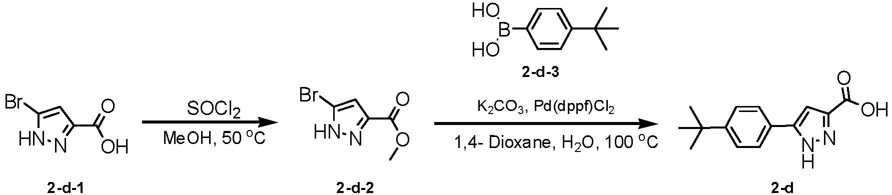

### Step 1. Synthesis of Compound 2-d-2

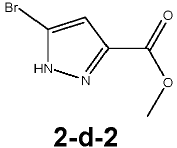

Synthesis step: Sulfoxide chloride (622.94 mg, 5.24 mmol, 379.84 uL) was added dropwise to Compound **2-d-1** (500 mg, 2.62 mmol) dissolved in 10 mL of anhydrous methanol at 0°C. The reaction system was heated to 50°C and stirred for 1.5 hours. LC-MS showed that the reaction was complete. After the reaction system was cooled to room temperature, it was cooled in an ice bath, and then water was added to quench the reaction. The reaction mixture was filtered, the resulting filtrate was concentrated and evaporated to dryness, and purified by MPLC to obtain Compound **2-d-2.** The obtained compound was characterized: MS (ESI) m/z =206.0 (M+1)⁺

### Step 2, Synthesis of Compound 2-d

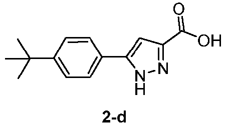

Under nitrogen protection, Compound **2-d-2** (50 mg, 243.89 µmol), Compound **2-d-3** (52.11 mg, 292.67 µmol), K₂CO₃ (100.97 mg, 731.67 µmol), 1,4-dioxane (5 mL) and Pd(dppf)Cl₂ (8.92 mg, 12.19 µmol) were added to a 25 mL reaction bottle in sequence. The reaction system was sealed, stirred and reacted at 100°C overnight, and then the reaction was quenched (monitored by LC-MS). Extraction was completed with saturated NaCl solution (20.0 mL) and ethyl acetate (3×20 mL), and the organic phases were combined and dried over anhydrous sodium sulfate, evaporated to remove solvent, separated by MPLC, and concentrated under reduced pressure to remove solvent to obtain Compound **2-d** (20 mg, 81.87 µmol, 33.57% yield). The obtained compound was characterized: MS (ESI) m/z =245.1 (M+1)⁺

### Synthesis of Compound 2-e

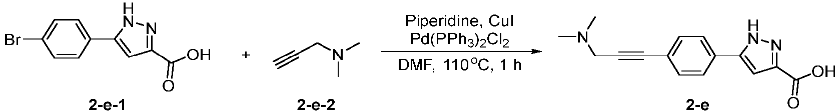

Under nitrogen protection, Compound **2-e-1** (265 mg, 992.22 µmol), Compound **2-e-2** (123.73 mg, 1.49 mmol), piperidine (168.97 mg, 1.98 mmol), DMF (4 mL), CuI (1.78 mg, 9.36 µmol) and Pd(dppf)Cl₂ (69.55 mg, 99.22 µmol) were added to a 50 mL microwave reaction tube in sequence. The reaction system was sealed, heated and stirred and reacted at 110 °C for 1 hour, and then the reaction was stopped (monitored by LC-MS). The solvent was evaporated and the product **2-e** (20 mg, 74.27 µmol, 7.48% yield) was obtained by separation and purification by MPLC. The obtained compound was characterized: MS (ESI) m/z = 270.1 (M+1)⁺

### Example 12, Synthesis of Compound 1B of Series B of the present invention:

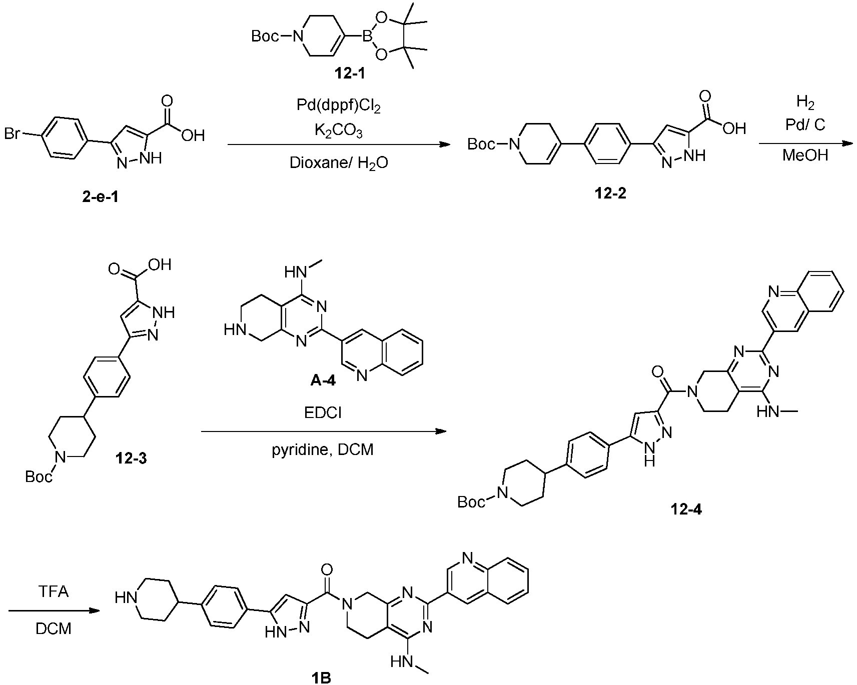

### Step 1, Synthesis of Compound 12-2

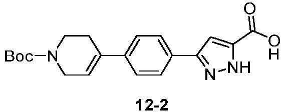

Under nitrogen protection, Compound **2-e-1** (114 mg, 427 µmol), Compound **12-1** (145 mg, 470 µmol), K₂CO₃ (138 mg, 177 µmol), 1,4-dioxane (5 mL) and Pd(dppf)Cl₂ (18 mg, 24.2 µmol) were added to a 25 mL reaction bottle in sequence. The reaction system was sealed, and the reaction was carried out under stirring at 80°C overnight and then quenched (monitored by LC-MS). Saturated NaCl solution (20.0 mL) and ethyl acetate (3×20 mL) were used to complete extraction. The organic phases were combined and dried over anhydrous sodium sulfate, evaporated to remove solvent, separated by MPLC, and concentrated under reduced pressure to remove solvent to obtain Compound **12-2** (100 mg, 270 µmol, 63.42% yield). The obtained compound was characterized: MS (ESI) m/z =370.2 (M+1)⁺

### Step 2, Synthesis of Compound 12-3

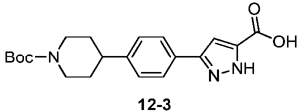

Compound **12-2** (100 mg, 270 µmol), methanol (5.0 mL), and palladium-on-carbon (10 mg) were added to a 50 mL reaction bottle in sequence. The reaction system was replaced with hydrogen, and the hydrogen atmosphere was maintained at one atmospheric pressure. The reaction was carried out under stirring at room temperature for 3 hours (monitored by LC-MS). The palladium-on-carbon was removed by filtration, washed with methanol (2×20 mL), the filtrate was collected, and concentrated under reduced pressure to remove solvent to obtain Compound **12-3** (100 mg, crude product), LCMS (ESI) [M+H]: 372.2.

### Step 3, Synthesis of Compound 12-4

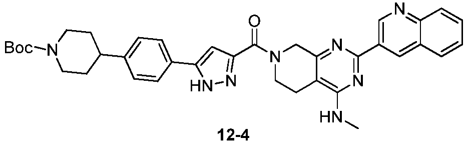

DCM (2 mL), Compound **A-4** (25 mg, 130 µmol), EDCI (30 mg, 156 µmol) and pyridine (21 mg, 260 µmol, 25 µL) were added to a 10 mL reaction bottle in sequence. After the temperature of the reaction system dropped to 0°C, Compound **12-3** (50 mg, 130 µmol) was added. The reaction was carried out under stirring and ice bath conditions for 0.5 hours, and then quenched. After purification by MPLC, the solvent was removed by concentration under reduced pressure to obtain Compound **12-4** (32 mg, 496 µmol, 38.14% yield). The obtained compound was characterized: MS (ESI) m/z = 645.3 (M+1)⁺.

### Step 4, Synthesis of Compound 1B

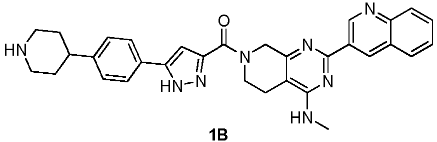

Compound **12-4** (32 mg, 496 µmol) was dissolved in dichloromethane (4.0 mL) in a 25 mL reaction bottle, and then TFA (1.5 mL) was added dropwise. The reaction was carried out under stirring at room temperature for 0.5 hours, and the solvent was removed by concentration under reduced pressure to obtain Compound **1B** (24.5 mg, 450 µmol, 90.73% yield, 99.9% purity). The obtained compound was characterized: MS (ESI) m/z = 545.3 (M+1)⁺. ¹H NMR (400 MHz, Methanol-d4) δ 9.80 (s, 1H), 9.51-9.47 (m, 1H), 8.28-8.21 (m, 2H), 8.05-7.72 (m, 5H), 7.39 (d, *J* = 9.6 Hz, 3H), 5.27 (s, 1H), 4.95 (s, 1H), 4.38-4.14 (m, 2H), 3.54 (s, 1H), 3.51 (s, 1H), 3.16 (s, 2H), 2.98 (s, 2H), 2.67 (s, 1H), 2.13-2.10 (m, 2H), 1.98-1.92 (m, 2H).

According to the synthetic scheme of Compound **7-5**, Compounds **2B** to **3B** could be obtained by replacing Compound **2-a** with the raw material as listed in Table 15.

**Table 15. Compounds 2B to 3B**

| Raw material | Product structure | H-NMR data characterization |
|---|---|---|
| 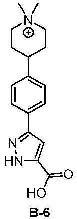 | 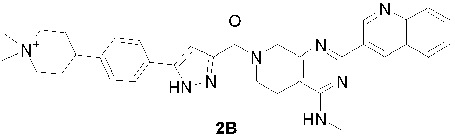 (LC-MS: C₃₄H₃₇N₈O, [M]⁺ 573.3; detected value: 573.5 purity: 87.6 %) | ¹H NMR (400 MHz, Methanol-*d*₄) δ 9.80 (s, 1H), 9.51-9.47 (m, 1H), 8.28-8.21 (m, 2H), 8.05-7.72 (m, 5H), 7.39 (d, *J =* 9.6 Hz, 3H), 5.27 (s, 1H), 4.95 (s, 1H), 4.38-4.14 (m, 2H), 3.54 (s, 1H), 3.51 (s, 1H), 3.16 (s, 2H), 2.98 (s, 2H), 2.67 (s, 1H), 2.13-2.10 (m, 2H), 1.98-1.92 (m, 2H). |
| 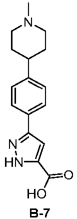 | 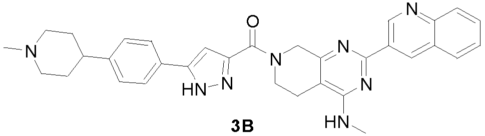 (LC-MS: C₃₃H₃₅N₈O, [M+H]⁺ 559.3; detected value: 559.6; purity: 96.1 %) | ¹H NMR (400 MHz, Methanol-*d*₄) δ 9.80 (s, 1H), 9.51-9.47 (m, 1H), 8.28-8.21 (m, 2H), 8.05-7.72 (m, 5H), 7.39 (d, *J =* 9.6 Hz, 3H), 5.27 (s, 1H), 4.95 (s, 1H), 4.38-4.14 (m, 2H), 3.54 (s, 1H), 3.51 (s, 1H), 3.16 (s, 2H), 2.98 (s, 2H), 2.67 (s, 1H), 2.13-2.10 (m, 2H), 1.98-1.92 (m, 2H). |

### Example 13. Synthesis of Compound 1C of Series C of the present invention

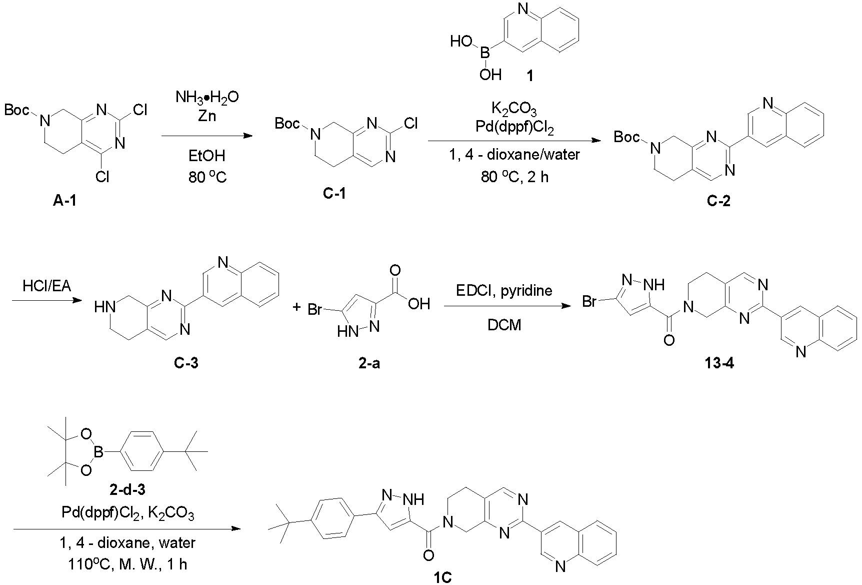

### Step 1. Synthesis of Compound C-1

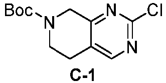

Compound **A-1** (400 mg, 1.32 mmol), concentrated aqueous ammonia (447 mg, 6.58 mmol), zinc powder (673.30 mg, 10.52 mmol) and anhydrous ethanol (5 mL) were added to a 50 mL flask. The reaction was carried out under stirring at 80 °C for 1 hour, and then stopped (monitored by LC-MS). The reaction mixture was filtered, the filtrate was collected and the solvent was evaporated to obtain the crude Compound **C-1** (355 mg). The obtained compound was characterized: MS (ESI) m/z = 270.1 (M+1)⁺

### Step 2, Synthesis of Compound C-2

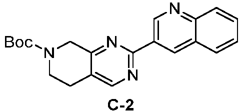

Under nitrogen protection, Compound **C-1** (355 mg, 1.32 mmol), Compound 1 (273.19 mg, 1.58 mmol), K₂CO₃ (544.88 mg, 3.95 mmol), 1,4-dioxane and water (4 mL/1 mL) and Pd(dppf)Cl₂ (10 mg, 1.32 mmol) were added to a 50 mL reaction bottle in sequence. The reaction system was sealed, and the reaction was carried out under stirring at 80°C for 2 hours and then stopped (monitored by LC-MS). Diatomaceous earth was used for filtration, the filtrate was concentrated, evaporated to remove solvent, separated and purified by preparative HPLC, and concentrated under reduced pressure to remove solvent to obtain Compound **C-2** (200 mg, 551.84 µmol, 41.93% yield). The obtained compound was characterized: MS (ESI) m/z = 363.2 (M+1)⁺

### Step 3, Synthesis of Compound C-3

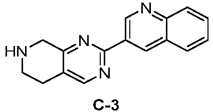

Compound **C-2** (150 mg, 413.88 µmol) was dissolved in a mixed solution of 6 N HCl and ethyl acetate (2 mL) in a 50 mL reaction bottle, stirred at room temperature for 0.5 hours, and concentrated under reduced pressure to remove solvent to obtain Compound **C-3** (109 mg, crude product). The obtained compound was characterized: MS (ESI) m/z = 263.1 (M+1)⁺

### Step 4, Synthesis of Compound 13-4

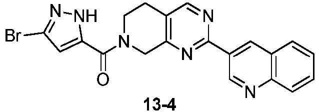

DCM (2 mL), Compound **2-a** (132 mg, 691.16 µmol), EDCI (158.41 mg, 829.39 µmol) and pyridine (109.34 mg, 1.38 mmol, 111.36 µL) were added to a 10 mL reaction bottle in sequence. After the temperature of the reaction system dropped to 0°C, Compound **C-3** (181.30 mg, 691.16 µmol) was added. The reaction was carried out under stirring and ice bath conditions for 0.5 hours and then quenched. After purification by MPLC and concentration under reduced pressure to remove solvent, Compound **13-4** (150 mg, 344.61 µmol, 49.86% yield) was obtained. The obtained compound was characterized: MS (ESI) m/z = 435.1 (M+1)⁺

### Step 5, Synthesis of Compound 1C

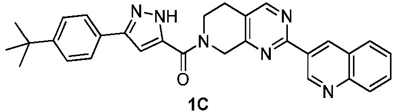

Under nitrogen protection, Compound **2-d-3** (22.50 mg, 126.36 µmol), Compound **13-4** (50 mg, 114.87 µmol), K₂CO₃ (47.56 mg, 344.61 µmol), 1,4-dioxane and water (4 mL/1 mL) and Pd(dppf)Cl₂ (5 mg, 114.87 µmol) were added to a 50 mL reaction bottle in sequence. The reaction system was sealed, and the reaction was carried out under microwaves at 110 °C for 2 hours, and then quenched (monitored by LC-MS). Saturated NaCl solution (20.0 mL) and ethyl acetate (3×10 mL) were used to complete extraction, and the organic phases were combined and dried over anhydrous sodium sulfate, evaporated to remove solvent, separated by MPLC, and concentrated under reduced pressure to remove solvent to obtain Compound **1C** (13.9 mg, 26.03 µmol, 22.66% yield, 91.5% purity). The obtained compound was characterized: MS (ESI) m/z = 489.2 (M+1)⁺. ¹H NMR (400 MHz, DMSO-*d₆*) δ 13.73 (s, 1H), 9.84-9.79 (m, 1H), 9.30-9.25 (m, 1H), 8.84 (s, 1H), 8.22 (d, *J =* 4.0 Hz, 1H), 8.10 (s, 1H), 7.84 (s, 1H), 7.73 (t, *J =* 7.6 Hz, 2H), 7.63 (t, *J* = 8.8 Hz, 2H), 7.06 (s, 1H), 5.46 (s, 1H), 4.98 (s, 1H), 4.35 (s, 1H), 4.01 (s, 1H), 3.05-2 .98 (m, 2H), 1.34 (t, *J =* 5.6 Hz, 9H).

### Example 14, Synthesis of Series C Compound 2C of the present invention:

According to the synthesis scheme of Compound **1C** above, Compound **2C** could be obtained by replacing Compound **2-a** with the raw material as listed in Table 16.

**Table 16. Compound 2C**

| Raw material | Product structure | H-NMR data characterization |
|---|---|---|
| 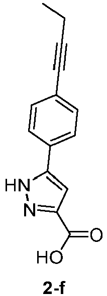 | 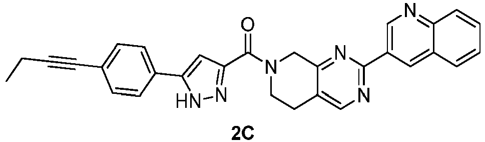 (LC-MS: C₃₀H₂₅N₆O, [M+H]⁺ 485.2; detected value: 485.3; purity: 98.2%) | ¹H NMR (400 MHz, DMSO-*d₆*) δ 13.83(s, 1H), 9.84-9.80 (m, 1H), 9.32-9.27 (m, 1H), 8.85 (s, 1H), 8.23 (d, *J* = 7.6 Hz, 1H), 8.10 (s, 1H), 7.95-7.81 (m, 3H), 7.69 (t, *J* = 3.6 Hz, 1H), 7.52-7.47 (m, 2H), 6.96 (s, 1H), 5.36 (s, 1H), 4.98 (s, 1H), 4.25-4.11 (m, 2H), 3.06-2.98 (m, 2H), 2.46-2.42 (m, 2H), 1.18 (s, 3H). |

### Synthesis of Compound 2-f

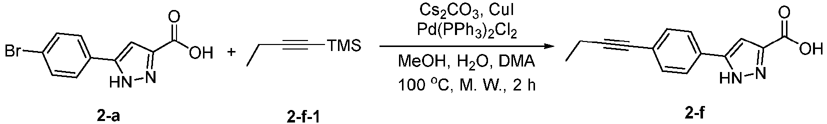

Under nitrogen protection, Compound **2-a** (50 mg, 261.80 µmol), Compound **2-f-1** (33.06 mg, 261.80 µmol), Cs₂CO₃ (170.17 mg, 523.61 µmol), MeOH (0.2 mL), H₂O (2 mL), DMA (0.2 mL), CuI (2.49 mg, 13.09 µmol) and Pd(dppf)Cl₂ (9.19 mg, 13.09 µmol) were added to a 5 mL microwave reaction tube in sequence. The reaction system was sealed, and the reaction was carried out under stirring and microwave heating at 100 °C for 2 hours, and then stopped (monitored by LC-MS). The organic phase was separated and concentrated, separated and purified by preparative HPLC to obtain the product 2-f (10 mg, 60.92 µmol, 23.27% yield). The obtained compound was characterized: MS (ESI) m/z = 241.1 (M+1)

### Example 15, Synthesis of Compound 3C of Series C of the present invention:

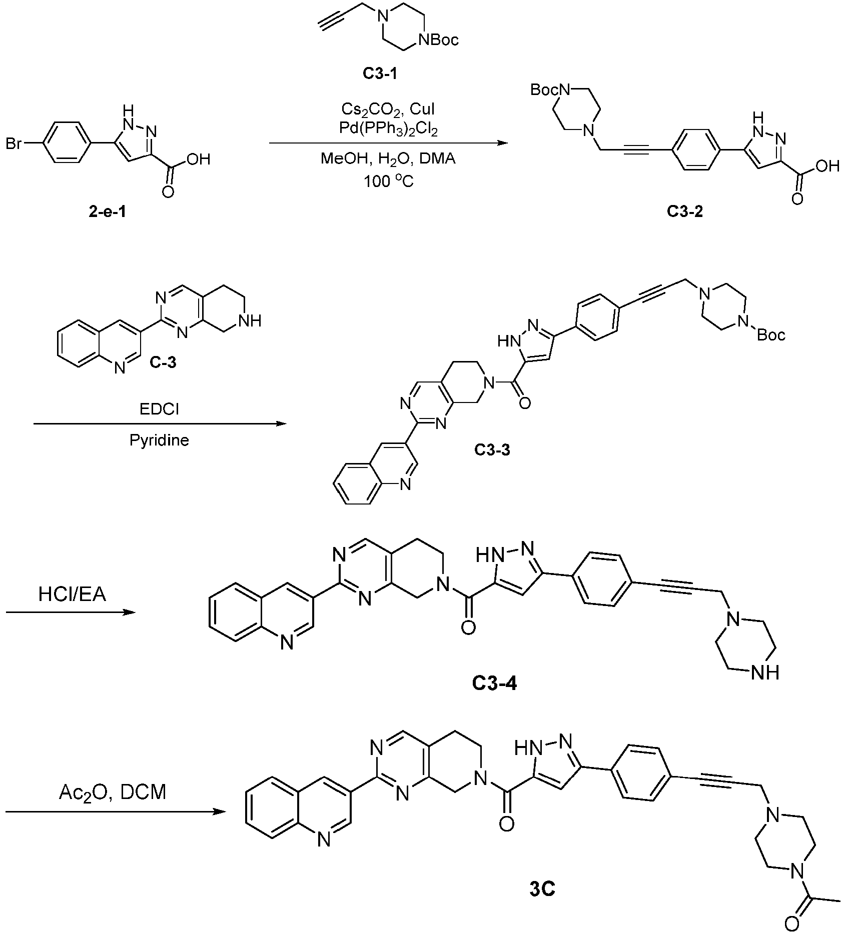

### Step 1, Synthesis of Compound C3-2

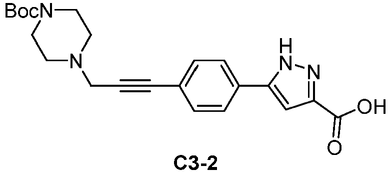

Under nitrogen protection, Compound **2-e-1** (50 mg, 187.21 µmol), Compound **C3-1** (41.99 mg, 187.21 µmol), Cs₂CO₃ (121.69 mg, 374.42 µmol), MeOH (0.2 mL), H₂O (2 mL), DMA (0.2 mL), CuI (1.78 mg, 9.36 µmol) and Pd(dppf)Cl₂ (6.57 mg, 9.36 µmol). The reaction system was sealed, and the reaction was carried out under stirring and microwave heating at 100 °C for 2 hours, and then stopped (monitored by LC-MS). The organic phase was separated and concentrated and separated and purified by preparative HPLC to obtain the product **C3-2** (7 mg, 17.05 µmol, 9.11% yield). The obtained compound was characterized: MS (ESI) m/z = 411.2 (M+1)

### Step 2, Synthesis of Compound C3-3

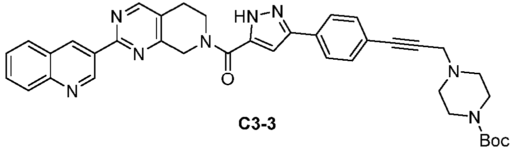

Compound **C3-2** (7 mg, 17.05 µmol), EDCI (3.91 mg, 20.46 µmol), DCM (1 mL), pyridine (2.70 mg, 34.11 µmol, 2.75 uL) were added to a 10 mL reaction bottle in sequence. After the temperature of the reaction system dropped to 0°C, Compound **C-3** (4.47 mg, 17.05 µmol) was added. The temperature was slowly raised to room temperature and stirring was carried out for one hour, and then the reaction was stopped (monitored by LC-MS). Saturated NaCl solution (5 mL) and dichloromethane (3×5 mL) were used to complete extraction, and the organic phases were combined and dried over anhydrous sodium sulfate, evaporated to remove solvent, purified by preparative HPLC, concentrated under reduced pressure to remove solvent to obtain Compound **C3-3** (6.00 mg, 9.16 µmol, 53.73% yield). The obtained compound was characterized: MS (ESI) m/z = 655.3 (M+1)

### Step 3, Synthesis of Compound C3-4

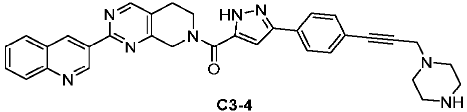

2mL of 6N HCl ethyl acetate solution was added to Compound **C3-3** and stirred at room temperature for 30 min to complete the reaction (detected by LC-MS). The solvent was evaporated and the obtained Compound **C3-4** (6.00 mg, crude product) was directly used for the next reaction. The obtained compound was characterized: MS (ESI) m/z = 555.3 (M+1)⁺

### Step 4, Synthesis of Compound 3C

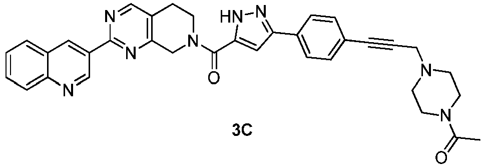

Compound **C3-4** (6 mg, crude product), DCM (1 mL) and acetic anhydride (1.21 mg, 11.90 µmol) were added to a 10 mL reaction bottle in sequence, stirred at room temperature for 0.5 h, and then the reaction was stopped (monitored by LC-MS). Evaporation was carried out to remove solvent. After purification by preparative HPLC, and concentration under reduced pressure to remove the solvent to obtain Compound **3C** (2.00 mg, 3.14 µmol, 29.00% yield, 93.6% purity). The prepared compound was characterized: MS (ESI) m/z =597.3 (M+1). ¹H NMR (400 MHz, DMSO-*d₆*) δ 13.89(s, 1H), 9.84-9.79 (m, 1H), 9.30-9.26 (m, 1H), 8.85 (s, 1H), 8.22 (d, *J =* 7.6 Hz, 1H), 8.10 (s, 1H), 7.98-7.84 (m, 3H), 7.70 (t, *J =* 3.6 Hz, 1H), 7.55-7.53 (m, 2H), 7.17 (s, 1H), 5.42 (s, 1H), 4.98 (s, 1H), 4.31 (s, 1H), 4.14 (t, *J =* 3.2 Hz, 1H), 4.03 (s, 2H), 3.95 (s, 2H), 3.05 (s, 2H), 2.98 (s, 2H), 2.88-2.76 (m, 4H), 2.00 (s, 3H).

### Example 16, Synthesis of Compound 1D of Series D of the present invention:

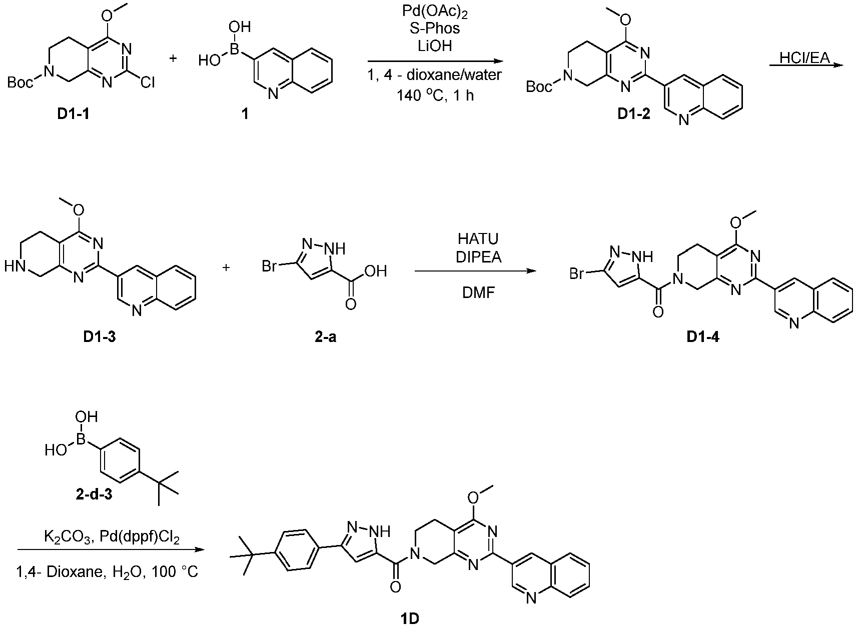

### Step 1, Synthesis of Compound D1-2

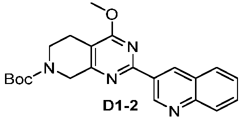

Under nitrogen protection, Compound **D1-1** (590 mg, 1.97 mmol), Compound **1** (340.47 mg, 1.97 mmol), LiOH (94.27 mg, 3.94 mmol), 1,4-dioxane and water (12 mL/3 mL), S-Phos (80.70 mg, 196.83 µmol) and Pd(OAc)₂ (44.09 mg, 196.83 µmol). After stirring at 140 °C for 2 hours, the reaction was quenched (monitored by LC-MS). Saturated NaCl solution (20.0 mL) and ethyl acetate (3×20 mL) were used to complete extraction, and the organic phases were combined, dried over anhydrous sodium sulfate, evaporated to remove solvent. After purification by MPLC, the crude product Compound **D1-2** (600.00 mg) was obtained after the solvent was removed by concentration under reduced pressure. The obtained compound was characterized: MS (ESI) m/z = 393.2 (M+1).

### Step 2, Synthesis of Compound D1-3

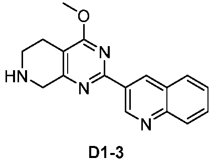

Compound **D1-2** (600.00 mg, crude product, 1.53 mmol) was dissolved in a mixed solution of 6 N HCl and ethyl acetate (10 mL) in a 50 mL reaction bottle, stirred at room temperature for 1 hour, and concentrated under reduced pressure to remove the solvent to obtain the crude product compound **D1-3**, which was directly used in the next step. The obtained compound was characterized: MS (ESI) m/z = 293.2 (M+1)⁺.

### Step 3, Synthesis of Compound D1-4

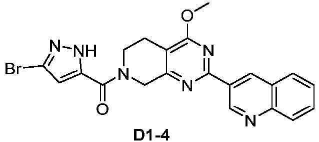

Compound **2-a** (287.45 mg, 1.51 mmol), DIPEA (583.58 mg, 4.52 mmol, 786.50 µL), HATU (629.14 mg, 1.66 mmol) and DMF (10 mL) were added to a 50 mL reaction bottle in sequence. After the temperature of the reaction system dropped to 0°C, Compound **D1-3** (440.00 mg, 1.51 mmol) was added. After stirring for 0.5 hours under ice bath conditions, the reaction was quenched (monitored by LC-MS). Saturated NaCl solution (20 mL) and ethyl acetate (3×20 mL) were used for extraction. The organic phases were combined, dried over anhydrous sodium sulfate, evaporated to remove solvent, purified by MPLC, and concentrated under reduced pressure to remove solvent to obtain Compound **D1-4** (400.00 mg, crude product). The obtained compound was characterized: MS (ESI) m/z = 465.1 (M+1)⁺.

### Step 4, Synthesis of Compound 1D

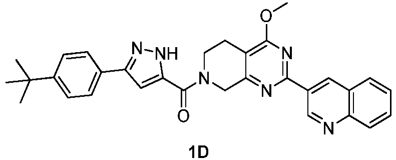

Under nitrogen protection, Compound **D1-4** (130 mg, 279.39 µmol), Compound **2-d-3** (49.74 mg, 279.39 µmol), K₂CO₃ (96.39 mg, 698.47 µmol), 1,4-dioxane and water (4 mL/1 mL) and Pd(dppf)Cl₂ (20.45 mg, 27.94 µmol) were added to a 50 mL reaction bottle in sequence. The reaction system was sealed and stirred at 100°C for 2 hours, then the reaction was quenched (monitored by LC-MS). Saturated NaCl solution (20.0 mL) and ethyl acetate (3×20 mL) were used to complete extraction, and the organic phases were combined and dried over anhydrous sodium sulfate, evaporated to remove solvent, purified by MPLC, and concentrated under reduced pressure to remove solvent to obtain Compound **1D** (30.00 mg, 57.38 µmol, 20.54% yield, 99.2% purity). The obtained compound was characterized: MS (ESI) m/z = 519.3 (M+1)⁺. ¹H NMR (400 MHz, DMSO-*d₆*) δ 13.73 (s, 1H), 9.81 (s, 1H), 9.25 (s, 1H), 8.19 - 8.09 (m, 2H), 7.84-7.72 (m, 4H), 7.49 (s, 2H), 7.03 (s, 1H), 5.34 (s, 2H), 4.88 (s, 2H),4.26-4.16 (m, 5H), 1.30 (s, 9H).

### Example 17, Synthesis of Compound 1E of Series E of the present invention:

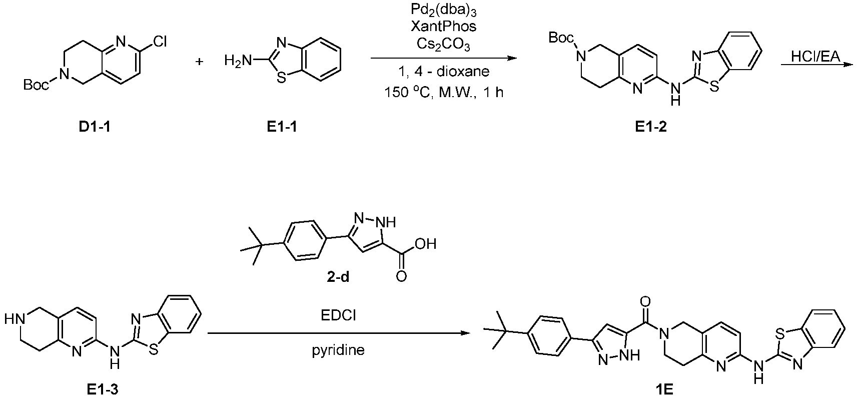

### Step 1, Synthesis of Compound E1-2

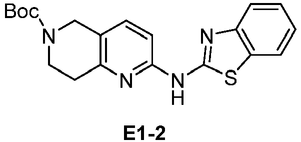

Under nitrogen protection, Compound **D1-1** (268.00 mg, 997.25 µmol), Compound **E1-1** (224.68 mg, 1.50 mmol), Cs₂CO₃ (975.31 mg, 2.99 mmol), 1,4-dioxane (5 mL), XantPhos (288.50 mg, 498.63 µmol) and Pd₂(dba)₃ (182.64 mg, 199.45 µmol) were added to a dry microwave reaction tube in sequence. The reaction system was sealed, heated with microwave and reacted at 150 °C for 1 hour, then the reaction was quenched. Saturated NaCl solution (20.0 mL) and ethyl acetate (3×20 mL) were used to complete extraction. The organic phases were combined and dried over anhydrous sodium sulfate, evaporated to remove solvent, purified by MPLC, and concentrated under reduced pressure to remove solvent to obtain Compound **E1-2** (300.00 mg, 784.36 µmol, 78.65% yield). The obtained compound was characterized: MS (ESI) m/z = 414.2 (M+1)⁺

### Step 2, Synthesis of Compound E1-3

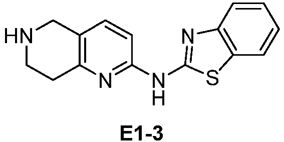

Compound **E1-2** (300 mg, 784.36 µmol) was dissolved in a mixed solution of 6 N HCl and ethyl acetate (10 mL) in a 50 mL reaction bottle, stirred and reacted at room temperature for 1 hour, and the crude product Compound **E1-3** was directly used in the next step after concentration under reduced pressure to remove solvent.

### Step 3, Synthesis of Compound E1

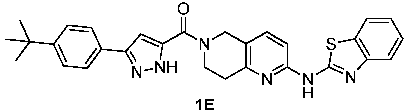

Compound **2-d** (20 mg, 81.87 µmol), EDCI (47.08 mg, 245.61 µmol) and pyridine (2 mL) were added to a 50 mL reaction bottle in sequence. After the temperature of the reaction system dropped to 0°C, Compound **E1-3** (23.12 mg, 81.87 µmol) was added. After the reaction was carried out under stirring at room temperature for 0.5 hours, 1 mL of water was added to quench the reaction. After purification by MPLC and concentration under reduced pressure to remove the solvent, Compound 1E (9.10 mg, 17.89 µmol, 21.85% yield, 99.1% purity) was obtained. The obtained compound was characterized: MS (ESI) m/z = 509.4 (M+1)⁺. ¹H NMR (400 MHz, DMSO-*d₆*) δ 11.53 (s, 1H), 7.90 (d, *J =* 7.7 Hz, 1H), 7.75-7.60 (m, 4H), 7.49 (d, *J =* 8.0 Hz, 2H), 7.36 (t, *J* = 7.6 Hz, 1H), 7.20 (dd, *J* = 7.5, 5.9 Hz, 1H), 7.05 (s, 2H), 5.11-4.77 (m, 2H), 4.23-4.01 (m, 2H), 3.09 - 3.02 (m, 3H), 1.31 (s, 9H).

### Example 18, Synthesis of Compound 1F of the present invention:

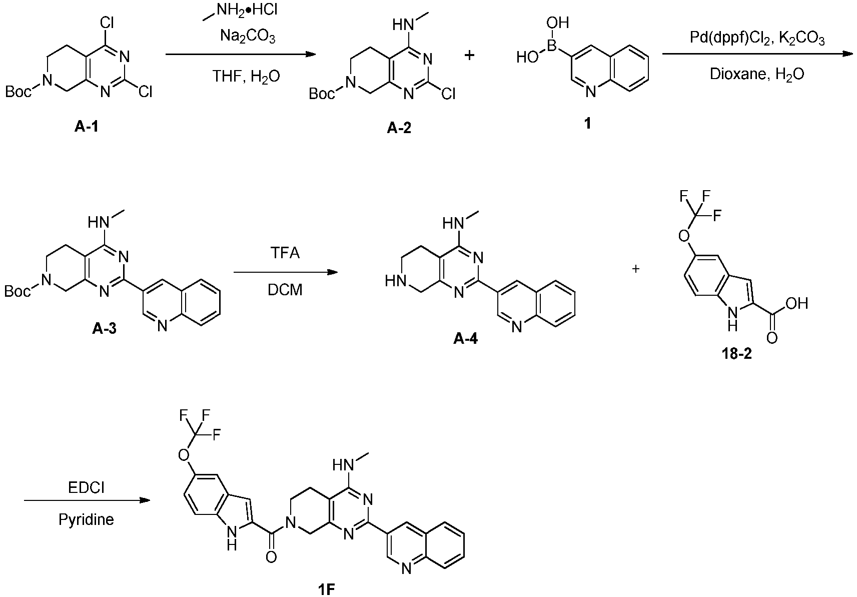

### Step 1, Synthesis of Compound A-2

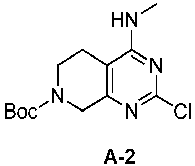

Compound **A-1** (304.00 mg, 999.44 µmol), methylamine hydrochloride (80.98 mg, 1.20 mmol), sodium carbonate (317.82 mg, 3.00 mmol) and tetrahydrofuran/water (4 mL/2 mL) were added to a 50 mL reaction bottle, stirred at room temperature until the reaction was completed, and then water was added to quench the reaction (monitored by LC-MS). Saturated sodium chloride solution (10 mL) and ethyl acetate (3 X 15 mL) were used for extraction, the organic phases were combined, dried over anhydrous sodium sulfate, evaporated to remove solvent, purified by MPLC, and concentrated under reduced pressure to remove solvent to obtain Compound **A-2** (132.00 mg, 441.81 µmol, 44.21% yield), LCMS: C₁₃H₂₀ClN₄O₂, (ESI) [M+H]: 299.1.

### Step 2, Synthesis of Compound A-3

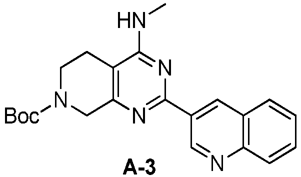

Under nitrogen protection, Compound **A-2** (132.00 mg, 441.81 µmol), Compound 1 (91.18 mg, 530.18 µmol), potassium carbonate (182.91 mg, 1.33 mmol), 1,4-dioxane and water (4 mL/1 mL) and Pd(dppf)Cl₂ (6.44 mg, 0.25 mol%) were added to a 50 mL reaction bottle in sequence. The reaction system was sealed, stirred and reacted at 80 °C for 2 hours, then the reaction was quenched (monitored by LC-MS). Saturated sodium chloride solution (20 mL) and ethyl acetate (3 × 20 mL) were used to complete extraction. The organic phases were combined and dried over anhydrous sodium sulfate, evaporated to remove solvent, separated by column chromatography with an eluent having a volume ratio of petroleum ether/ethyl acetate = 1:100~1:5, and concentrated under reduced pressure to remove solvent to obtain Compound **A-3** (96.00 mg, 245.85 µmol, 55.65% yield). LCMS: C₂₂H₂₆N₅O₂, (ESI) [M+H]: 392.2.

### Step 3, Synthesis of Compound A-4

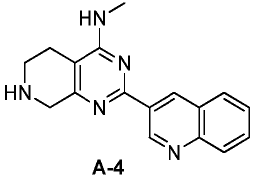

Compound **A-3** (96.00 mg, 245.85 µmol) was dissolved in dichloromethane (2 mL) in a 50 mL reaction bottle, and trifluoroacetic acid (1 mL) was then added dropwise. The reaction was carried out under stirring at room temperature for 3 hours, and the solvent was removed by concentration under reduced pressure to obtain Compound **A-4** (crude product), LCMS: C₁₇H₁₈N₅, (ESI) [M+H]: 292.2.

### Step 4, Synthesis of Compound 1F

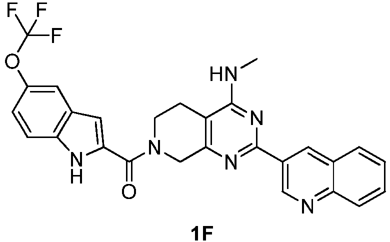

Compound **18-2** (10 mg, 40.79 µmol), EDCI (7.79 mg, 40.79 µmol) and pyridine (0.5 mL) were added to a 3 mL reaction bottle in sequence. After the temperature of the reaction system dropped to 0°C, Compound A-4 (12 mg, 41.19 umol) was added. The reaction was carried out under stirring and ice bath conditions for 0.5 hours, and then quenched. After purification by MPLC and concentration under reduced pressure to remove solvent, Compound 1F (10 mg, 19.29 µmol, 47.28% yield) was obtained. LC-MS: C₃₁H₂₈N₅O₂, [M+H]⁺ 519.2; detected value: 519.3. ¹H NMR (400 MHz, DMSO-*d₆*) δ 11.99 (s, 1H), 9.74 (s, 1H), 9.26 (s, 1H), 8.21 (d, *J* = 4.0 Hz, 1H), 8.11 (d, *J* = 4.0 Hz, 1H), 7.88 (t, *J* = 7.6 Hz, 1H), 7.71 (t, *J* = 7.6 Hz, 2H), 7.52 (t, *J* = 8.8 Hz, 2H), 7.20 (d, *J* = 8.4 Hz, 1H), 7.05 (s, 1H), 4.91 - 4.83 (m, 2H), 4.38 (s, 2H), 3.10 (d, *J* = 4.0 Hz, 3H), 2.65 (s, 2H).

The following test examples demonstrated the beneficial effects of the present invention.

### Experimental Example 1. BCL-XL surface plasmon resonance (SPR) binding experiment

### 1. Experimental method

Preparation of running buffer: The compositions of protein fixation buffer and running buffer **A** were the same, in which the concentration of NaH₂PO₄ was 10 mM, the concentration of Na₂HPO₄ was 40 mM, the concentration of NaCl was 150 mM, the content of Tween 20 was 0.03%, and the pH was adjusted to 7.4; in running buffer **B,** the concentration of NaH₂OP₄ was 10 mM, the concentration of Na₂HOP₄ was 40 mM, the concentration of NaCl was 150 mM, the content of Tween 20 was 0.03%, the content of DMSO was 5.00%, and the pH was adjusted to 7.4. After the running buffers were prepared, they were filtered with 0.22 µm filter membrane.

BCL-XL protein fixation: The protein fixation buffer was used to fix BCL-XL on NTA chip through His capture and amino coupling. The NTA chip surface was washed with 50 mM NaOH and 350 mM EDTA at a flow rate of 60.0 µL/min for 60 seconds, respectively; then activated with 10 mM NiCl₂ for 1100 seconds, and then activated with a mixture of EDC (75.00 mg/mL) and NHS (11.50 mg/mL) with a volume ratio of 1:1 for 650 seconds at an activation flow rate of 10.0 µL/min; then BCL-XL (0.04 mg/mL) was injected at 4.0 µL/min for 850 seconds. After the BCL-XL injection was completed, a mixture of EDC (75.00 mg/mL) and NHS (11.50 mg/mL) with a volume ratio of 1:1 was used for cross-linking at a rate of 10.0 µL/min for 200 seconds, and finally 1 M ethanolamine (pH 8.5) was injected at a rate of 6.0 µL/min for 7 minutes to block the chip surface. The final fixed amount of BCL-XL was 4690.00 RU.

Compound dilution: The test compound was diluted with 100% DMSO to 100 times the required final concentration, after mixing well, 4.0 µL was pipetted and added to 396 µL of the running buffer **A,** centrifuged at 15000 rpm for 5 minutes, to obtain a 1X compound solution containing 1% DMSO for subsequent dilution. The compound to be tested was diluted gradiently by 2-fold dilution from 50 µM to 9 concentrations (the 2-fold dilution was carried out by adding 200 µL of the compound solution to 200 µL of the running buffer **B,** 10 concentration gradients comprised one 0 concentration (using the running buffer **B** instead), and the starting concentration was 50 µM). The diluted compound was transferred to a 96-well plate for sample injection.

Running program: The experiment was run at 25 °C, the running buffer B was used during the running program and had a flow rate of 30.0 µL/min. After 8 injections of the running buffer **B** to complete equilibrium, the compound was injected from the lowest concentration to the highest concentration, and the association time and dissociation time were both 120 seconds. After each injection, the injection needle was washed with 50% DMSO. The solvent difference caused by DMSO was corrected with 0.50%, 0.75%, 1.00%, 1.25% and 1.50% DMSO.

### 2. Experimental results

The response value of the compound to bind to BCL-XL protein was analyzed after deducting the reference channel and 0 concentration, and the affinity Kd was fitted by Biacore T200 Evaluation Software using the steady state affinity model (1: 1 binding model).

The prepared compounds were subjected to SPR detection according to the above method, and the experimental results were shown in Table 17.

**Table 17. Response values of compounds to bind to BCL-XL**

| Compound # | Kd | Compound # | Kd | Compound # | Kd |
|---|---|---|---|---|---|
| **A1** | +++ | **A27** | + | **D4** | ++++ |
| **A2** | +++ | **B1** | +++ | **D5** | +++ |
| **A3** | ++ | **B2** | +++ | **D6** | ++++ |
| **A4** | ++ | **C1** | +++ | **E1** | + |
| **A5** | + | **C2** | ++++ | **E6** | + |
| **A6** | + | **C3** | +++ | **E7** | + |
| **A8** | ++ | **C4** | ++ | **E8** | + |
| **A9** | + | **C5** | ++++ | **E9** | + |
| **A13** | + | **C6** | ++++ | **F6** | + |
| **A14** | + | **C7** | ++++ | **1A** | +++ |
| **A15** | + | **C8** | + | **2A** | +++ |
| **A16** | + | **C9** | + | **3A** | ++++ |
| **A21** | + | **C10** | +++ | **4A** | +++ |
| **D1** | ++++ | **C11** | + | **5A** | +++ |
| **D2** | +++ | **C12** | + | **6A** | ++ |
| **D3** | ++++ | **C14** | + | **7A** | + |
| **1B** | ++ | **2B** | ++ | **3B** | + |
| **1C** | +++ | **1D** | ++++ | **1E** | + |
| **1F** | +++ | | | | |

| | | | | | |
|---|---|---|---|---|---|
| Note: "+" was used in the table to indicate the range of Kd value, and the specific information was as follows: +: 10 µM to 100 µM; ++: 1 µM to 10 µM; +++: 0.1 µM to 1µM; ++++: <0.1 µM; -: > 100 µM. | | | | | |

The experimental results showed that the compounds of the present invention could effectively bind to BCL-XL protein, thereby further inhibiting the activity of BCL-XL protein. The compounds of the present invention could be used to prepare BCL-XL inhibitors, and medicaments for preventing and/or treating diseases related to BCL-XL protein activity.

In summary, the compounds provided by the present invention can effectively bind to BCL-XL protein, thereby further inhibiting the activity of BCL-XL protein. The compounds of the present invention can be used to prepare BCL-XL inhibitors and medicaments for preventing and/or treating diseases (e.g., cancer, autoimmune diseases, etc.) related to BCL-XL protein activity, and have broad prospects for clinical application. The compounds of the present invention have good pharmacological properties, good stability, high drugability, and are simple to prepare, with high yield and low cost, and are suitable for industrial production.

Although the specific embodiments of the present invention have been described in detail, those skilled in the art will understand that various modifications and substitutions can be made to those details based on all the teachings disclosed, and these changes are within the scope of protection of the present invention. The full scope of the present invention is given by the appended claims and any equivalents thereof.

## Claims

1. A compound, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein the structure of the compound is shown in Formula I: 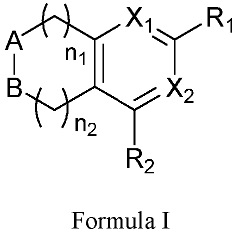
X₁, X₂ are each independently selected from the group consisting of N and CR^{x};
R^{x} is independently selected from the group consisting of hydrogen, -C_{1~6} alkyl, halogen-substituted C_{1~6} alkyl, halogen, -OH, -O(C_{1~6} alkyl), -NH₂, -NH(C_{1~6} alkyl), and -N(C_{1~6} alkyl)(C_{1~6} alkyl);
n₁, n₂ are each independently selected from the group consisting of 0, 1 and 2;
R₁ is selected from the group consisting of 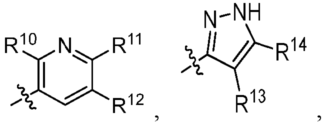 8- to 12-membered fused heteroaromatic ring, -NH(6- to 12-membered fused heteroaromatic ring), 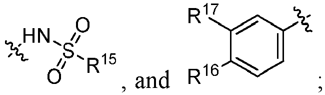 preferably, the 8- to 12-membered fused heteroaromatic ring is 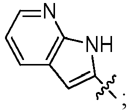
R¹⁰ is selected from the group consisting of hydrogen, halogen, cyano, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl), and -N(C_{1~10} alkyl)(C_{1~10} alkyl);
R¹¹ is selected from the group consisting of hydrogen, -C_{1~10} alkyl, halogen-substituted -C_{1~10} alkyl, -NH₂, -NH(C_{1~10} alkyl), -N(C_{1~10} alkyl)(C_{1~10} alkyl), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-C(O)NH(C_{1~10} alkyl), -C_{0~2} alkylene-C(O)N(C_{1~10} alkyl)(C_{1~10} alkyl), -C_{0~2} alkylene-O-(C_{6~10} aromatic ring), and -C_{0~2} alkylene-O-(5- to 10-membered aromatic heterocyclic ring);
R¹² is selected from the group consisting of hydrogen, halogen, cyano, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl), -N(C_{1~10} alkyl)(C_{1~10} alkyl), -C_{0~2} alkylene-C(O)NH(C_{1~10} alkyl), and -C_{0~2} alkylene-C(O)N(C_{1~10} alkyl)(C_{1~10} alkyl);
or, R¹¹, R¹² together with the atoms connected thereto form a 3- to 10-membered carbocyclic ring, a 3- to 10-membered heterocyclic ring, a C_{6~10} aromatic ring, or a 5- to 10-membered aromatic heterocyclic ring; the carbocyclic ring, heterocyclic ring, aromatic ring, or aromatic heterocyclic ring is unsubstituted or substituted with one, two, or three substituents independent selected from the group consisting of halogen, oxo, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl) and -N(C_{1~10} alkyl)(C_{1~10} alkyl);
R¹³ is selected from the group consisting of hydrogen, halogen, cyano, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl), and -N(C_{1~10} alkyl)(C_{1~10} alkyl);
R¹⁴ is selected from the group consisting of hydrogen, -C_{1~10} alkyl, halogen-substituted -C_{1~10} alkyl, -OH, -O(C_{1~10} alkyl), -NH₂, -NH(C_{1~10} alkyl), - and N(C_{1~10} alkyl)(C_{1~10} alkyl);
or, R¹³, R¹⁴ together with the atoms connected thereto form a C_{6~10} aromatic ring, or a 5- to 10-membered aromatic heterocyclic ring;
R¹⁵ is selected from the group consisting of -C_{1~10} alkyl, halogen-substituted -C_{1~10} alkyl, -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), and -C_{0~2} alkylene-(5- to 10-membered aromatic heterocyclic ring);
R¹⁶ is selected from the group consisting of hydrogen, halogen, cyano, -C_{2~10} alkenyl, -C_{2~10} alkynyl, -C_{1~10} alkyl, halogen-substituted -C_{1~10} alkyl, -NH₂, -NH(C_{1~10} alkyl), -N(C_{1~10} alkyl)(C_{1~10} alkyl), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-C(O)NH(C_{1~10} alkyl), -C_{0~2} alkylene-C(O)NH(C_{6~10} aromatic ring), -C_{0~2} alkylene-NHC(O)(C_{1~10} alkyl), -C_{0~2} alkylene-O-(C_{6~10} aromatic ring), -C_{0~2} alkylene-O-(5- to 10-membered aromatic heterocyclic ring), and -C_{0~2} alkylene-NHS(O)O(C_{1~10} alkyl);
R¹⁷ is selected from the group consisting of hydrogen, halogen, cyano, -C_{2~10} alkenyl, -C_{2~10} alkynyl, -C_{1~10} alkyl, halogen-substituted C_{1~10} alkyl, -OH, -O(C_{1~10} alkyl), -NH₂, -NH(C_{1~10} alkyl), -N(C_{1~10} alkyl)(C_{1~10} alkyl), -C_{0~2} alkylene-C(O)NH(C_{1~10} alkyl), -C_{0~2} alkylene-C(O)N(C_{1~10} alkyl)(C_{1~10} alkyl), -C_{0~2} alkylene-NHC(O)(C_{1~10} alkyl), and -C_{0~2} alkylene-NHS(O)O(C_{1~10} alkyl);
R¹⁶ and R¹⁷ are not hydrogen at the same time;
R₂ is selected from the group consisting of hydrogen, -OH, -O(C_{1~10} alkyl), -NH₂, -NH(C_{1~10} alkyl), and -N(C_{1~10} alkyl)(C_{1~10} alkyl); the alkyl is unsubstituted or substituted with one, two or three independent R²¹;
R²¹ is selected from the group consisting of halogen, -OH, -O(C_{1~10} alkyl), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), and -C_{0~2} alkylene-(5- to 10-membered aromatic heterocyclic ring);
one of A and B is CR⁴R⁵ and the other is NR₃;
R⁴ is selected from the group consisting of hydrogen, -C_{1~6} alkyl, and halogen-substituted C_{1~6} alkyl;
R⁵ is selected from the group consisting of hydrogen, -C_{1~6} alkyl, and halogen-substituted C_{1~6} alkyl;
R₃ is selected from the group consisting of hydrogen, -C_{1~6} alkyl, halogen-substituted C_{1~6} alkyl, -C_{0~2} alkylene-C(O)R³¹, -C_{0~2} alkylene-(3- to 10-membered cycloalkyl), -C_{0~2} alkylene-(3-to 10-membered heterocycloalkyl), -C_{0~2} alkylene-(C_{6~10} aromatic ring), and -C_{0~2} alkylene-(5-to 10-membered aromatic heterocyclic ring); the alkylene, alkyl, cycloalkyl, heterocycloalkyl, aromatic ring, aromatic heterocyclic ring are unsubstituted or substituted with one, two or three independent R³²;
R³¹ is selected from the group consisting of hydrogen, -C_{1~10} alkyl, halogen-substituted C_{1~10} alkyl, halogen, -OH, -O(C_{1~10} alkyl), -NH₂, -NH(C_{1~6} alkyl), -N(C_{1~6} alkyl)(C_{1~6} alkyl), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), and -C_{0~2} alkylene-(9- to 12-membered fused aromatic ring); the alkylene, alkyl, cycloalkyl, heterocycloalkyl, aromatic ring, aromatic heterocyclic ring are unsubstituted or substituted with one, two or three independent R^{32'};
R³² and R^{32'} are each independently selected from the group consisting of carboxyl, halogen, cyano, carbonyl, nitro, -C_{2~10} alkenyl, -C_{2~10} alkynyl, -C_{1~10} alkyl, halogen-substituted C_{1~10} alkyl, -OH, -O(C_{1~10} alkyl), -O(halogen-substituted C_{1~6} alkyl), -NH₂, -NH(C_{1~10} alkyl), -N(C_{1~10} alkyl)(C_{1~10} alkyl), -C_{0~2} alkylene-O-(C_{6~10} aromatic ring), -C_{0~2} alkylene-O-(5- to 10-membered aromatic heterocyclic ring), -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), -C_{0~2} alkylene-C(O)OLi, -C_{0~2} alkylene-C(O)OH, -C_{0~2} alkylene-C(O)NHR³³, and -C_{0~2} alkylene-NHC(O)R³³; the alkylene, alkyl, alkenyl, alkynyl, cycloalkyl, heterocycloalkyl, aromatic ring, aromatic heterocyclic ring are unsubstituted or substituted with one, two or three independent R^{33'};
R³³ and R^{33'} are each independently selected from the group consisting of hydrogen, carboxyl, halogen, -C_{1~10} alkyl, -C_{2~10} alkenyl, -C_{2~10} alkynyl, -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-NR³⁴R³⁵, -C_{0~2} alkylene-NHC(O)R³⁴, -C_{0~4} alkylene-C(O)NHR³⁴, and -C_{0~2} alkylene-NHC(O)OR³⁴; the alkylene, alkyl, alkenyl, alkynyl, cycloalkyl, heterocycloalkyl, aromatic ring, aromatic heterocyclic ring are unsubstituted or substituted with one, two or three independent R^{34'};
R³⁴ and R^{34'} are each independently selected from the group consisting of hydrogen, halogen, -C_{1~10} alkyl, -C_{2~10} alkenyl, -C_{2~10} alkynyl, -NH₂, -NH(C_{1~10} alkyl), -N(C_{1~10} alkyl)(C_{1~10} alkyl), -C_{0~2} alkylene-(3- to 10-membered cycloalkyl), -C_{0~2} alkylene-(3- to 10-membered heterocycloalkyl), -C_{0~2} alkylene-NHC(O)R³⁵, and -C_{0~2} alkylene-C(O)NHR³⁵; the alkylene, alkyl, alkenyl, alkynyl, cycloalkyl, heterocycloalkyl are unsubstituted or substituted with one, two or three independent R^{35'};
R³⁵ and R^{35'} are each independently selected from the group consisting of hydrogen, halogen, -C_{1~10} alkyl, -NH₂, -NH(C_{1~6} alkyl), -N(C_{1~6} alkyl)(C_{1~6} alkyl), -C_{0~2} alkylene-(3- to 10-membered cycloalkyl), -C_{0~2} alkylene-(3- to 10-membered heterocycloalkyl), -C_{0~2} alkylene-NHC(O)R³⁶, -C_{0~2} alkylene-C(O)NHR³⁶, -C_{0~2} alkylene-C(O)OR³⁶, and -C_{0~2} alkylene-C(O)R³⁶; the alkylene, alkyl, cycloalkyl, heterocycloalkyl are unsubstituted or substituted with one, two or three independent R^{36'};
R³⁶ and R^{36'} are each independently selected from the group consisting of hydrogen, halogen, -C_{1~10} alkyl, -NH₂, -NH(C_{1~6} alkyl), and -N(C_{1~6} alkyl)(C_{1~6} alkyl).

2. The compound according to claim 1, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein the structure of the compound is as shown in Formula IIa, Formula IIb, Formula IIc, Formula IId or Formula IIe: 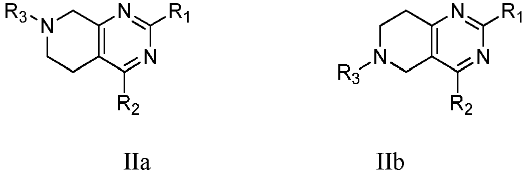 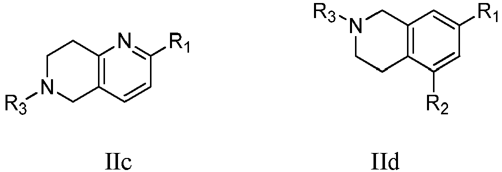 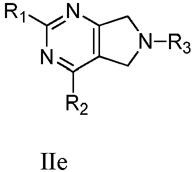 wherein, R₁, R₂, and R₃ are as described in claim 1.

3. The compound according to any one of claims 1 to 2, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein:
R¹⁰ is selected from the group consisting of hydrogen, -OH, halogen, and -C_{1~6} alkyl;
R¹¹ is selected from the group consisting of hydrogen, -C_{1~6} alkyl, -C_{0~2} alkylene-C(O)NH(C_{1~6} alkyl), and -C_{0~2} alkylene-O-(C₆ aromatic ring);
R¹² is selected from the group consisting of hydrogen, halogen, -C_{1~6} alkyl, and -C(O)NH(C_{1~6} alkyl);
or, R¹¹, R¹² together with the atoms connected thereto form a 3- to 6-membered heterocyclic ring or a C₆ aromatic ring; the heterocyclic ring or aromatic ring is unsubstituted or substituted with one, two or three independent substituents selected from the group consisting of halogen, hydroxyl, oxo, -C_{1~6} alkyl and halogen-substituted C_{1~6} alkyl;
R¹³ is selected from the group consisting of hydrogen, halogen, cyano, and -C_{1~6} alkyl;
R¹⁴ is selected from the group consisting of hydrogen, -C_{1~6} alkyl, halogen-substituted -C_{1~6} alkyl, -OH, -O(C_{1~10} alkyl), -NH₂, and -NH(C_{1~6} alkyl);
or, R¹³ and R¹⁴ together with the atoms connected thereto form a C₆ aromatic ring or a 5- to 6-membered aromatic heterocyclic ring;
R¹⁵ is selected from the group consisting of -C_{1~6} alkyl, halogen-substituted -C_{1~6} alkyl, -C_{0~2} alkylene-(C₆ aromatic ring), and -C_{0~2} alkylene-(5- to 6-membered aromatic heterocyclic ring);
R¹⁶ is selected from the group consisting of hydrogen, halogen, -C_{1~6} alkyl, -C_{0~2} alkylene-C(O)NH(C₆ aromatic ring), -C_{0~2} alkylene-NHC(O)(C_{1~6} alkyl), and -C_{0~2} alkylene-NHS(O)O(C_{1~6} alkyl);
R¹⁷ is selected from the group consisting of hydrogen, halogen, cyano, -C_{1~6} alkyl, halogen-substituted C_{1~6} alkyl, -C_{0~2} alkylene-C(O)NH(C_{1~6} alkyl), -C_{0~2} alkylene-C(O)N(C_{1~6} alkyl)(C_{1~6} alkyl), -C_{0~2} alkylene-NHC(O)(C_{1~6} alkyl), and -C_{0~2} alkylene-NHS(O)O(C_{1~6} alkyl);
R¹⁶ and R¹⁷ are not hydrogen at the same time.

4. The compound according to claim 3, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein:
R₁ is selected from the group consisting of 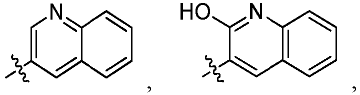 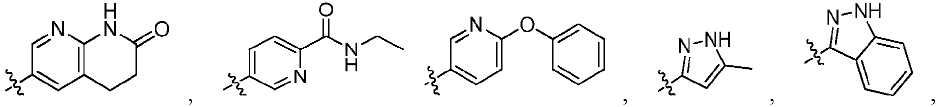 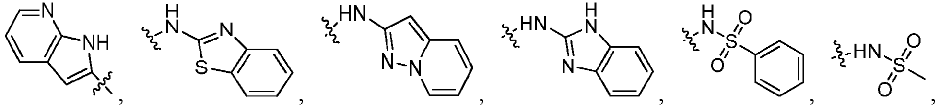 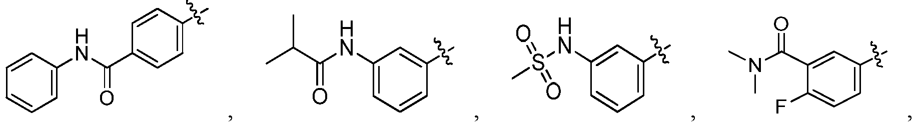 and

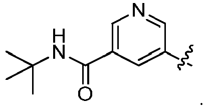

5. The compound according to any one of claims 1 to 4, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein: R₂ is selected from the group consisting of hydrogen, -OH, -O(C_{1~6} alkyl), -NH₂, -NH(C_{1~6} alkyl), and -N(C_{1~6} alkyl)(C_{1~6} alkyl); the alkyl is unsubstituted or substituted with one, two or three independent R²¹;
R²¹ is selected from the group consisting of halogen, -C_{0~2} alkylene-(C₆ aromatic ring), and -C_{0~2} alkylene-(5- to 6-membered aromatic heterocyclic ring).

6. The compound according to claim 5, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein: R₂ is selected from the group consisting of hydrogen, -OH, 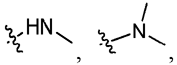 methoxy and

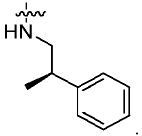

7. The compound according to any one of claims 1 to 6, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein: the R₃ is selected from the group consisting of hydrogen, -C_{1~6} alkyl, halogen-substituted C_{1~6} alkyl, -C_{0~2} alkylene-C(O)R³¹, -C_{0~2} alkylene-(C_{6~10} aromatic ring), and -C_{0~2} alkylene-(5- to 10-membered aromatic heterocyclic ring); the alkylene, alkyl, aromatic ring, aromatic heterocyclic ring are unsubstituted or substituted with one, two or three independent R³²;
R³¹ is selected from the group consisting of hydrogen, -C_{1~6} alkyl, halogen-substituted C_{1~6} alkyl, -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), and -C_{0~2} alkylene-(9- to 12-membered fused aromatic ring); the alkylene, alkyl, aromatic ring, aromatic heterocyclic ring are unsubstituted or substituted with one, two or three independent R^{32'};
R³² and R^{32'} are each independently selected from the group consisting of carboxyl, halogen, -C_{2~6} alkenyl, -C_{2~6} alkynyl, -C_{1~6} alkyl, halogen-substituted C_{1~6} alkyl, -OH, -O(C_{1~6} alkyl), -O(halogen-substituted C_{1~6} alkyl), -NH₂, -C_{0~2} alkylene-O-(C_{6~10} aromatic ring), -C_{0~2} alkylene-O-(5- to 10-membered aromatic heterocyclic ring), -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), -C_{0~2} alkylene-C(O)OLi, -C_{0~2} alkylene-C(O)OH, -C_{0~2} alkylene-C(O)NHR³³, and -C_{0~2} alkylene-NHC(O)R³³; the alkylene, alkyl, alkenyl, alkynyl, aromatic ring, aromatic heterocyclic ring are unsubstituted or substituted with one, two or three independent R^{33'};
R³³ and R^{33'} are each independently selected from the group consisting of hydrogen, halogen, -C_{1~6} alkyl, -C_{2~6} alkenyl, - C_{2~6} alkynyl, -C_{0~2} alkylene-(3- to 10-membered cycloalkyl), -C_{0~2} alkylene-(3- to 10-membered heterocycloalkyl), -C_{0~2} alkylene-(C_{6~10} aromatic ring), -C_{0~2} alkylene-(5- to 10-membered aromatic heterocyclic ring), -C_{0~2} alkylene-NR³⁴R³⁵, -C_{0~2} alkylene-NHC(O)R³⁴, -C_{0~4} alkylene-C(O)NHR³⁴, and -C_{0~2} alkylene-NHC(O)OR³⁴; the alkylene, alkyl, alkenyl, alkynyl, cycloalkyl, heterocycloalkyl, aromatic ring, aromatic heterocyclic ring are unsubstituted or substituted with one, two or three independent R^{34'};
R³⁴ and R^{34'} are each independently selected from the group consisting of hydrogen, halogen, -C_{1~6} alkyl, -C_{2~6} alkenyl, -C_{2~6} alkynyl, -N(C_{1~6} alkyl)(C_{1~6} alkyl), -C_{0~2} alkylene-(3- to 10-membered cycloalkyl), -C_{0~2} alkylene-(3- to 10-membered heterocycloalkyl), -C_{0~2} alkylene-NHC(O)R³⁵, and -C_{0~2} alkylene-C(O)NHR³⁵; the alkylene, alkyl, alkenyl, alkynyl, cycloalkyl, heterocycloalkyl are unsubstituted or substituted with one, two or three independent R^{35'};
R³⁵ and R^{35'} are each independently selected from the group consisting of hydrogen, halogen, -C_{1~6} alkyl, -C_{0~2} alkylene-(3- to 10-membered heterocycloalkyl), -C_{0~2} alkylene-C(O)OR³⁶, and -C_{0~2} alkylene-C(O)R³⁶; the alkylene, alkyl, heterocycloalkyl are unsubstituted or substituted with one, two or three independent R^{36'};
R³⁶ and R^{36'} are each independently selected from the group consisting of hydrogen, halogen, and -C_{1~6} alkyl.

8. The compound according to claim 7, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein: R₃ is selected from the group consisting of 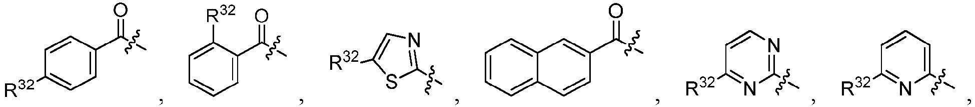 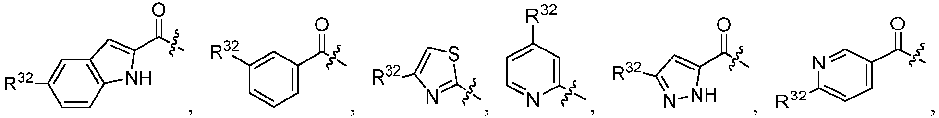 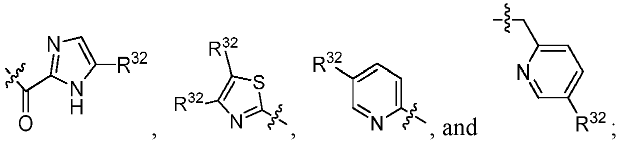
R³² is independently selected from the group consisting of hydrogen, carboxyl, halogen, -C_{2~6} alkenyl, -C_{2~6} alkynyl, -C_{1~6} alkyl, halogen-substituted C_{1~6} alkyl, -O(C_{1~6} alkyl), -O(halogen-substituted C_{1~6} alkyl), -NH₂, -C_{0~2} alkylene-O-(C_{6~10} aromatic ring), -C_{0~2} alkylene-O-(5- to 10-membered aromatic heterocyclic ring), -C_{0~2} alkylene-(C_{6~10} aromatic ring), -C_{0~2} alkylene-(5- to 10-membered aromatic heterocyclic ring), -C_{0~2} alkylene-(8- to 12-membered fused heteroaromatic ring), -C_{0~2} alkylene-C(O)OLi, -C_{0~2} alkylene-C(O)OH, and -C_{0~2} alkylene-C(O)NHR³³; the alkylene, alkyl, alkenyl, alkynyl, aromatic ring, aromatic heterocyclic ring are unsubstituted or substituted with one, two or three independent R^{33'};
R³³ and R^{33'} are each independently selected from the group consisting of hydrogen, halogen, -C_{1~6} alkyl, -C_{2~6} alkenyl, -C_{2~6} alkynyl, -C_{0~2} alkylene-(3- to 10-membered heterocycloalkyl), -C_{0~2} alkylene-(C_{6~10} aromatic ring), -C_{0~2} alkylene-NR³⁴R³⁵, -C_{0~2} alkylene-NHC(O)R³⁴, -C_{0~4} alkylene-C(O)NHR³⁴, and -C_{0~2} alkylene-NHC(O)OR³⁴; the alkylene, alkyl, alkenyl, alkynyl, heterocycloalkyl, aromatic ring are unsubstituted or substituted with one, two or three independent R^{34'};
R³⁴ and R^{34'} are each independently selected from the group consisting of hydrogen, -C_{1~6} alkyl, -C_{2~6} alkynyl, -N(C_{1~6} alkyl)(C_{1~6} alkyl), -C_{0~2} alkylene-(3- to 10-membered heterocycloalkyl), and -C_{0~2} alkylene-NHC(O)R³⁵; the alkylene, alkyl, alkynyl, heterocycloalkyl are unsubstituted or substituted with one, two or three independent R^{35'};
R³⁵ and R^{35'} are each independently selected from the group consisting of hydrogen, -C_{1~6} alkyl, -C_{0~2} alkylene-(3- to 10-membered heterocycloalkyl), -C_{0~2} alkylene-C(O)OR³⁶, and -C_{0~2} alkylene-C(O)R³⁶; the alkylene, alkyl, heterocycloalkyl are unsubstituted or substituted with one, two or three independent R^{36'}; R³⁶ and R^{36'} are each independently selected from the group consisting of hydrogen, and -C_{1~6} alkyl;
preferably, R₃ is 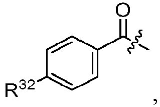
R³² is selected from the group consisting of carboxyl, -C₂ alkenyl, -C_{0~2} alkylene-O-phenyl, -C_{0~2} alkylene-O-benzimidazolyl, and -C_{0~2} alkylene-C(O)NHR³³; the alkylene, alkenyl, phenyl, benzimidazolyl are unsubstituted or substituted with one, two or three independent R^{33'};
R³³ and R^{33'} are each independently selected from the group consisting of hydrogen, halogen, -C_{1~3} alkyl, -C₂ alkynyl, -C_{0~2} alkylene-phenyl, -C_{0~2} alkylene-piperidinyl, -C_{0~2} alkylene-NHC(O)R³⁴, and -C_{0~4} alkylene-C(O)NHR³⁴; the alkylene, alkyl, alkynyl, phenyl, piperidinyl are unsubstituted or substituted with one, two or three independent R^{34'};
R³⁴ and R^{34'} are each independently selected from the group consisting of hydrogen, -C_{1~2} alkyl, -N(C_{1~2} alkyl)(C_{1~2} alkyl), and -C_{0~2} alkylene-piperazinyl; the alkylene, alkyl, piperazinyl are unsubstituted or substituted with one, two or three independent R^{35'};
R^{35'} is selected from the group consisting of hydrogen, -C_{1~2} alkyl, and -C_{0~2} alkylene-C(O)OR³⁶; the alkyl is unsubstituted or substituted with one, two or three independent R^{36'}; R³⁶ and R^{36'} are independently selected from the group consisting of hydrogen, and -C_{1~4} alkyl;
or preferably, R₃ is 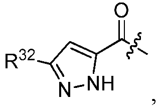
R³² is selected from the group consisting of hydrogen, and phenyl; the phenyl is unsubstituted or substituted with one, two or three independent R^{33'};
R^{33'} is selected from the group consisting of hydrogen, halogen, -C_{1~6} alkyl, -C_{2~6} alkynyl, -C_{0~2} alkylene-piperidinyl, -C_{0~2} alkylene-NHC(O)R³⁴, -C_{0~4} alkylene-C(O)NHR³⁴, and -C_{0~2} alkylene-NHC(O)OR³⁴; the alkylene, alkyl, alkynyl, piperidinyl are unsubstituted or substituted with one, two or three independent R^{34'};
R³⁴ and R^{34'} are each independently selected from the group consisting of hydrogen, -C_{1~2} alkyl, piperazinyl, and -N(C_{1~2} alkyl)(C_{1~2} alkyl); the alkyl and piperazinyl are unsubstituted or substituted with one, two or three independent R^{35'};
R^{35'} is selected from the group consisting of hydrogen, and -C_{0~2} alkylene-C(O)R³⁶; the alkylene is unsubstituted or substituted with one, two or three independent R^{36'}; R³⁶ and R^{36'} are each independently selected from the group consisting of hydrogen, and -C_{1~2} alkyl.

9. The compound according to claim 8, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein: R₃ is selected from the group consisting of 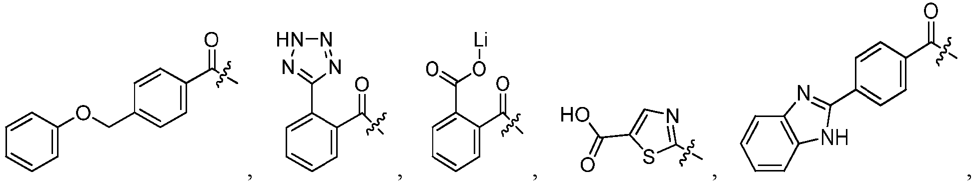 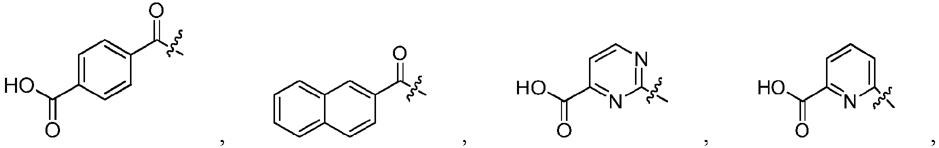 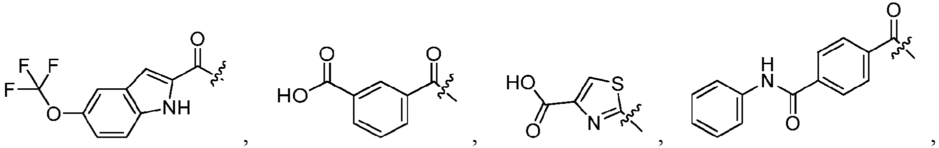 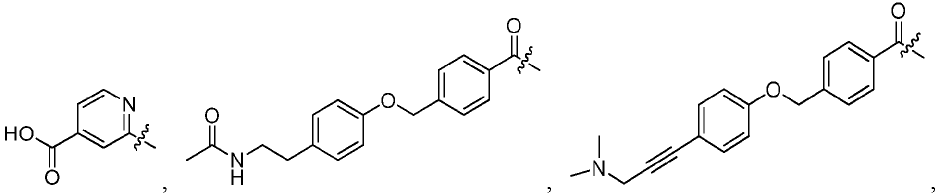 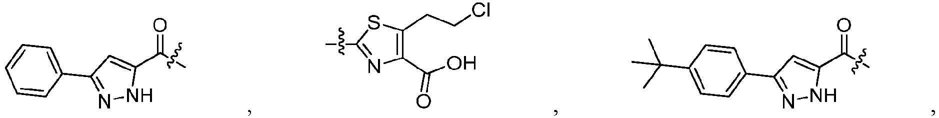 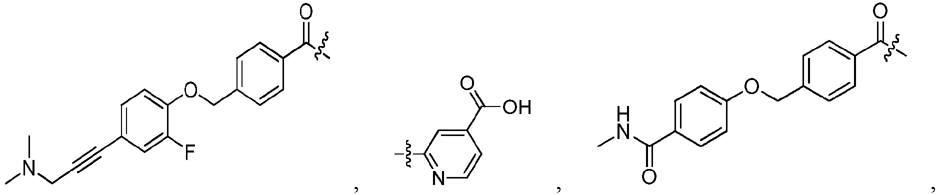 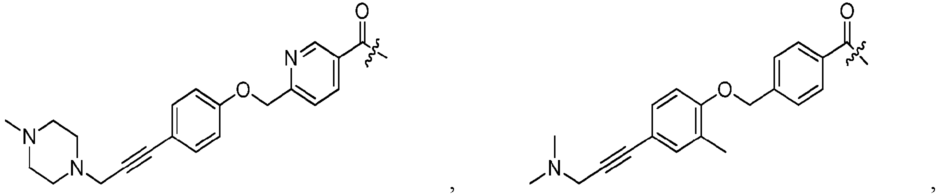 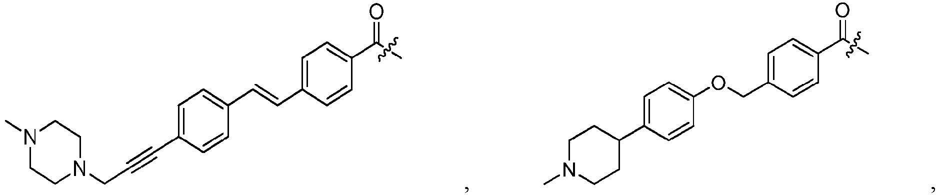 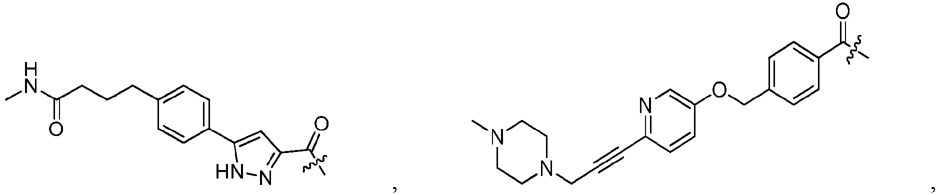 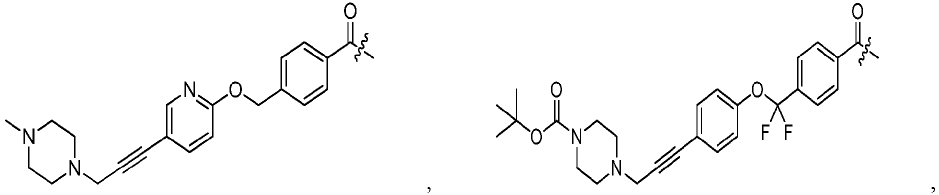 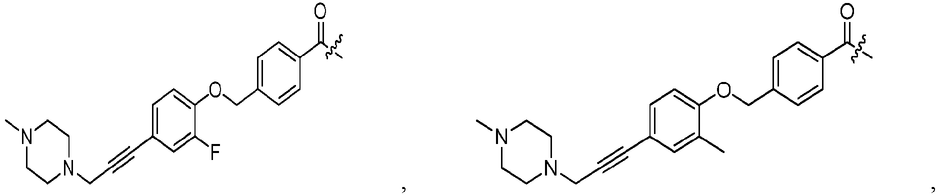 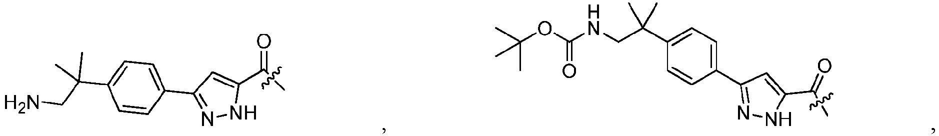 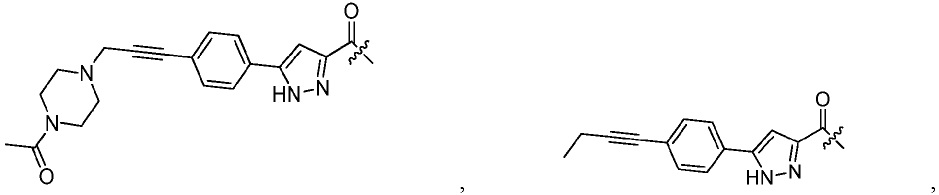 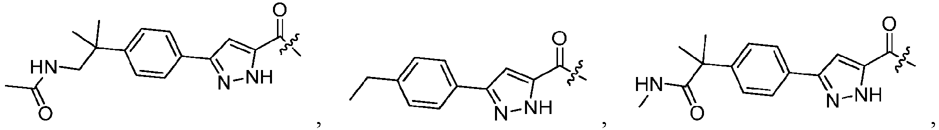 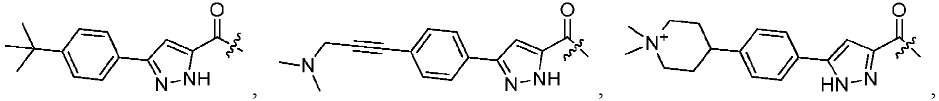 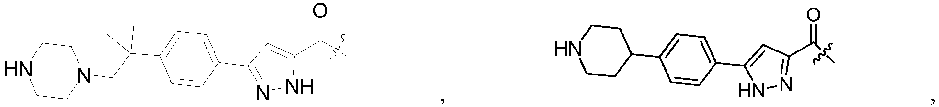 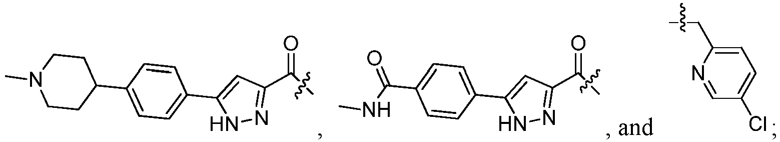 preferably, R³ is selected from the group consisting of: 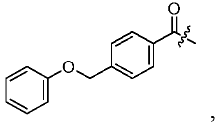 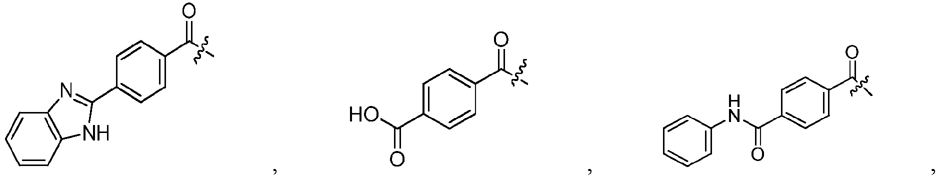 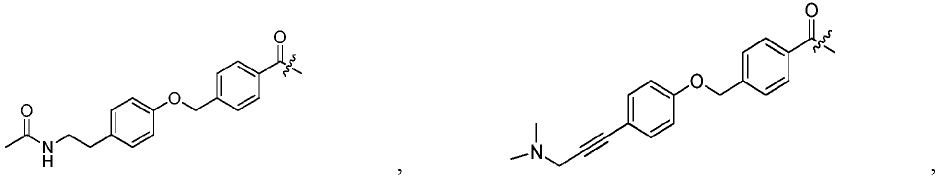 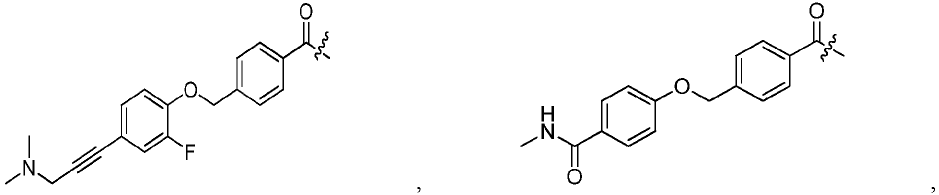 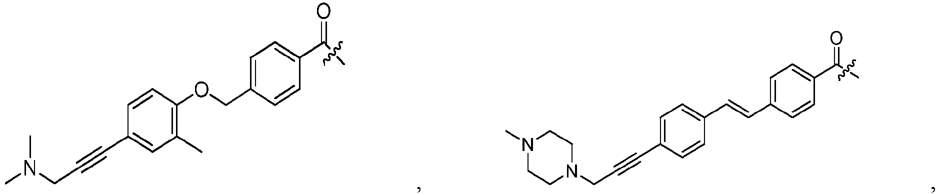 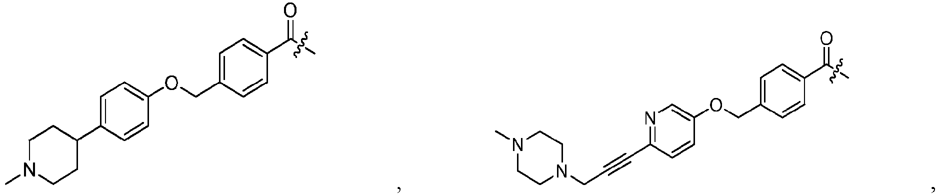 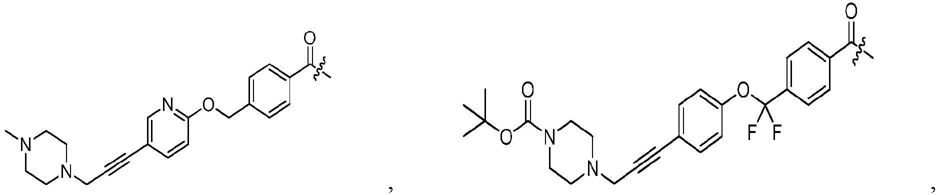 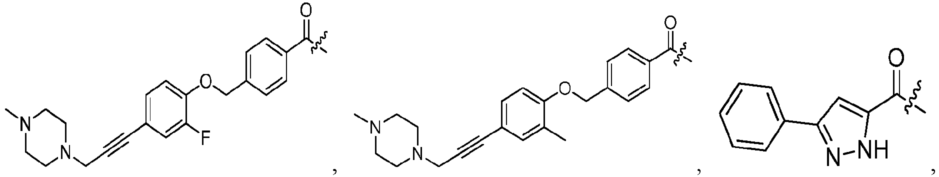 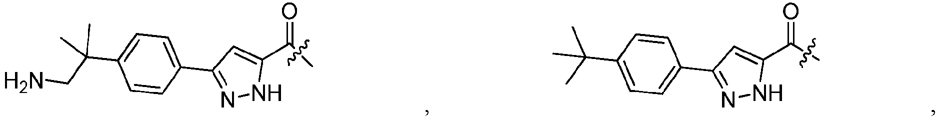 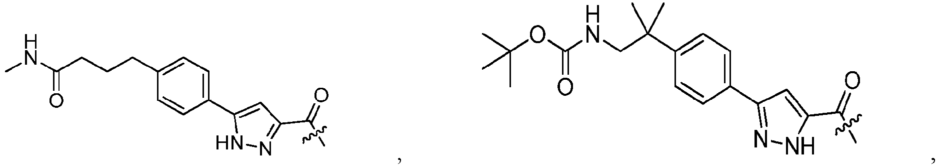 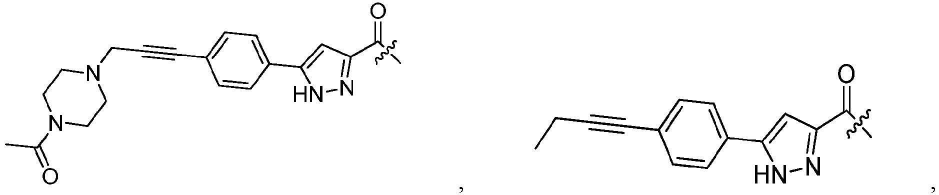 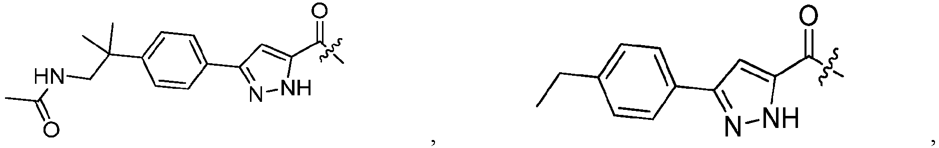 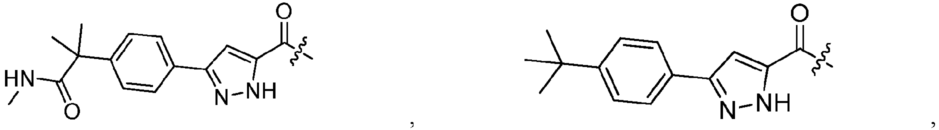 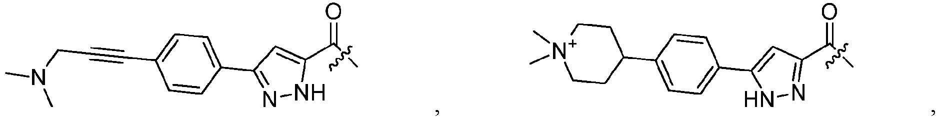 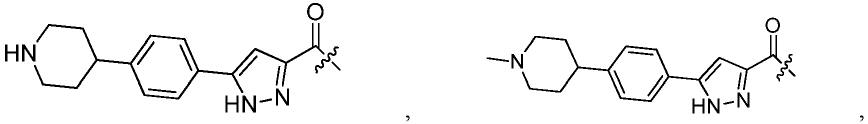 and

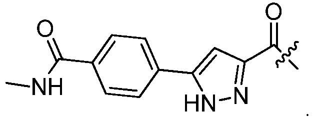

10. The compound according to any one of claims 1 to 9, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein: the structure of the compound is as shown in Formula III: 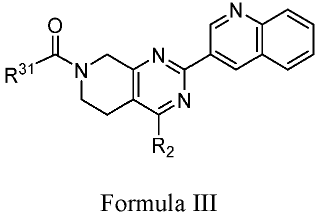 preferably, R₂ is selected from the group consisting of hydrogen, and

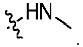

11. The compound according to claim 10, or stereoisomer thereof, or pharmaceutically acceptable salt thereof, wherein: R³¹ is selected from the group consisting of 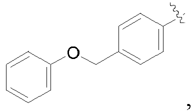 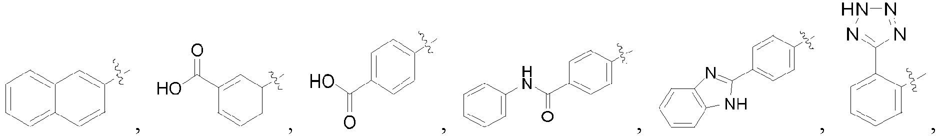 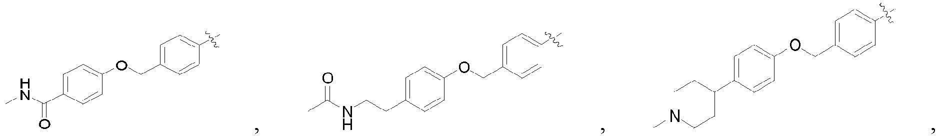 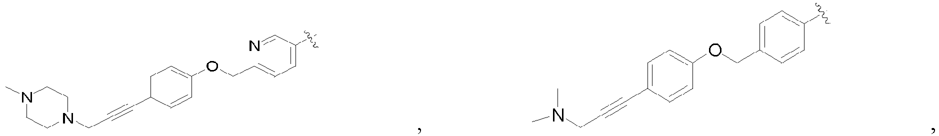 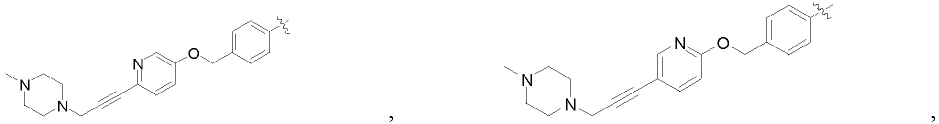 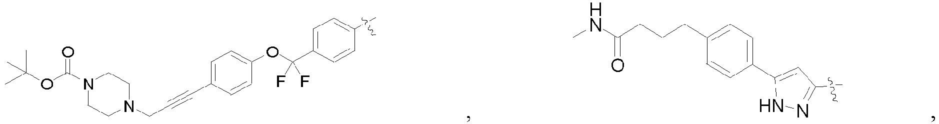 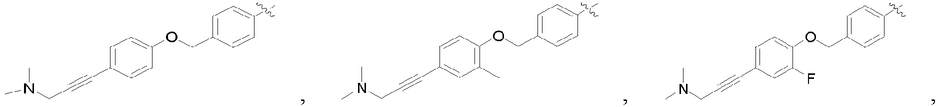 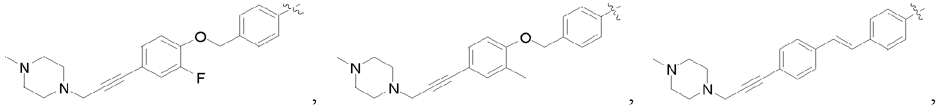 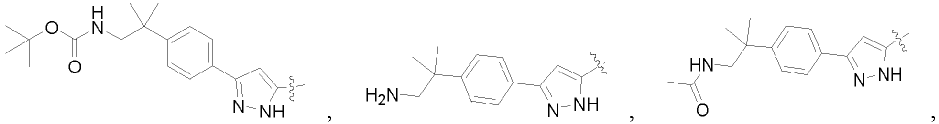 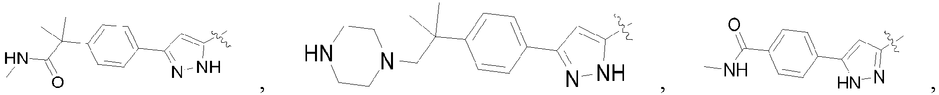 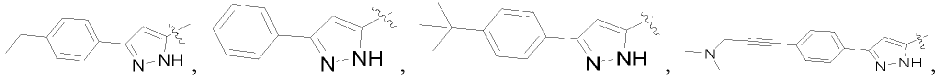 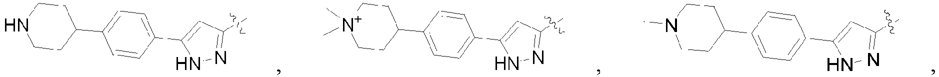 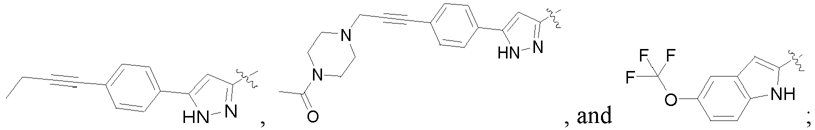 preferably, R³¹ is selected from the group consisting of:

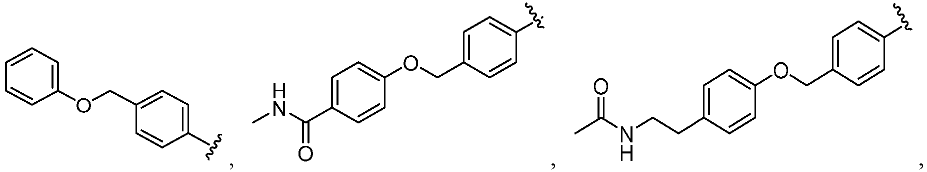

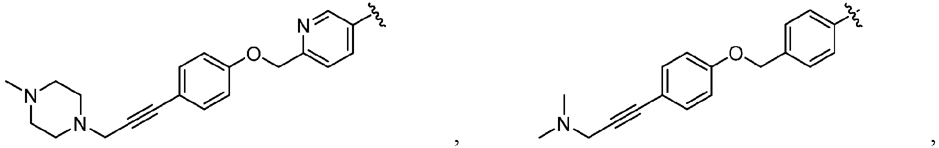

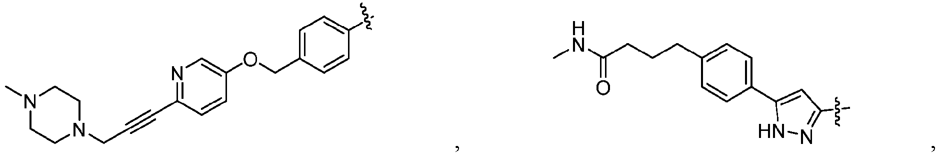

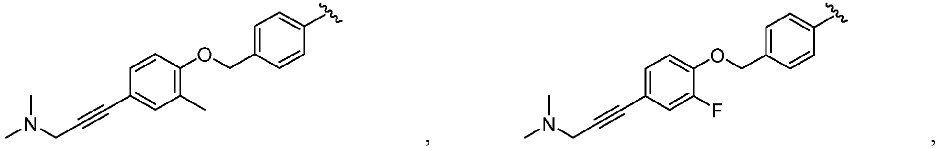

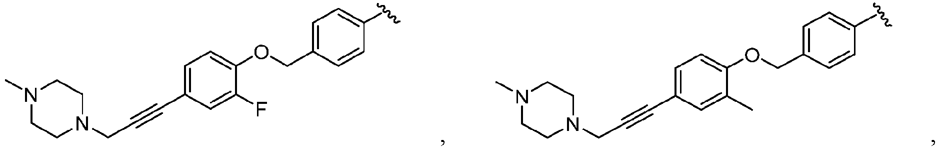

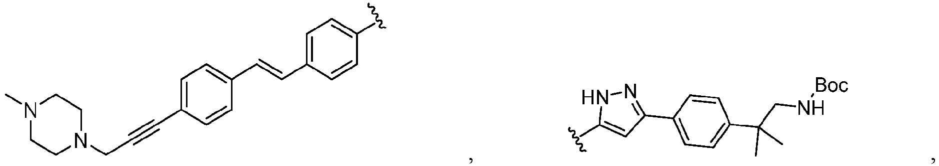

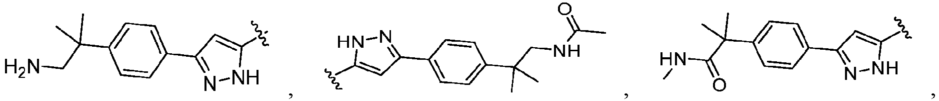

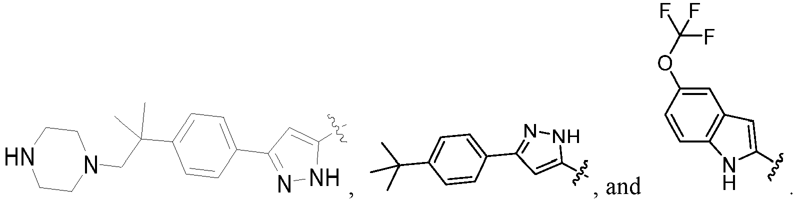

12. The compound according to claim 1, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof, wherein the structure of the compound is selected from the group consisting of

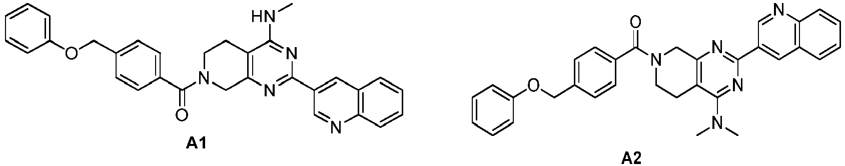

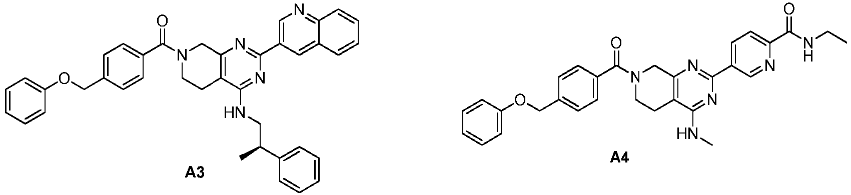

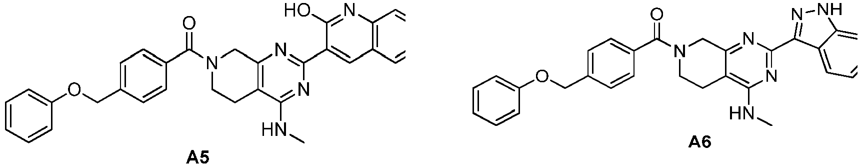

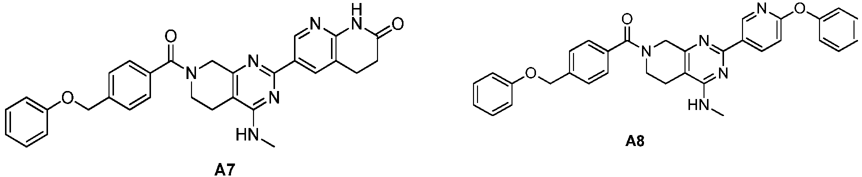

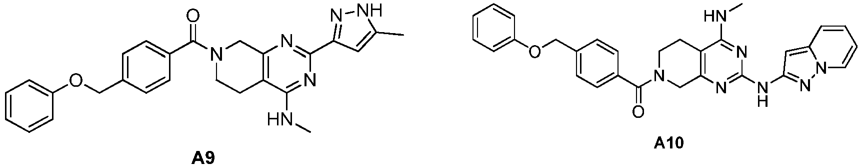

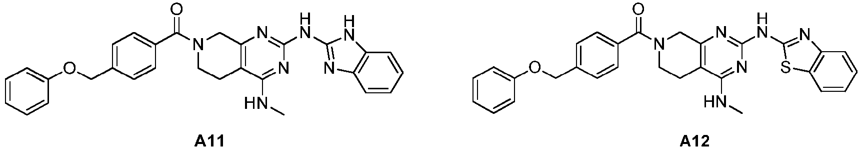

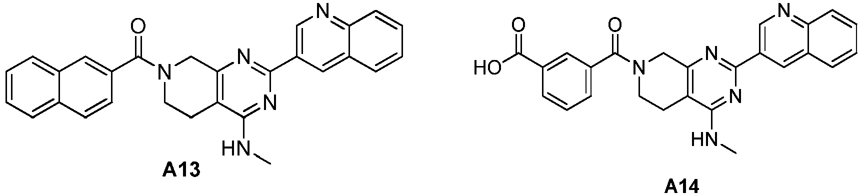

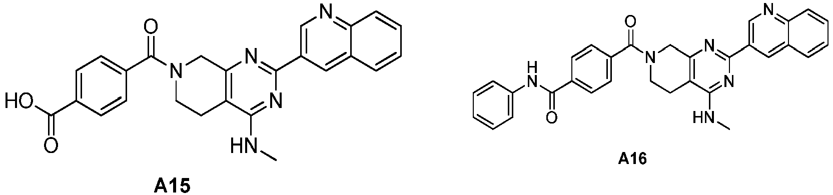

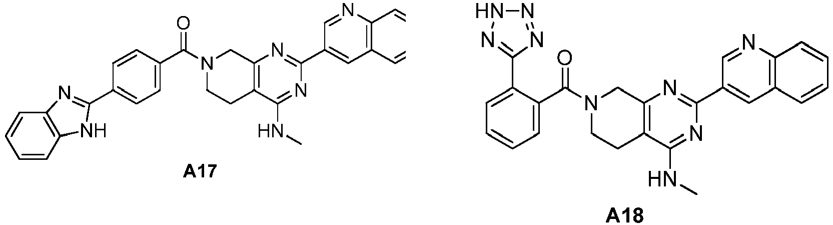

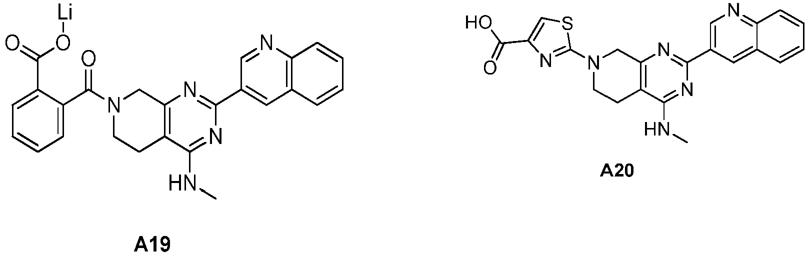

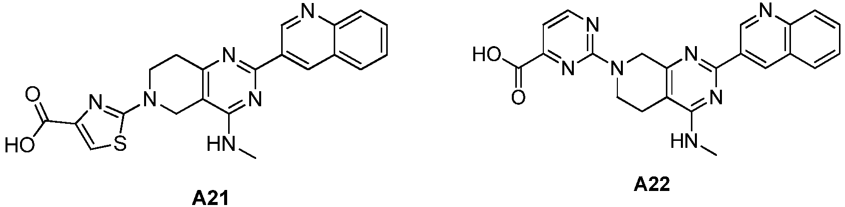

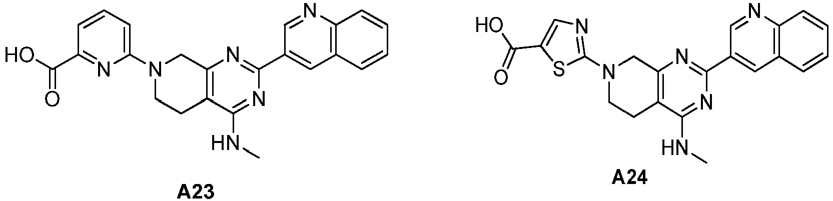

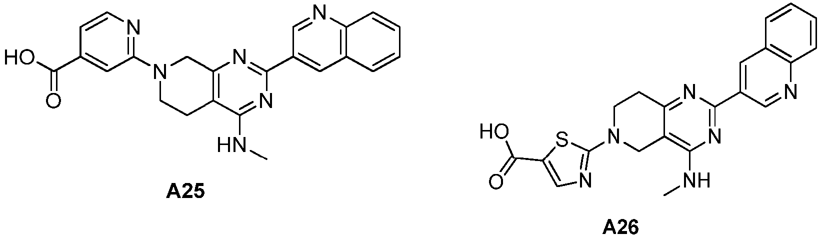

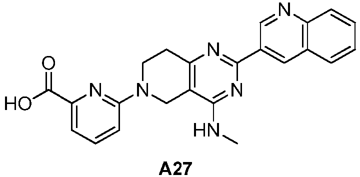

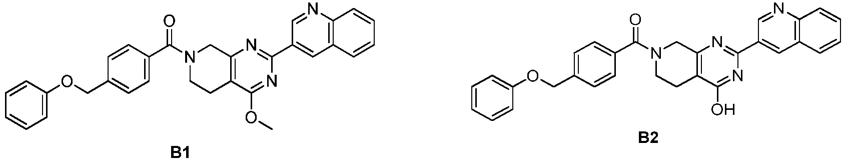

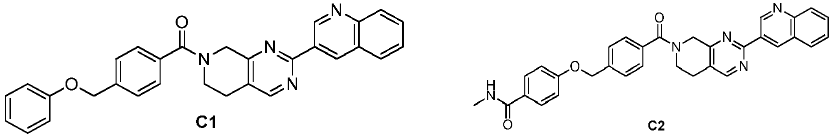

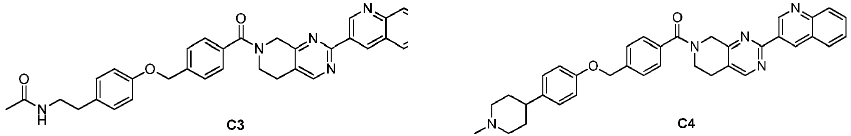

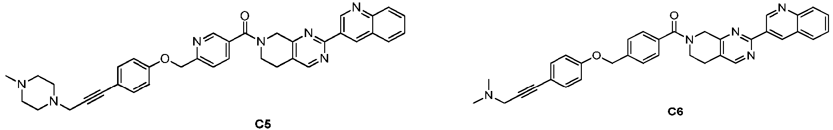

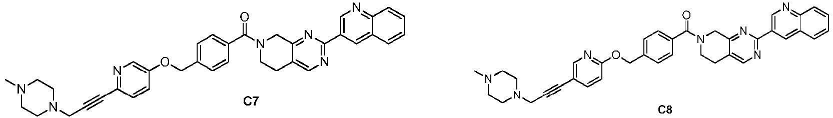

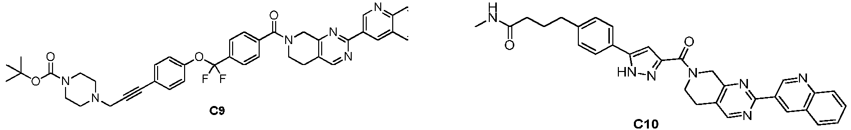

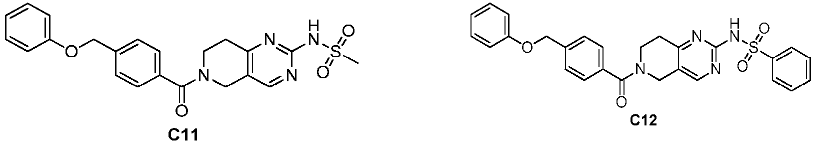

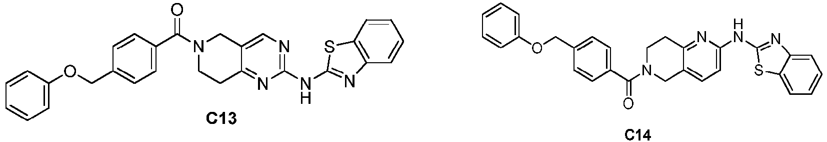

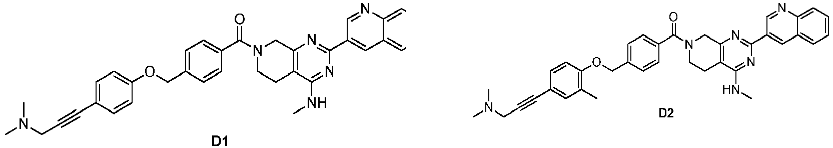

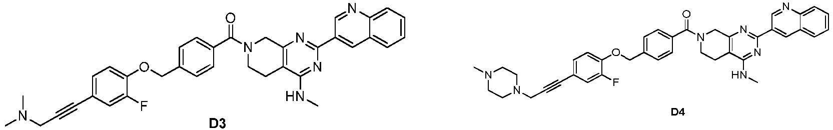

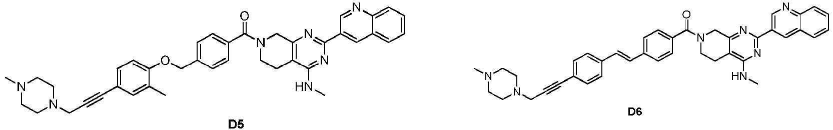

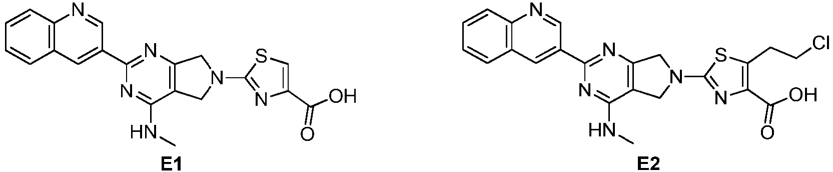

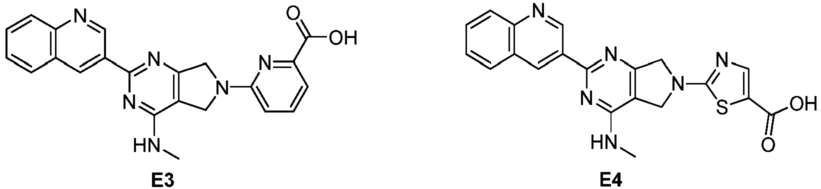

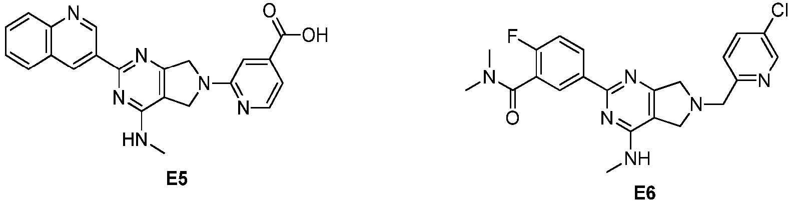

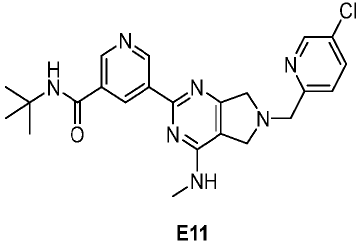

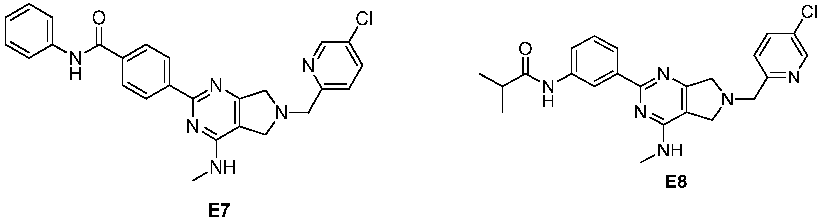

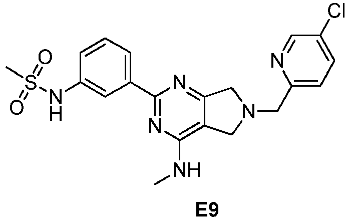

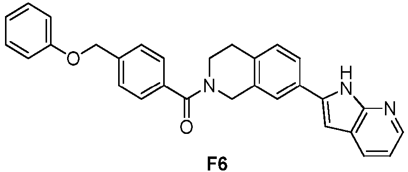

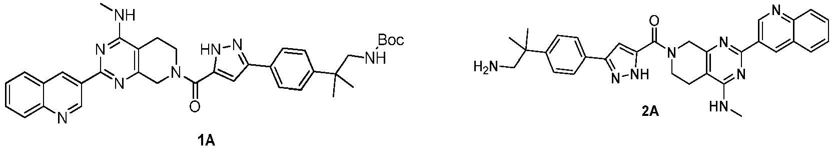

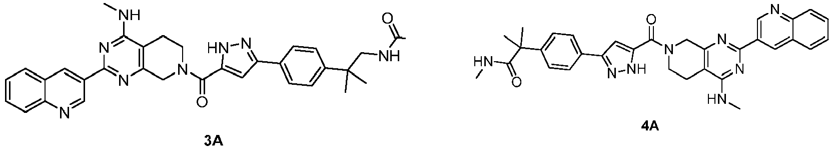

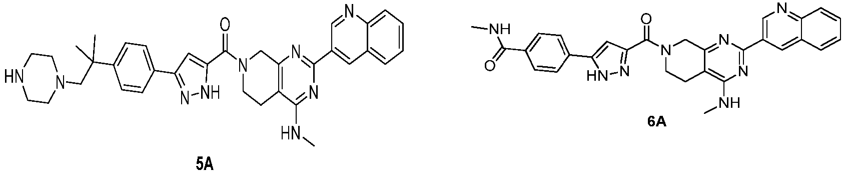

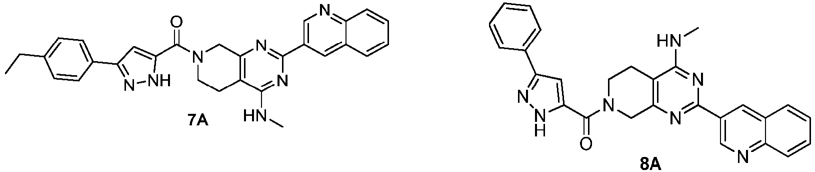

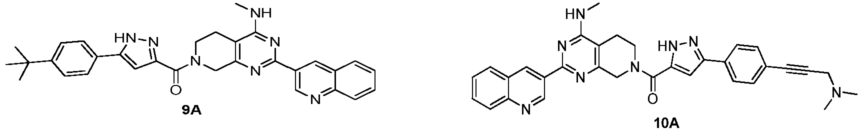

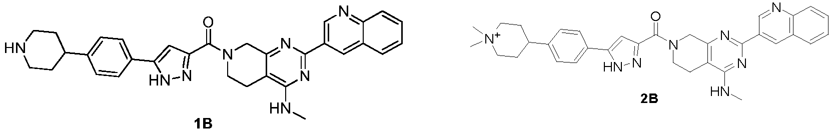

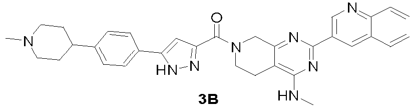

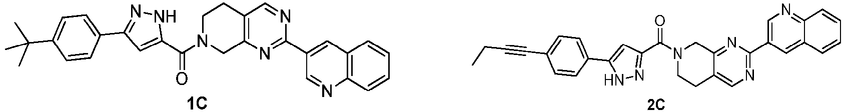

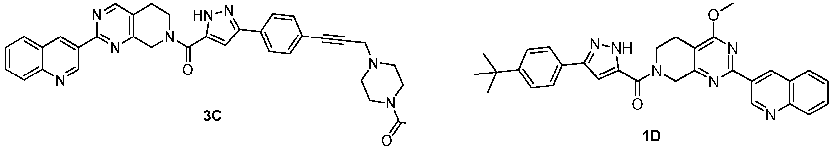

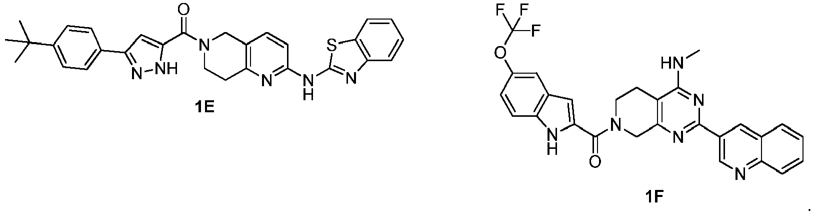

13. A pharmaceutical composition, comprising the compound, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof according to any one of claims 1 to 12, and a pharmaceutically acceptable excipient.

14. Use of the compound, or stereoisomer thereof, or pharmaceutically acceptable salt thereof, or deuterated compound thereof, or tautomer thereof, or polymorph thereof, or solvate thereof, or N-oxide thereof, or isotope-labeled compound thereof, or metabolite thereof, or prodrug thereof according to any one of claims 1 to 12, or the pharmaceutical composition according to claim 13 in the manufacture of a BCL-XL inhibitor.

15. The use according to claim 14, wherein the BCL-XL inhibitor is capable of binding to BCL-XL protein.

16. The use according to claim 14 or 15, wherein the BCL-XL inhibitor is a medicament for preventing and/or treating a disease related to BCL-XL protein activity.

17. The pharmaceutical use according to claim 16, wherein the disease related to BCL-XL protein activity is selected from the group consisting of autoimmune disease, bladder cancer, brain cancer, breast cancer, bone marrow cancer, cervical cancer, colorectal cancer, esophageal cancer, hepatocellular carcinoma, follicular lymphoma, lymphoid malignancy of T cell or B cell origin, melanoma, myeloma, oral cancer, ovarian cancer, leukemia, non-small cell lung cancer, prostate cancer, small cell lung cancer and spleen cancer; the leukemia is preferably chronic lymphocytic leukemia, primitive lymphocytic leukemia or granulocytic leukemia.
